(19) Europäisches Patentamt
European Patent Office
Office européen des brevets

(11) EP 4 484 566 A1

(12) **EUROPEAN PATENT APPLICATION**

(43) Date of publication:
**01.01.2025 Bulletin 2025/01**

(21) Application number: **23181910.3**

(22) Date of filing: **27.06.2023**

(51) International Patent Classification (IPC):
***C12P 19/32*** $^{(2006.01)}$ ***C12N 15/52*** $^{(2006.01)}$
***C25B 3/09*** $^{(2021.01)}$

(52) Cooperative Patent Classification (CPC):
**C12P 19/32; C12N 15/52;** C12Y 101/01027; C12Y 102/01003; C12Y 102/01004; C12Y 102/0101; C12Y 102/01012; C12Y 102/01013; C12Y 102/01038; C12Y 102/01059; C12Y 102/07005; C12Y 203/01008; C12Y 203/01019; C12Y 207/01001; C12Y 207/02015; (Cont.)

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Max-Planck-Gesellschaft zur Förderung der Wissenschaften e.V.**
**80539 München (DE)**

(72) Inventor: **The designation of the inventor has not yet been filed**

(74) Representative: **Arth, Hans-Lothar**
**ABK Patent Attorneys**
**Jasminweg 9**
**14052 Berlin (DE)**

Remarks:
The complete document including Reference Table(s) and the Sequence Listing(s) can be downloaded from the EPO website

(54) **ATP PRODUCTION FROM ELECTRICITY WITH A NEW-TO-NATURE ELECTROBIOLOGICAL MODULE**

(57) The present invention relates to a method and system for producing adenosine triphosphate (ATP) through an acid/aldehyde-ATP (AAA) cycle in an electrochemical cell.

**Figure 2**

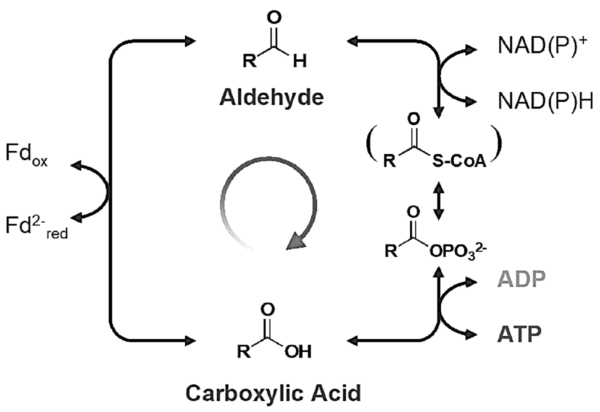


$$Fd^{2-}_{red} + NAD(P)^{+} + ADP + Pi \rightarrow Fd_{ox} + NAD(P)H + ATP$$

$(\Delta G'^{0} = 8.9\ kJ/mol)$

(52) Cooperative Patent Classification (CPC): (Cont.)
C25B 3/09

## Description

The present invention relates to a method and system for producing adenosine triphosphate (ATP) through an acid/aldehyde-ATP (AAA) cycle in an electrochemical cell.

## Background of the invention

The transition to renewable energy systems is instrumental for achieving a carbon neutral society. Electrification, especially when based on renewable energies, is a key element in reaching this goal and is expected to increase worldwide from 21% to 30% of the total energy consumption by 2030. Despite the growing availability of electricity from renewables, one major challenge is the use and storage of electrical power, which currently amounts to ~3 terawatt (TW) per year (ca. 28,000 TWh, ca. 100 Exajoule). Several energy storage systems have been developed, with batteries being one of the most prominent technical storage solutions.

Even though biological systems are able to use and store more than 130 TW per year, interfacing them directly with electricity has been explored only sparsely. This is mainly due to the fact that organisms have not evolved to convert externally provided electrical energy, which has limited the direct use of electricity in bio(techno)logy, beyond simple redox reactions.

Current efforts to use (and store) electrical energy in biological systems mainly focus on the electricity-powered production of electron-carrying substrates, such as hydrogen, CO, formate, methanol, or acetate. These electron carriers are subsequently metabolized and/or respired by microbial cells to conserve chemical energy in form of ATP, the primary energy carrier of the cell. Alternatively, electromicrobial systems have been developed in which microbial cells are directly powered with electricity. In these cases, electrons are directly fed through redox shuttles into the cellular respiratory chain to create ATP.

However, common to all these approaches is that electrical energy is only indirectly converted into ATP through a rather complicated process that involves several membrane-bound (redox) protein complexes. Through multi-step electron transfers between different redox centers, a proton motive force is built up across the membrane, which is subsequently harvested for ATP synthesis (Fig. 1). At each step of this process, free energy is dissipated (Fig. 1), which limits the overall thermodynamic efficiency of ATP synthesis. Current estimates assume a thermodynamic efficiency ($\eta$) between 40% and 70% to generate one molecule ATP from ADP and inorganic phosphate. Overall, the complex biochemistry and thermodynamic constraints of these naturally evolved membrane-based systems or comparable vesicle-based derivatives, have restricted the use of external electricity to power cell-free and/or in vitro systems.

It is the objective of the present invention to provide a method for producing ATP from electricity.

The objective of the present invention is solved by the teaching of the independent claims. Further advantageous features, aspects and details of the invention are evident from the dependent claims, the description, the figures, and the examples of the present application.

## Brief description of the invention

Electric power is paramount to the technical world and plays an increasingly important role as a future energy carrier. Yet, most biological systems have not evolved to use this form of energy. The present invention relates to a new-to-nature electrobiological module, the acid/aldehyde ATP cycle (AAA cycle), for the direct conversion of electrical energy into ATP. The AAA cycle contains a minimum set of enzymes and does not require membrane-based charge separation. Realizing a propionate-based version of the AAA cycle, the inventors of the present invention could demonstrate continuous, electricity-driven regeneration of ATP and other energy storage molecules from 0.6 V vs. SHE at 2.7 $\mu$mol $cm^{-2}h^{-1}$ and with faradaic efficiencies of up to 47%. Notably, the AAA cycle is compatible with more complex biological systems, such as in vitro transcription/-translation, powering biological information storage directly from electricity. This new link between the technical and biological world opens several possibilities for future applications in synthetic biology, electrobiotechnology, and bioelectrocatalysis.

Thus, the present invention relates to a new-to-nature module for the conversion of electricity into chemical energy, which consists of only four enzyme components, does not require membrane-based charge separation, and can be coupled to different *in vitro* processes. Compared to natural or reconstructed membrane-based systems, the inventive $AAA_P$ cycle allows the direct use of electrical energy for biological processes, such as catalytic conversions and information processing, and its further storage in biological molecules (such as G6P, nucleic acids, or proteins).

When coupled to downstream processes, the $AAA_P$ cycle shows a faradaic efficiency of up to 47% even without extensive optimizing.

Overall, the $AAA_P$ cycle represents a new interface between electrical and biological systems, which might lay the foundation for different applications in bio(electro)catalysis, biotechnology, and synthetic biology in the future. Using electricity directly for storing energy and information in (synthetic) biological systems will open new ways to link the

technical and natural world.

Thus, the present invention relates to a method for producing adenosine triphosphate (ATP) through an acid/aldehyde-ATP (AAA) cycle in an electrochemical cell comprising the steps:

i) providing an AAA cycle reaction mixture in the electrochemical cell, the AAA cycle comprising:

a) a carboxylic acid or a salt thereof,
b) a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2) a CoA-acetylating aldehyde dehydrogenase for catalyzing the conversion of the aldehyde into the corresponding CoA ester;
(b3) a phosphate acyltransferase for catalyzing the conversion of the CoA ester to the corresponding acyl phosphate;
(b4) a carboxylic acid kinase for catalyzing the conversion of the acyl phosphate to the corresponding carboxylic acid;

or a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2-3) a phosphate reductase for catalyzing the conversion of the aldehyde to the corresponding acyl phosphate;
(b4) a carboxylic acid kinase for catalyzing the conversion of the acyl phosphate to the corresponding carboxylic acid;

c) adenosine diphosphate and Pi;
d) coenzyme A;
e) $NAD^+$ or $NADP^+$,
f) a low-potential electron carrier;

ii) applying electricity to the electrochemical cell for conversion of the electron carrier into its reduced form, and
iii) producing adenosine triphosphate from the AAA cycle and electricity.

In some embodiments, the carboxylic acid is selected from the group comprising short chain fatty acids, medium chain fatty acids, glyceric acid, glyceric acid derivatives, benzoic acid, benzoic acid derivatives, aromatic acids.

In some embodiments, the set of enzymes for the AAA cycle further comprises a (b5) a lactate dehydrogenase for catalyzing the conversion of NADPH to $NADP^+$, and wherein the reaction mixture further comprises pyruvate.

In some embodiments, the ferredoxin-dependent aldehyde oxidoreductase is a tungsten-containing aldehyde oxidoreductase from *Aromatoleum aromaticum* EbN1 $AOR_{Aa}$, and /or the CoA-acetylating aldehyde dehydrogenase is a NADPH-dependent CoA-acetylating aldehyde dehydrogenase, and wherein $NADP^+$ is provided in step e).

In embodiments, the carboxylic acid is propionic acid or propionate.

In other embodiments, the CoA-acetylating aldehyde dehydrogenase is PduP-NP (SEQ ID NO: 9), and/or the phosphate acyltransferase is phosphate acetyltransferase Pta from E. *coli*; and the carboxylic acid kinase is propionate kinase TdcD from E. *coli*.

In further embodiments, the low-potential electron carrier is selected from a viologen derivative, hexamethyl viologen, tetramethyl viologen, Ti(III) citrate, and Eu (II)-EGTA.

In further embodiments, the electrochemical cell comprises a cathodic compartment, an anodic compartment, and a proton-conducting membrane, wherein the proton-conducting membrane physically separates the cathodic and anodic compartments.

In a particular embodiment of the invention, the method comprises the steps:

i) providing an AAA cycle reaction mixture in the electrochemical cell, the AAA cycle comprising:

a) propionic acid or propionate;
b) a set of enzymes comprising:

(b1) a ferredoxin-dependent aldehyde oxidoreductase for catalyzing the conversion of propionic acid to propionaldehyde;
(b2) a NADPH-dependent CoA-acetylating aldehyde dehydrogenase for catalyzing the conversion of proprionaldehyde into propionyl-CoA,
(b3) a phosphate acyltransferase for catalyzing the conversion of propionyl-CoA to propionylphosphate;
(b4) a propionate kinase for catalyzing the conversion of propionylphosphate to propionic acid; and
(b5) optionally lactate dehydrogenase;

c) adenosine diphosphate and Pi;
d) coenzyme A and optionally pyruvate;
e) $NADP^+$,
f) a low-potential electron carrier selected from hexamethyl viologen, tetramethyl viologen, Ti(III) citrate, and Eu (II)-EGTA;

ii) applying electricity to the electrochemical cell for conversion of the electron carrier into its reduced form, and
iii) producing adenosine triphosphate from the AAA cycle and electricity.

In a more particular embodiment, the AAA cycle further comprises glucose and (b6) hexokinase, and step iii) further comprises producing glucose-6-phosphate.
In a still more particular embodiment, the method further comprises the steps:

iv) obtaining the AAA cycle reaction mixture comprising adenosine triphosphate;
v') using the AAA cycle reaction mixture of step iv) for cell-free *in-vitro* production of nucleic acids.

Preferably, step v') above further comprises providing a transcription system comprising CTP, GTP, UTP, a thermostable inorganic pyrophosphatase, an adenylate kinase inhibitor, a T7 RNA polymerase, a DNA template.
In another more particular embodiment, the method further comprises the steps:

iv) obtaining the AAA cycle reaction mixture comprising adenosine triphosphate;
v") using the AAA cycle reaction mixture of step iv) for cell-free *in vitro* production of proteins.

Preferably, step v") further comprises providing a protein synthesis system comprising transcription factors, translational factors, aminoacyl-tRNA synthetases, ribosome, amino acids, CTP, GTP, TTP, a DNA template, and wherein said protein synthesis system does not comprises ATP and ATP regenerating enzymes.
In preferred embodiments, step ii) comprises applying electricity to the electrochemical cell at potential between -0.57 to -0.53 mV *versus* SHE (Standard hydrogen Electrode) preferably for 4 - 5 h.

**Description of the invention**

Surprisingly it has been found that a method for producing adenosine triphosphate (ATP) through an acid/-aldehyde-ATP (AAA) cycle in an electrochemical cell comprising the steps:

i) providing an AAA cycle reaction mixture in the electrochemical cell, the AAA cycle comprising:

a) a carboxylic acid or a salt thereof,
b) a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2) a CoA-acetylating aldehyde dehydrogenase for catalyzing the conversion of the aldehyde into the corresponding CoA ester;
(b3) a phosphate acyltransferase for catalyzing the conversion of the CoA ester to the corresponding acyl phosphate;
(b4) a carboxylic acid kinase for catalyzing the conversion of the acyl phosphate to the corresponding carboxylic acid;

or a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2-3) a phosphate reductase for catalyzing the conversion of the aldehyde to the corresponding acyl phosphate;
(b4) a carboxylic acid kinase for catalyzing the conversion of the acyl phosphate to the corresponding carboxylic acid;

c) adenosine diphosphate (ADP) and inorganic phosphate (Pi);
d) coenzyme A (CoA);
e) $NAD^+$ or $NADP^+$,
f) a low-potential electron carrier;

ii) applying electricity to the electrochemical cell for conversion of the electron carrier into its reduced form, and
iii) producing adenosine triphosphate from the AAA cycle and electricity,

solves the above objective.

Definitions

The term **"$NAD^+$"** or **"nicotinamide adenine dinucleotide",** as used herein, refers to the well-known coenzyme central to metabolism. $NAD^+$ is the oxidized form and can be reduced to its reduced form NADH (H for hydrogen).
The term **"$NADP^+$"** or **"nicotinamide adenine dinucleotide phosphate",** as used herein, refers to the well-known cofactor used in anabolic reactions, such as the Calvin cycle and lipid and nucleic acid syntheses, which require NADPH as a reducing agent. NADPH is the reduced form of $NADP^+$, which is the oxidized form.
The term **"ATP"** or **"adenosine triphosphate",** as used herein, refers to the well-known organic compound that provides energy to drive and support many processes in living cells, such as muscle contraction, nerve impulse propagation, condensate dissolution, and chemical synthesis.
The term **"aldehyde",** as used herein, refers to an organic compound containing a functional group with the structure R-CH=O. Propionaldehyde or propanal is the organic compound with the formula $CH_3CH_2CHO$. Acetaldehyde or ethanal is an organic chemical compound with the formula $CH_3CHO$.
The term **"carboxylic acid",** as used herein, refers to an organic acid that contains a carboxyl group (C(=O)OH) attached to an R-group. The general formula of a carboxylic acid is R-COOH or R-$CO_2H$, with R referring to the alkyl, alkenyl, aryl, or other group. Deprotonation of a carboxylic acid gives a carboxylate anion. Propionic acid is a naturally occurring carboxylic acid with chemical formula $CH_3CH_2CO_2H$. The anion $CH_3CH_2CO_2^-$ as well as the salts and esters of propionic acid are known as propionates. Acetic acid or ethanoic acid is an organic compound with the chemical formula $CH_3COOH$. Acetate is the ion resulting from loss of $H^+$ from acetic acid. The name "acetate" can also refer to a salt containing this anion, or an ester of acetic acid.
Preferably, a carboxylic acid is selected from the group comprising or consisting of **short chain fatty acids, medium chain fatty acids, glyceric acid, glyceric acid derivatives, benzoic acid, benzoic acid derivatives, aromatic acids.**
**Short-chain fatty acids** (SCFAs) are fatty acids of one to five carbon atoms. **Medium-chain fatty acids** (MCFAs) are fatty acids of six to twelve carbon atoms. Exemplary **SCFAs and MCFAs** are formic acid, acetic acid, propionic acid, butyric acid, valeric acid, crotonic acid, glutaric acid, caproic acid, caprylic acid, capric acid, and lauric acid.
**Glyceric acid** (IUPAC name = 2,3-Dihydroxypropanoic acid) refers to an organic compound with the formula $HOCH_2CH(OH)CO_2H$.
Exemplary **glyceric acid derivatives** are 2-phosphoglyceric acid, 3-phosphoglyceric acid, 2,3-biphosphoglyceric acid, and 1,3-biphosphoglyceric acid.
**Aromatic acids** include compounds that contain a COOH group bonded to an aromatic ring. The simplest aromatic acid is benzoic acid.
Exemplary **aromatic acids** are benzoic acid, benzoic acid derivatives, phenylacetic acid (toluic acid), terephthalic acid, cinnamic acid, salicylic acid, gallic acid, phtalic acid, isophtalic acid.
Exemplary **benzoic acids** derivatives are 2-aminobenzoic acid, methylbenzoic acid, hydroxybenzoic acid, fluorobenzoic acid, 4-methoxy-benzoic acid, 4-hydroxy-3-methoxy-benzoic acid, 3,5-dimethoxy-4-hydroxy-benzoic acid, and 3,4-dimethoxy-5-hydroxy-benzoic acid.
Therefore, a more preferred **carboxylic acid** is selected from the group comprising or consisting of formic acid, acetic acid, propionic acid, butyric acid, valeric acid, crotonic acid, glutaric acid, caproic acid, caprylic acid., capric acid, lauric acid, glyceric acid, 2-phosphoglyceric acid, 3-phosphoglyceric acid, 2,3-biphosphoglyceric acid, and 1,3-biphosphoglyceric acid, benzoic acid, 2-aminobenzoic acid, methylbenzoic acid, hydroxybenzoic acid, fluorobenzoic acid, 4-methoxy-benzoic acid, 4-hydroxy-3-methoxy-benzoic acid, 3,5-dimethoxy-4-hydroxy-benzoic acid, and 3,4-dimethoxy-5-hydroxy-benzoic acid, phenylacetic acid (toluic acid), terephthalic acid, cinnamic acid, salicylic acid, gallic acid, phtalic acid, isophtalic acid.

The term **"low-potential electron carrier",** as used herein, refers to an electron mediator with a redox potential lower than -400 mV. Preferred low-potential electron carriers are viologen derivatives, such as tetramethyl viologen or hexamethyl viologen, having redox potentials lower than -500 mV, and suitable for catalyzing the reduction of carboxylic acids to the corresponding aldehydes. Further suitable low-potential electron carriers for the invention disclosed herein are Ti(III) citrate, and Eu (II)-EGTA.

## Enzymes of the AAA cycle

The term **"AOR", "aldehyde ferredoxin oxidoreductase"** or **"ferredoxin dependent aldehyde oxidoreductase"** (enzyme b1 of the AAA cycle), as used herein, refers to an enzyme (EC 1.2.7.5) capable of catalyzing the chemical reaction: an aldehyde + $H_2O$ + 2 oxidized ferredoxin $\rightleftharpoons$ an acid + 2 $H^+$ + 2 reduced ferredoxin. This enzyme belongs to the family of oxidoreductases, specifically those acting on the aldehyde or oxo group of donor with an iron-sulfur protein as acceptor and is also called AOR. It is a relatively rare example of a tungsten-containing protein. The enzyme family includes AOR, formaldehyde ferredoxin oxidoreductase (FOR), glyceraldehyde-3-phosphate ferredoxin oxidoreductase (GAPOR), all isolated from hyperthermophilic archea; carboxylic acid reductase found in clostridia; and hydroxycarboxylate viologen oxidoreductase from Proteus vulgaris, the sole member of the AOR family containing molybdenum. Most W-dependent enzymes of the AOR family described to date are from hyperthermophilic archaea. For example, five hyperthermophilic and extremely O2-sensitive W-enzymes of the AOR family are encoded in the genome of Pyrococcus furiosus, which have all been described as aldehyde oxidoreductases of various specificities and represent five separate subfamilies in a phylogenetic tree of the AOR family enzymes. The most important of these are the homodimeric aldehyde oxidoreductases (AOR sensu stricto), FOR, and GAPOR. Two further tungsten-containing oxidoreductases, WOR-4 and WOR-5) have been purified. Further orthologs of these enzymes have also been characterized from various other hyperthermophilic archaeal species, such as *Thermococcus litoralis, T. paralvinellae, Methanobacterium thermoautotrophicum* or *Pyrobaculum aerophilum.* In addition, they also have been described in some bacteria as "carboxylic acid reductase" (CAR, e.g., in *Moorella thermoacetica*), or as AORs from *Clostridium formicoaceticum, Eubacterium acidaminophilum or Desulfovibrio gigas.* An enzyme of the AOR family from the anaerobic thermophilic bacterial genus Caldicellulosiruptor has been identified as a member of a new subclass called XOR. Finally, a separate branch of W-dependent enzymes of the AOR family was discovered in obligatory anaerobic aromatic-degrading bacteria, which were identified as benzoyl-CoA reductases. A tungsten-dependent AOR-like enzyme was detected in the denitrifying *betaproteobacterium Aromatoleum aromaticum* EbN1. The enzyme is induced during anaerobic growth on phenylalanine (Phe) and a number of other substrates, although the degradation of aldehydes in the respective pathways occurs mainly via $NAD(P)^+$-dependent dehydrogenases.

The term **"mutated aldehyde dehydrogenase"** (enzyme b1' of the AAA cycle) refers to an enzyme for catalyzing the conversion of the carboxylic acid to the corresponding aldehyde. The term mutated **"aldehyde dehydrogenase"** (EC 1.2.1.3 and EC 1.2.1.4) refers to an engineered version of aldehyde dehydrogenase that can use low-potential electron carriers, such as NADH or NADPH derivatives, to convert the carboxylic acid to its corresponding aldehyde.

The term **"CoA-acetylating aldehyde dehydrogenase", ALDH,** EC 1.2.1.10, (enzyme b2 of the AAA cycle), as used herein, refers to an enzyme capable of catalyzing the chemical reaction: an aldehyde + CoA + $NAD(P)^+$ $\rightleftharpoons$ an acyl-CoA + NAD(P)H + $H^+$. For example, PduP-NP is a CoA-acetylating aldehyde dehydrogenase used in the propanediol pathway for the conversion of propionaldehyde to propionyl-CoA. PduP-NP (SEQ ID NO 9) represents a mutated form of the wildtype RpPduP from *Rhodopseudomonas palustris* BisB18. PduP-NP has been mutated to accept $NADP^+$ as electron acceptor (Trudeau DL, et al., 2018. Design and in vitro realization of carbon-conserving photorespiration. Proc Natl Acad Sci USA. 115(49):E11455-E11464).

The term **"phosphate reductase"** (enzyme b2-3 of the AAA cycle), as used herein, refers to an enzyme capable of catalyzing the chemical reaction: an aldehyde + phosphate + $NAD(P)^+$ $\rightleftharpoons$ an acyl-phosphate + NAD(P)H + $H^+$. A preferred **"phosphate reductase"** for the method disclosed herein is selected from the group comprising a glyceraldehyde-3-phosphate dehydrogenase (phosphorylating) EC 1.2.1.12, a glyceraldehyde-3-phosphate dehydrogenase ($NADP^+$) (phosphorylating) EC 1.2.1.13, a N-acetyl-gamma-glutamyl-phosphate reductase EC 1.2.1.38, a glyceraldehyde-3-phosphate dehydrogenase ($NAD(P)^+$) (phosphorylating) EC 1.2.1.59. In some embodiments, a preferred **"phosphate reductase"** for the method disclosed herein is an enzyme having promiscuous formylphosphate reductase activity selected from N-acetyl-γ-glutamyl phosphate dehydrogenase (ArgC), glyceraldehyde-3-phosphate dehydrogenase (GapA), aspartate semialdehyde dehydrogenase (ASD), γ-glutamyl phosphate reductase (ProA), N-acetyl-γ-glutamyl phosphate dehydrogenase (ArgC), mutant *Denitrovibrio acetiphilus N*-acetyl-γ-glutamyl phosphate dehydrogenase

(DaArgC1), mutant DaArgC2, mutant DaArgC3 (Nattermann, M., et al. 2023). A formylphosphate reductase (Fpr) is a phosphate reductase which can convert formaldehyde to formylphosphate. (Nattermann, M., et al. 2023. *Engineering a new-to-nature cascade for phosphate-dependent formate to formaldehyde conversion in vitro and in vivo.* Nat Commun 14, 2682). A "promiscuous enzyme activity" refers to an activity of an enzyme to catalyze a secondary reaction different from the reaction for which it has been specialized. In some embodiments, a preferred **"phosphate reductase"** for the method disclosed herein is an enzyme having promiscuous formylphosphate reductase activity selected from *Escherichia coli* N-acetyl-γ-glutamyl phosphate dehydrogenase (EcArgC), *Escherichia coli* glyceraldehyde-3-phosphate dehydrogenase (EcGapA), *Escherichia coli* aspartate semialdehyde dehydrogenase (EcASD), *Escherichia coli* γ-glutamyl phosphate reductase (EcProA), *Denitrovibrio acetiphilus* N-acetyl-γ-glutamyl phosphate dehydrogenase (DaArgC), mutant DaArgC1, mutant DaArgC2, mutant DaArgC3 (Nattermann, M., et al. 2023).

The term **"phosphate acyltransferase",** (enzyme b3 of the AAA cycle), as used herein, refers to an enzyme capable of catalyzing the chemical reaction: an acyl-CoA + phosphate $\rightleftharpoons$ an acyl-phosphate + CoA. The term "**phosphate acyltransferase**" refers to an enzyme of the general EC class 2.3.1. A preferred "**phosphate acyltransferase"** for the method disclosed herein is selected from the group comprising a phosphate acetyltransferase EC 2.3.1.8 (Pta), phosphate butyryltransferase EC 2.3.1.19, phosphate propanoyltransferase EC 2.3.1.222. For example, Pta (Uniprot P0A9M8, EC: 2.3.1.8) can convert acetyl-CoA to acetylphosphate. It can also convert propionyl-CoA to propinylphosphate.

The term **"carboxylic acid kinase",** EC: 2.7.2, (enzyme b4 of the AAA cycle) refers to an enzyme catalyzing the conversion of the acyl phosphate to the corresponding carboxylic acid. An exemplary **"carboxylic acid kinase"** is propionate kinase TdcD (Uniprot: P11868, EC 2.7.2.15) from *E. coli* which catalizes conversion of propionylphosphate to propionic acid.

The term **"lactate dehydrogenase"** or LDH, EC 1.1.1.27, (enzyme b5 of the AAA cycle) as used herein, refers to an enzyme capable of catalyzing the conversion of NADPH to $NADP^+$, and more in particular the chemical reaction: pyruvate + NADPH + $H^+$ = lactate + $NADP^+$.

The term " **hexokinase",** EC 2.7.1.1 (enzyme b6 added to the AAA cycle) refers to an enzyme capable of catalyzing the chemical reaction : ATP + D-hexose = ADP + D-hexose 6-phosphate.

The term **"glucokinase",** EC 2.7.1.2 (enzyme b6 added to the AAA cycle) refers to an enzyme capable of catalyzing the chemical reaction: ATP + D-glucose = ADP + D-glucose 6-phosphate.

**"Suitable reaction conditions"** refer to those conditions in the biocatalytic reaction mixture (e.g., ranges of enzyme loading, substrate loading, cofactor loading, temperature, pH, buffers, co-solvents, etc.) under which ATP is produced through the AAA cycle as described herein. Exemplary "suitable reaction conditions" are provided in the present disclosure and are illustrated by the Examples.

**"Cofactor regeneration system**" or "cofactor recycling system" refers to a set of reactants that participate in a reaction that reduces the oxidized form of the cofactor (e.g., $NADP^+$ to NADPH). Cofactor regeneration systems comprise a stoichiometric reductant that is a source of reducing hydrogen equivalents and is capable of reducing the oxidized form of the cofactor. The cofactor regeneration system may further comprise a catalyst, for example an enzyme catalyst that catalyzes the reduction of the oxidized form of the cofactor by the reductant. Cofactor regeneration systems to regenerate NADH from $NAD^+$ or NADPH from $NADP^+$, respectively, are known in the art and may be used in the methods described herein.

The complex biochemistry and thermodynamic constraints of the naturally evolved membrane-based systems or comparable vesicle-based derivatives have restricted the use of external electricity to power cell-free and/or in vitro systems. To overcome these challenges, the inventors sought to design an interface between electricity and biological systems that would provide a more direct way from electrical energy into ATP. Specifically, the inventors aimed to realize a new-to-nature electrobiological module that (1) comprises a minimal set of biological components, (2) does not involve membrane-bound charge separation, (3) is able to operate at energetic efficiencies compared to (or even better than) naturally evolved systems, and (4) can be coupled to other *in vitro* modules (Fig. 3).

The term **"electrochemical cell with two compartments"** or **"two compartments electrochemical cell"** refers to an "electrochemical cell" comprising a cathodic compartment, an anodic compartment, and a proton-conducting membrane, wherein the proton-conducting membrane physically separates the cathodic and anodic compartments.

**"AAA cycle"**

The term **"acid/aldehyde ATP cycle"** or **"AAA cycle"** refers to a minimum set of enzymes for the direct conversion of electrical energy into ATP. In some embodiments, the AAA cycle contains a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2) a CoA-acetylating aldehyde dehydrogenase for catalyzing the conversion of the aldehyde into the corresponding

CoA ester;

(b3) a phosphate acyltransferase for catalyzing the conversion of the CoA ester to the corresponding acyl phosphate;
(b4) a carboxylic acid kinase for catalyzing the conversion of the acyl phosphate to the corresponding carboxylic acid;
(b5) optionally lactate dehydrogenase;

and carboxylic acid or a salt thereof, adenosine diphosphate (ADP), inorganic phosphate (Pi); coenzyme A (CoA), optionally pyruvate; NAD or $NADP^+$, a low-potential electron carrier.

In other embodiments, the AAA cycle described above contains a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2-3) a phosphate reductase for catalyzing the conversion of the aldehyde to the corresponding acyl phosphate;
(b4) a carboxylic acid kinase for catalyzing the conversion of the acyl phosphate to the corresponding carboxylic acid;
(b5) optionally lactate dehydrogenase.

In further embodiments, the AAA cycle described above is a $AAA_P$ cycle containing a set of enzymes comprising:

(b1) a ferredoxin-dependent aldehyde oxidoreductase for catalyzing the conversion of propionic acid to propionaldehyde;
(b2) a NADPH-dependent CoA-acetylating aldehyde dehydrogenase for catalyzing the conversion of proprionaldehyde into propionyl-CoA,
(b3) a phosphate acyltransferase for catalyzing the conversion of propionyl-CoA to propionylphosphate;
(b4) a propionate kinase for catalyzing the conversion of propionylphosphate to propionic acid; and
(b5) optionally lactate dehydrogenase;

and the reaction mixture contains propionic acid, ADP, Pi, coenzyme A and optionally pyruvate; $NADP^+$, a low-potential electron carrier selected from hexamethyl viologen, tetramethyl viologen, Ti(III) citrate, and Eu (II)-EGTA.

In further embodiments, the $AAA_P$ cycle described above contains a set of enzymes comprising:

(b1) $AOR_{Aa}$;
(b2) PduP-NP (SEQ ID NO: 9),
(b3) Pta from *E.coli;*
(b4) TdcD from *E. coli*; and
(b5) optionally lactate dehydrogenase;

and the reaction mixture contains propionic acid, ADP, Pi, coenzyme A and optionally pyruvate; $NADP^+$, a low-potential electron carrier selected from hexamethyl viologen, tetramethyl viologen, Ti(III) citrate, and Eu (II)-EGTA.

**Design of the AAA cycle**

To realize a minimal electrobiological module, the inventors first looked into possible ways to generate ATP from ADP and inorganic phosphate (Pi) enzymatically. Besides membrane-bound ATP synthase, several membrane-independent enzyme systems have been described that convert the energy stored in mixed phosphoanhydrides (e.g., acetate kinase, 3-phosphoglycerate kinase), enol phosphates (pyruvate kinase), or thioesters (GDP-forming succinyl-CoA synthetase, ADP-forming 4-hydoxybutyryl-CoA synthetase) into ATP under release of the free acid, making them prime targets for the ATP-forming step.

However, to continuously employ these ATP-generating reactions in an electrobiological module, the free acids need to be recycled back to the substrates through electrochemical activation. Several enzymes have been described that use low-potential electrons to directly reduce free acids into their aldehydes. The energy stored in the aldehyde can be subsequently employed to oxidatively generate phospho-acid anhydrides, either directly or via concomitant coenzyme A (CoA) thioester formation and successive transfer of the phospho-group. Overall, this closes a 3-4 reaction cycle, in which low-potential electrons are used to drive the formation of an ATP (as well as a NAD(P)H) through an acid/aldehyde redox couple (acid/aldehyde-ATP cycle, AAA cycle, Fig. 2)

**Realization of the AAA cycle**

To realize the AAA cycle, the inventors focused on identifying an enzyme candidate for the reduction of non-activated carboxylic acids into their corresponding aldehydes, which is thermodynamically challenging due to the very low

redox potential of acid/aldehyde couples (E'° = -580 mV). Ferredoxin-dependent aldehyde oxidoreductases (AORs) have been shown to catalyze this reaction *in vivo* with reduced ferredoxin, and *in vitro* with low-potential electron carriers such as reduced tetramethyl viologen (TMV) ($E'^0$ = -536 mV), Ti (III) citrate ($E'^0$ = -800 mV), or Eu (II)-EGTA (E'° = -1,100 mV). AORs are classified into several branches, including formaldehyde ferredoxin oxidoreductase (FOR), glyceraldehyde-3-phosphate ferredoxin oxidoreductase (GAPOR), and broad-substrate range aldehyde ferredoxin oxidoreductase (WORs or bona fide AORs). Thus, various AAA cycles can be designed based on different AORs (Fig. 4).

Recently, a tungsten-containing AOR from the mesophilic betaproteobacterium *Aromatoleum aromaticum* strain EbN1 ($AOR_{Aa}$, NCBI Ref. Seq. aorA: WP_011238651, aorB: WP_011238652, aorC: WP_011238653) has been isolated and characterized in detail. This enzyme catalyzes the oxidation of different aldehydes, including benzoate, acetaldehyde, and propionaldehyde, with either benzyl viologen (BV) or $NAD^+$ as electron acceptors. Compared to related enzymes from other microorganisms, $AOR_{Aa}$ is highly active at ambient temperatures, remarkably stable after exposure to air (>1 h half-life) and can be conveniently purified from a homologous expression system. Based on the favorable characteristics and availability of $AOR_{Aa}$, the inventors decided to use this protein to realize an AAA cycle with acetate or propionate. To catalyze the acid reduction reaction, the inventors sought to use hexamethyl viologen (HMV, E'0 = -610 mV) as a soluble electron carrier and thermodynamically favorable conditions, including a low pH (pH=6), high substrate concentration (60 mM free acid), as well as constant removal of the aldehyde product with phenylhydrazine. Under these conditions, $AOR_{Aa}$ catalyzed the reduction of acetate and propionate into the corresponding aldehydes (demonstrated as their phenylhydrazones, Fig. 5, 6) at 72 $\pm$ 3 mU/mg and 57 $\pm$ 3 mU/mg, respectively (Fig. 7).

Next, the inventors aimed at coupling $AOR_{Aa}$ with a CoA-acetylating aldehyde dehydrogenase (Ald) to further convert the aldehydes into their corresponding CoA esters. Note that $AOR_{Aa}$ accepts $NAD^+$ as electron acceptor, but not $NADP^+$. To avoid direct transfer of electrons from reduced HMV onto NAD+ by $AOR_{Aa}$, which would shortcut the AAA cycle, the inventors sought to employ an NADPH-dependent Ald. The inventors used a CoA-acylating propionaldehyde dehydrogenase (PduP-NP) from *Rhodopseudomonas palustris* BisB18 that was recently engineered to accept $NADP^+$ as electron acceptor. Kinetic characterization of PduP-NP demonstrated that the enzyme showed better catalytic properties for propionaldehyde ($K_M$ = 0.77 mM, $V_{max}$ = 9.8 U/mg) compared to acetaldehyde ($K_M$ = 2.0 mM and $V_{max}$ = 2.8 U/mg) (Fig. 8). The inventors decided to realize the AAA cycle first based on propionate ($AAA_P$ cycle).

When $AOR_{Aa}$ and PduP-NP were coupled, the inventors could demonstrate the formation of propionyl-CoA from propionate, using LC-MS. Adding pyruvate and lactate dehydrogenase (Ldh) to recycle NADPH further improved the yield (Fig. 9). To further convert propionyl-CoA into propionylphosphate, the inventors decided to use phosphate acetyltransferase (Pta) from *E. coli*. For the ATP production step (i.e., converting propionylphosphate into propionate), the inventors tested acetate kinase (AckA) and propionate kinase (TdcD) from *E. coli* and chose TdcD because of its favorable kinetic parameters with propionyl-phosphate (Fig. 10).

With all enzymes for the $AAA_P$ cycle at hand, the inventors aimed at assembling the complete cycle. The inventors first verified that the PduP-NP, Pta, and TdcD cascade produced ATP from propionaldehyde under the working conditions of $AOR_{Aa}$ (i.e., pH=6 in the presence of 60 mM propionate, Fig. 11). The inventors then reconstructed the full cycle with $AOR_{Aa}$, used Ti(III)-reduced HMV as electron donor, and added 60 mM propionate to start the cycle. Initial tests showed that the negative control without propionate also produced ATP, which we could trace back to myokinase contamination (Fig. 12). Increasing the purity of protein preparations by additional size exclusion chromatography together with the addition of myokinase inhibitor, P1,P5-di(adenosine-5')pentaphosphate (AP5A), suppressed background ATP formation in the controls. Under these optimized conditions, the $AAA_P$ cycle produced about 30 $\mu$M ATP in 80 minutes from Ti(III)-reduced HMV (Fig. 13).

**Coupling of the AAA cycle with electricity**

Having established the $AAA_P$ cycle with reduced HMV, the inventors aimed at coupling it to an electrochemical cell to directly power ATP formation from electricity. In the initial setup, all three electrodes of the electrochemical cell (working, counter, and reference electrode) were placed in a single compartment. Although HMV was readily reduced and oxidized at the working electrode (Fig. 14), the purple color characteristic of reduced HMV quickly disappeared after turning off electricity, indicating that the HMV reduced by electricity was not stable. Moreover, the inventors also observed that the three-electrode setup negatively affected the AOR reaction (Fig. 15). The inventors therefore assumed that the counter electrode produced oxygen and/or reactive oxygen species (ROS), from water splitting, which would be detrimental for both reduced HMV and AOR. Therefore, the inventors switched to an H-cell type, two-compartment electrochemical cell with a proton-conducting Nafion membrane, to physically separate the cathodic and anodic compartments. With this setup, reduced HMV was functionally stable and could be used as mediator to power the production of 57 $\mu$M ATP in 45 minutes (Fig. 16) in a discontinuous fashion (i.e., when the $AAA_P$ cycle was subsequently added to the reaction mixture).

The inventors next aimed at a continuous operation of the $AAA_P$ cycle in the two-compartment system (Fig. 17). Initial experiments demonstrated that continuous ATP-regeneration was indeed possible and directly depended on the

current applied (Fig. 18). However, an excess of reduced HMV lowered ATP yield (Fig. 19), likely due to the direct reduction of $NADP^+$ at high concentrations of reduced HMV. The inventors thus decreased the HMV reduction rate by operating the system between - 0.57 to -0.53 mV vs. SHE. Under these conditions, the reaction mixture showed a very light purple color (at least for the initial 2 hours), indicating low steady-state concentrations of reduced HMV in the system. With this setup, the inventors achieved continuous ATP production from electricity with up to 165 $\mu$M ATP produced in 5 hours. The steady-state (first hour) production rate was 1.03 $\mu mol\ cm^{-2}\ h^{-1}$ with a faradaic efficiency for HMV reduction of about 69%-80% (Fig. 20) and a 22% faradaic efficiency for ATP production (Fig. 21, 22).

Thus, the present invention relates to a method for producing adenosine triphosphate (ATP) through an acid/aldehyde-ATP (AAA) cycle in an electrochemical cell comprising the steps:

i) providing an AAA cycle reaction mixture in the electrochemical cell, the AAA cycle comprising:

a) a carboxylic acid or a salt thereof,
b) a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2) a CoA-acetylating aldehyde dehydrogenase for catalyzing the conversion of the aldehyde into the corresponding CoA ester;
(b3) a phosphate acyltransferase for catalyzing the conversion of the CoA ester to the corresponding acyl phosphate;
(b4) a carboxylic acid kinase for catalyzing the conversion of the acyl phosphate to the corresponding carboxylic acid,

or a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2-3) a phosphate reductase for catalyzing the conversion of the aldehyde to the corresponding acyl phosphate;
(b4) a carboxylic acid kinase for catalyzing the conversion of the acyl phosphate to the corresponding carboxylic acid;

c) adenosine diphosphate (ADP) and inorganic phosphate (Pi);
d) coenzyme A (CoA);
e) NAD or $NADP^+$,
f) a low-potential electron carrier;

ii) applying electricity to the electrochemical cell for conversion of the electron carrier into its reduced form, and
iii) producing adenosine triphosphate from the AAA cycle and electricity.

With different words, the present invention relates to a method for producing adenosine triphosphate (ATP) through an acid/aldehyde-ATP (AAA) cycle in an electrochemical cell comprising the steps:

i) providing an AAA cycle reaction mixture in the electrochemical cell, the AAA cycle comprising:

a) a carboxylic acid or a salt thereof,
b) a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2) a CoA-acetylating aldehyde dehydrogenase for catalyzing the conversion of the aldehyde into the corresponding CoA ester;
(b3) a phosphate acyltransferase for catalyzing the conversion of the CoA ester to the corresponding acyl phosphate;
(b4) a carboxylic acid kinase for catalyzing the conversion of the acyl phosphate to the corresponding carboxylic acid,

or a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2-3) a phosphate reductase for catalyzing the conversion of the aldehyde to the corresponding acyl phosphate;
(b4) a carboxylic acid kinase for catalyzing the conversion of the acyl phosphate to the corresponding carboxylic acid;

c) adenosine diphosphate (ADP) and inorganic phosphate (Pi);
d) coenzyme A (CoA);
e) $NAD^+$ or $NADP^+$,
f) a low-potential electron carrier;

ii) applying a current to the electrochemical cell for conversion of the electron carrier into its reduced form, and
iii) producing adenosine triphosphate from the AAA cycle in the electrochemical cell.

Preferably, the present invention relates to a method for producing adenosine triphosphate (ATP) through an acid/aldehyde-ATP (AAA) cycle in an electrochemical cell comprising the steps:

i) providing an AAA cycle reaction mixture in the electrochemical cell, the AAA cycle comprising:

a) a carboxylic acid or a salt thereof,
b) a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2) a CoA-acetylating aldehyde dehydrogenase for catalyzing the conversion of the aldehyde into the corresponding CoA ester;
(b3) a phosphate acyltransferase for catalyzing the conversion of the CoA ester to the corresponding acyl phosphate;
(b4) a carboxylic acid kinase for catalyzing the conversion of the acyl phosphate to the corresponding carboxylic acid;

c) adenosine diphosphate (ADP) and inorganic phosphate (Pi);
d) coenzyme A (CoA);
e) $NAD^+$ or $NADP^+$,
f) a low-potential electron carrier;

ii) applying electricity to the electrochemical cell for conversion of the electron carrier into its reduced form, and
iii) producing adenosine triphosphate from the AAA cycle and electricity.

Moreover, the present invention also relates to a method for producing adenosine triphosphate (ATP) through an acid/aldehyde-ATP (AAA) cycle in an electrochemical cell comprising the steps:

i) providing an AAA cycle reaction mixture in the electrochemical cell, the AAA cycle comprising:

a) a carboxylic acid or a salt thereof,
b) a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2-3) a phosphate reductase for catalyzing the conversion of the aldehyde to the corresponding acyl phosphate;
(b4) a carboxylic acid kinase for catalyzing the conversion of the acyl phosphate to the corresponding carboxylic acid;

c) adenosine diphosphate (ADP) and inorganic phosphate (Pi);

d) coenzyme A (CoA);
e) NAD or $NADP^+$,
f) a low-potential electron carrier;

ii) applying electricity to the electrochemical cell for conversion of the electron carrier into its reduced form, and
iii) producing adenosine triphosphate from the AAA cycle and electricity.

More preferably, the present invention relates to a method for producing adenosine triphosphate (ATP) through an acid/aldehyde-ATP (AAA) cycle in an electrochemical cell comprising the steps:

i) providing an AAA cycle reaction mixture in the electrochemical cell, the AAA cycle comprising:

a) a carboxylic acid or a salt thereof,
b) a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2) a CoA-acetylating aldehyde dehydrogenase for catalyzing the conversion of the aldehyde into the corresponding CoA ester;
(b3) a phosphate acyltransferase for catalyzing the conversion of the CoA ester to the corresponding acyl phosphate;
(b4) a carboxylic acid kinase for catalyzing the conversion of the acyl phosphate to the corresponding carboxylic acid,

or a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2-3) a phosphate reductase for catalyzing the conversion of the aldehyde to the corresponding acyl phosphate;
(b4) a carboxylic acid kinase for catalyzing the conversion of the acyl phosphate to the corresponding carboxylic acid;

c) adenosine diphosphate (ADP) and inorganic phosphate (Pi);
d) coenzyme A (CoA);
e) $NAD^+$ or $NADP^+$,
f) a low-potential electron carrier;

ii) applying electricity to the electrochemical cell for conversion of the electron carrier into its reduced form, and
iii) producing adenosine triphosphate from the AAA cycle and electricity, and

wherein the carboxylic acid is selected from the group comprising short to medium chain fatty acids, glyceric acids, glyceric acid derivatives, benzoic acids, benzoic acid derivatives, aromatic acids.

In preferred embodiments, the set of enzymes further comprises (b5) a lactate dehydrogenase for catalyzing the conversion of NADPH to $NADP^+$, and wherein the reaction mixture further comprises pyruvate. In preferred embodiments, step ii) comprises applying electricity or a current to the electrochemical cell at potential between -0.57 to -0.53 mV *versus* SHE (Standard hydrogen Electrode) preferably for 4 - 5 h. In preferred embodiments, the ferredoxin-dependent aldehyde oxidoreductase is a tungsten-containing aldehyde oxidoreductase from the mesophilic betaproteobacterium *Aromatoleum aromaticum* EbN1 $AOR_{Aa}$. In preferred embodiments, the CoA-acetylating aldehyde dehydrogenase is a NADPH-dependent CoA-acetylating aldehyde dehydrogenase, and $NADP^+$ is provided in step e). In preferred embodiment, the CoA-acetylating aldehyde dehydrogenase is PduP-NP (SEQ ID NO: 9), and/or the phosphate acyltransferase is phosphate acetyltransferase Pta from *E. coli*; and the carboxylic acid kinase is proprionate kinase TdcD from E. *coli.* In preferred embodiments, the carboxylic acid is acetic acid or propionic acid or acetate or propionate, more preferably propionic acid or propionate. In preferred embodiments, the low-potential electron carrier is selected from a viologen derivative, hexamethyl viologen, tetramethyl viologen, Ti(III) citrate, and Eu (II)-EGTA. In preferred embodiments, the electrochemical cell comprises a cathodic compartment, an anodic compartment, and a proton-conducting membrane, wherein the proton-conducting membrane physically separates the cathodic and anodic compartments.

Thus, the present invention relates to a method for producing adenosine triphosphate (ATP) through an

acid/aldehyde-ATP (AAA) cycle in an electrochemical cell comprising the steps:

i) providing an AAA cycle reaction mixture in the electrochemical cell, the AAA cycle comprising:

a) a carboxylic acid or a salt thereof,
b) a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2) a CoA-acetylating aldehyde dehydrogenase for catalyzing the conversion of the aldehyde into the corresponding CoA ester;
(b3) a phosphate acyltransferase for catalyzing the conversion of the CoA ester to the corresponding acyl phosphate;
(b4) a carboxylic acid kinase for catalyzing the conversion of the acyl phosphate to the corresponding carboxylic acid;
(b5) a lactate dehydrogenase for catalyzing the conversion of NADPH to $NADP^+$;

or a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2-3) a phosphate reductase for catalyzing the conversion of the aldehyde to the corresponding acyl phosphate;
(b4) a carboxylic acid kinase for catalyzing the conversion of the acyl phosphate to the corresponding carboxylic acid;
(b5) a lactate dehydrogenase for catalyzing the conversion of NADPH to $NADP^+$;

c) adenosine diphosphate (ADP) and inorganic phosphate (Pi);
d) coenzyme A (CoA) and pyruvate;
e) $NAD^+$ or $NADP^+$,
f) a low-potential electron carrier;

ii) applying electricity to the electrochemical cell for conversion of the electron carrier into its reduced form, and
iii) producing adenosine triphosphate from the AAA cycle and electricity,

wherein the carboxylic acid is preferably selected from the group comprising short to medium chain fatty acids, glyceric acids, glyceric acid derivatives, benzoic acids, benzoic acid derivatives, aromatic acids.

In preferred embodiments, step ii) comprises applying electricity to the electrochemical cell at potential between -0.57 to -0.53 mV *versus* SHE (Standard hydrogen Electrode) preferably for 4 - 5 h. In preferred embodiments, the ferredoxin-dependent aldehyde oxidoreductase is a tungsten-containing aldehyde oxidoreductase from the mesophilic betaproteobacterium *Aromatoleum aromaticum* EbN1 $AOR_{Aa}$. In preferred embodiments, the CoA-acetylating aldehyde dehydrogenase is a NADPH-dependent CoA-acetylating aldehyde dehydrogenase, and $NADP^+$ is provided in step e). In preferred embodiments, the carboxylic acid is acetic acid or propionic acid or acetate or propionate, more preferably propionic acid or propionate. In preferred embodiment, the CoA-acetylating aldehyde dehydrogenase is PduP-NP (SEQ ID NO: 9), and/or the phosphate acyltransferase is phosphate acetyltransferase Pta from *E. coli*; and the carboxylic acid kinase is propionate kinase TdcD from E. *coli.* In preferred embodiments, the low-potential electron carrier is selected from a viologen derivative, hexamethyl viologen, tetramethyl viologen, Ti(III) citrate, and Eu (II)-EGTA. In alternative embodiments, the electrochemical cell comprises a cathodic compartment, an anodic compartment, and a proton-conducting membrane, wherein the proton-conducting membrane physically separates the cathodic and anodic compartments.

Thus, the present invention preferably relates to a method for producing adenosine triphosphate (ATP) through an acid/aldehyde-ATP ($AAA_P$) cycle in an electrochemical cell comprising the steps:

i) providing an $AAA_P$ cycle reaction mixture in the electrochemical cell, the $AAA_P$ cycle comprising:

a) propionic acid or propionate;
b) a set of enzymes comprising:

(b1) a ferredoxin-dependent aldehyde oxidoreductase for catalyzing the conversion of propionic acid to

propionaldehyde;
(b2) a NADPH-dependent CoA-acetylating aldehyde dehydrogenase for catalyzing the conversion of proprionaldehyde into propionyl-CoA,
(b3) a phosphate acyltransferase for catalyzing the conversion of propionyl-CoA to propionylphosphate;
(b4) a propionate kinase for catalyzing the conversion of propionylphosphate to propionic acid; and
(b5) optionally lactate dehydrogenase;

c) adenosine diphosphate and inorganic phosphate;
d) coenzyme A and optionally pyruvate;
e) $NADP^+$,
d) an electron carrier selected from hexamethyl viologen, tetramethyl viologen, Ti(III) citrate, and Eu (II)-EGTA;

ii) applying electricity to the electrochemical cell for conversion of the electron carrier into its reduced form, and
iii) producing adenosine triphosphate from the AAA cycle and electricity.

In preferred embodiments, step ii) comprises applying electricity to the electrochemical cell at potential between -0.57 to -0.53 mV *versus* SHE (Standard hydrogen Electrode) preferably for 4 - 5 h. In alternative embodiments, the electrochemical cell comprises a cathodic compartment, an anodic compartment, and a proton-conducting membrane, wherein the proton-conducting membrane physically separates the cathodic and anodic compartments.

With other words, the present invention relates to a method for producing adenosine triphosphate (ATP) through an acid/aldehyde-ATP ($AAA_P$) cycle in an electrochemical cell comprising the steps:

i) providing an $AAA_P$ cycle reaction mixture in the electrochemical cell, the $AAA_P$ cycle reaction mixture comprising:

a) propionic acid or propionate;
b) a set of enzymes comprising:

(b1) a ferredoxin-dependent aldehyde oxidoreductase for catalyzing the conversion of propionic acid to propionaldehyde;
(b2) a NADPH-dependent CoA-acetylating aldehyde dehydrogenase for catalyzing the conversion of proprionaldehyde into propionyl-CoA,
(b3) a phosphate acyltransferase for catalyzing the conversion of propionyl-CoA to propionylphosphate;
(b4) a propionate kinase for catalyzing the conversion of propionylphosphate to propionic acid; and
(b5) optionally lactate dehydrogenase;

c) adenosine diphosphate and inorganic phosphate;
d) coenzyme A and optionally pyruvate;
e) $NADP^+$,
d) a low-potential electron carrier selected from hexamethyl viologen, tetramethyl viologen, Ti(III) citrate, and Eu (II)-EGTA;

ii) applying **a current to** the electrochemical cell for conversion of the electron carrier into its reduced form, and
iii) producing adenosine triphosphate from the AAA cycle in the electrochemical cell.

In preferred embodiments, step ii) comprises applying a current to the electrochemical cell at potential between -0.57 to -0.53 mV *versus* SHE (Standard hydrogen Electrode) preferably for 4 - 5 h.

The present invention more preferably relates to a method for producing adenosine triphosphate (ATP) through an acid/aldehyde-ATP ($AAA_P$) cycle in an electrochemical cell comprising the steps:

i) providing an $AAA_P$ cycle reaction mixture in the electrochemical cell, the $AAA_P$ cycle comprising:

a) propionic acid or propionate;
b) a set of enzymes comprising:

(b1) $AOR_{Aa}$;
(b2) PduP-NP (SEQ ID NO: 9);
(b3) Pta from *E.coli;*
(b4) TdcD from *E. coli*; and

(b5) optionally lactate dehydrogenase;

c) adenosine diphosphate and inorganic phosphate;
d) coenzyme A and optionally pyruvate;
e) $NADP^+$,
d) an electron carrier selected from hexamethyl viologen, tetramethyl viologen, Ti(III) citrate, and Eu (II)-EGTA;

ii) applying electricity to the electrochemical cell for conversion of the electron carrier into its reduced form, and
iii) producing adenosine triphosphate from the AAA cycle and electricity.

In preferred embodiments, step ii) comprises applying electricity to the electrochemical cell at potential between -0.57 to -0.53 mV *versus* SHE (Standard hydrogen Electrode) preferably for 4 - 5 h. In alternative embodiments, the electrochemical cell comprises a cathodic compartment, an anodic compartment, and a proton-conducting membrane, wherein the proton-conducting membrane physically separates the cathodic and anodic compartments.

**Application of the AAA cycle for energy and information storage**

Having demonstrated the direct production of ATP from electricity with the $AAA_P$ cycle, the inventors sought to couple the minimal electrobiological module of the present invention to different *in vitro* systems.

***In situ* production of glucose-6-phosphate**

The inventors first coupled the $AAA_P$ cycle to hexokinase for *in situ* production of glucose 6-phosphate (G6P). G6P is used as a redox and energy carrier for different biocatalytic systems, and is an important building block for energy storage in biological molecules, such as starch or glycogen. In the coupled system about 400 μM G6P was produced in 4 hours with a steady state (first hour) production rate of 2.71 μmol $cm^{-2}$ $h^{-1}$ and at 47% overall faradaic efficiency (Fig. 23). This showed that coupling of ATP production to downstream molecules (G6P) creates sinks that increase ATP production efficiencies so that the efficiency of the electrobiological module of the present invention (41%) becomes comparable to those of the naturally existing membrane-bound energy conversion ($\eta$ = 40-70%).
Thus, in a preferred embodiment, the AAA cycle further comprises glucose and (b6) hexokinase, and step iii) further comprises producing glucose-6-phosphate. In other words, the AAA cycle further comprises glucose and (b6) glucokinase, and step iii) further comprises producing glucose-6-phosphate.
Thus, in a preferred embodiment, the AAA cycle reaction mixture further comprises glucose and (b6) hexokinase, preferably a (b6) glucokinase.
Thus, in a preferred embodiment, the method further comprises the steps:
iii) adding glucose and a (b6) hexokinase to the AAA cycle reaction mixture for *in situ* production of glucose-6-phosphate.
Therefore, a preferred embodiment of the invention is directed to a method for producing adenosine triphosphate (ATP) through an acid/aldehyde-ATP (AAA) cycle in an electrochemical cell comprising the steps:

i) providing an AAA cycle reaction mixture in the electrochemical cell, the AAA cycle comprising:

a) a carboxylic acidor a salt thereof, and glucose;
b) a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2) a CoA-acetylating aldehyde dehydrogenase for catalyzing the conversion of the aldehyde into the corresponding CoA ester;
(b3) a phosphate acyltransferase for catalyzing the conversion of the CoA ester to the corresponding acyl phosphate;
(b4) a carboxylic acid kinase for catalyzing the conversion of the acyl phosphate to the corresponding carboxylic acid;
(b5) optionally a lactate dehydrogenase;
(b6) a hexokinase;

or a set of enzymes comprising:

(b1) a ferredoxin-dependent aldehyde oxidoreductase or (b1') a mutated aldehyde dehydrogenase for

catalyzing the conversion of the carboxylic acid to the corresponding aldehyde;
(b2-3) a phosphate reductase for catalyzing the conversion of the aldehyde to the corresponding acyl phosphate;
(b4) a carboxylic acid kinase for catalyzing the conversion of the acyl phosphate to the corresponding carboxylic acid;
(b5) optionally a lactate dehydrogenase;
(b6) a hexokinase;

c) adenosine diphosphate and Pi;
d) coenzyme A and optionally pyruvate;
e) $NAD^+$ or $NADP^+$,
f) a low-potential electron carrier;

ii) applying electricity to the electrochemical cell for conversion of the electron carrier into its reduced form, and
iii) producing adenosine triphosphate and glucose-6-phosphate from the AAA cycle and electricity,

wherein the carboxylic acid is preferably selected from the group comprising short chain fatty acids, medium chain fatty acids, glyceric acid, glyceric acid derivatives, benzoic acid, benzoic acid derivatives, aromatic acids.

In preferred embodiments, step ii) comprises applying electricity to the electrochemical cell at potential between -0.57 to -0.53 mV *versus* SHE (Standard hydrogen Electrode) preferably for 4 - 5 h. In preferred embodiments, the ferredoxin-dependent aldehyde oxidoreductase is a tungsten-containing aldehyde oxidoreductase from the mesophilic betaproteobacterium *Aromatoleum aromaticum* EbN1 $AOR_{Aa}$. In preferred embodiments, the CoA-acetylating aldehyde dehydrogenase is a NADPH-dependent CoA-acetylating aldehyde dehydrogenase, and $NADP^+$ is provided in step e). In preferred embodiments, the carboxylic acid is acetic acid or propionic acid or acetate or propionate, more preferably propionic acid or propionate. In preferred embodiment, the CoA-acetylating aldehyde dehydrogenase is PduP-NP (SEQ ID NO: 9), and/or the phosphate acyltransferase is phosphate acetyltransferase Pta from *E. coli*; and the carboxylic acid kinase is propionate kinase TdcD from *E. coli.* In preferred embodiments, the low-potential electron carrier is selected from a viologen derivative, hexamethyl viologen, tetramethyl viologen, Ti(III) citrate, and Eu (II)-EGTA. In alternative embodiments, the electrochemical cell comprises a cathodic compartment, an anodic compartment, and a proton-conducting membrane, wherein the proton-conducting membrane physically separates the cathodic and anodic compartments.

A more preferred embodiment of the invention is directed to a method for producing adenosine triphosphate (ATP) through an acid/aldehyde-ATP (AAA) cycle in an electrochemical cell comprising the steps:

i) providing an $AAA_P$ cycle reaction mixture in the electrochemical cell, the $AAA_P$ cycle comprising:

a) propionic acid or propionate, and glucose;
b) a set of enzymes comprising:

(b1) a ferredoxin-dependent aldehyde oxidoreductase for catalyzing the conversion of propionic acid to propionaldehyde, preferably $AOR_{Aa}$;
(b2) a NADPH-dependent CoA-acetylating aldehyde dehydrogenase for catalyzing the conversion of proprionaldehyde into propionyl-CoA, preferably PduP-NP (SEQ ID NO: 9);
(b3) a phosphate acyltransferase for catalyzing the conversion of propionyl-CoA to propionylphosphate, preferably Pta from *E.coli;*
(b4) a propionate kinase for catalyzing the conversion of propionylphosphate to propionic acid, preferably TdcD from *E. coli*; and
(b5) optionally a lactate dehydrogenase;
(b6) a hexokinase;

c) adenosine diphosphate and Pi;
d) coenzyme A and optionally pyruvate;
e) $NAD^+$ or $NADP^+$,
f) a low-potential electron carrier selected from a viologen derivative, hexamethyl viologen, tetramethyl viologen, Ti(III) citrate, and Eu (II)-EGTA;

ii) applying electricity to the electrochemical cell for conversion of the electron carrier into its reduced form, and
iii) producing adenosine triphosphate and glucose-6-phosphate from the AAA cycle and electricity.

In preferred embodiments, step ii) comprises applying electricity to the electrochemical cell at potential between -0.57 to -0.53 mV *versus* SHE (Standard hydrogen Electrode) preferably for 4 - 5 h. In alternative embodiments, the electrochemical cell comprises a cathodic compartment, an anodic compartment, and a proton-conducting membrane, wherein the proton-conducting membrane physically separates the cathodic and anodic compartments.

**Use of the AAA cycle for powering nucleic acid transcription or transcription and translation.**

Next, the inventors aimed at powering more complex *in vitro* systems with the electrobiological module of the present invention. One of the most fundamental processes in biology is information processing, in particular transcription (TX) and translation (TL) of nucleic acids. To demonstrate $AAA_P$ cycle-powered TX, we used a modified RNA Output Sensor Activated by Ligand Induction (ROSALIND) platform. In this TX system, a fluorogenic dye-binding RNA aptamer (3WJdB, "Broccoli") is expressed by T7 RNA polymerase under consumption of nucleotide triphosphates including ATP (Fig. 24A). The inventors run the $AAA_P$ cycle for 5 h and added the reaction mixture into above TX system (lacking ATP) at a 1:10 dilution to detect a visible RNA-output corresponding to about 20 $\mu$M ATP (Fig. 24B). These results demonstrated that the ATP produced by the $AAA_P$ cycle is incorporated into RNA, successfully linking biological information processing with electricity.

Thus, in a preferred embodiment, the method further comprises the steps:

iv) obtaining the AAA cycle reaction mixture comprising adenosine triphosphate;
v') using the AAA cycle reaction mixture of step iv) for cell-free *in-vitro* production of nucleic acid.

Thus, in a preferred embodiment, the method further comprises the steps:
iii) use of the AAA cycle reaction mixture of step iii) comprising adenosine triphosphate for cell-free *in-vitro* production of nucleic acids.

Thus, in a preferred embodiment, the method further comprises the steps:

iv) obtaining the AAA cycle reaction mixture comprising adenosine triphosphate;
v') using the AAA cycle reaction mixture of step iv) for cell-free *in-vitro* production of nucleic acids,

wherein step v') further comprises providing a transcription system comprising CTP, GTP, UTP, a thermostable inorganic pyrophosphatase, an adenylate kinase inhibitor, a T7 RNA polymerase, a DNA template.

Therefore, a preferred embodiment of the invention is directed to a method for producing adenosine triphosphate (ATP) through an acid/aldehyde-ATP (AAA) cycle in an electrochemical cell comprising the steps:

i) providing an AAA cycle reaction mixture in the electrochemical cell, the AAA cycle comprising:

a) a carboxylic acidor a salt thereof;
b) a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2) a CoA-acetylating aldehyde dehydrogenase for catalyzing the conversion of the aldehyde into the corresponding CoA ester;
(b3) a phosphate acyltransferase for catalyzing the conversion of the CoA ester to the corresponding acyl phosphate;
(b4) a carboxylic acid kinase for catalyzing the conversion of the acyl phosphate to the corresponding carboxylic acid;
(b5) optionally a lactate dehydrogenase;
(b6) a hexokinase;

or a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2-3) a phosphate reductase for catalyzing the conversion of the aldehyde to the corresponding acyl phosphate;
(b4) a carboxylic acid kinase for catalyzing the conversion of the acyl phosphate to the corresponding carboxylic acid;

(b5) optionally a lactate dehydrogenase;

c) adenosine diphosphate and Pi;
d) coenzyme A and optionally pyruvate;
e) $NAD^+$ or $NADP^+$,
f) a low-potential electron carrier;

ii) applying electricity to the electrochemical cell for conversion of the electron carrier into its reduced form, and
iii) producing adenosine triphosphate from the AAA cycle and electricity,
iv) obtaining the AAA cycle reaction mixture comprising adenosine triphosphate;
v') using the AAA cycle reaction mixture of step iv) for cell-free *in-vitro* production of nucleic acids, preferably providing a transcription system comprising CTP, GTP, UTP, a thermostable inorganic pyrophosphatase, an adenylate kinase inhibitor, a T7 RNA polymerase, a DNA template.

In some embodiments, the carboxylic acid is preferably selected from the group comprising short chain fatty acids, medium chain fatty acids, glyceric acid, glyceric acid derivatives, benzoic acid, benzoic acid derivatives, aromatic acids. In preferred embodiments, step ii) comprises applying electricity or a current to the electrochemical cell at potential between -0.57 to -0.53 mV *versus* SHE (Standard hydrogen Electrode) preferably for 4 - 5 h. In preferred embodiments, the ferredoxin-dependent aldehyde oxidoreductase is a tungsten-containing aldehyde oxidoreductase from the mesophilic betaproteobacterium *Aromatoleum aromaticum* EbN1 $AOR_{Aa}$. In preferred embodiments, the CoA-acetylating aldehyde dehydrogenase is a NADPH-dependent CoA-acetylating aldehyde dehydrogenase, and $NADP^+$ is provided in step e). In preferred embodiment, the CoA-acetylating aldehyde dehydrogenase is PduP-NP (SEQ ID NO: 9), and/or the phosphate acyltransferase is phosphate acetyltransferase Pta from E. *coli;* and the carboxylic acid kinase is proprionate kinase TdcD from E. *coli.* In preferred embodiments, the carboxylic acid is acetic acid or propionic acid or acetate or propionate, more preferably propionic acid or propionate. In preferred embodiments, the low-potential electron carrier is selected from a viologen derivative, hexamethyl viologen, tetramethyl viologen, Ti(III) citrate, and Eu (II)-EGTA. In alternative embodiments, the electrochemical cell comprises a cathodic compartment, an anodic compartment, and a proton-conducting membrane, wherein the proton-conducting membrane physically separates the cathodic and anodic compartments.

A still more preferred embodiment of the invention is directed to a method for producing adenosine triphosphate (ATP) through an acid/aldehyde-ATP (AAA) cycle in an electrochemical cell comprising the steps:

i) providing an $AAA_P$ cycle reaction mixture in the electrochemical cell, the $AAA_P$ cycle comprising:

a) propionic acid or propionate;
b) a set of enzymes comprising:

(b1) a ferredoxin-dependent aldehyde oxidoreductase for catalyzing the conversion of propionic acid to propionaldehyde, preferably $AOR_{Aa}$;
(b2) a NADPH-dependent CoA-acetylating aldehyde dehydrogenase for catalyzing the conversion of proprionaldehyde into propionyl-CoA, preferably PduP-NP (SEQ ID NO: 9);
(b3) a phosphate acyltransferase for catalyzing the conversion of propionyl-CoA to propionylphosphate, preferably Pta from *E.coli;*
(b4) a propionate kinase for catalyzing the conversion of propionylphosphate to propionic acid, preferably TdcD from E. *coli*; and
(b5) optionally a lactate dehydrogenase;

c) adenosine diphosphate and Pi;
d) coenzyme A and optionally pyruvate;
e) $NAD^+$ or $NADP^+$,
f) a low-potential electron carrier;

ii) applying electricity to the electrochemical cell for conversion of the electron carrier into its reduced form, and
iii) producing adenosine triphosphate from the AAA cycle and electricity,
iv) obtaining the AAA cycle reaction mixture comprising adenosine triphosphate;
v') using the AAA cycle reaction mixture of step iv) for cell-free *in-vitro* production of nucleic acids, preferably providing a transcription system comprising CTP, GTP, UTP, a thermostable inorganic pyrophosphatase, an adenylate kinase inhibitor, a T7 RNA polymerase, a DNA template.

In preferred embodiments, step ii) comprises applying electricity or a current to the electrochemical cell at potential between -0.57 to -0.53 mV *versus* SHE (Standard hydrogen Electrode) preferably for 4 - 5 h. In preferred embodiments, the low-potential electron carrier is selected from a viologen derivative, hexamethyl viologen, tetramethyl viologen, Ti(III) citrate, and Eu (II)-EGTA. In alternative embodiments, the electrochemical cell comprises a cathodic compartment, an anodic compartment, and a proton-conducting membrane, wherein the proton-conducting membrane physically separates the cathodic and anodic compartments.

In the next step, the inventors wanted to power both, transcription and translation (TX-TL) with the electrobiological module of the present invention to demonstrate the electrification of protein production from DNA templates. To establish such a system, the inventors made use of a custom-prepared cell-free TX-TL system, custom PUREfrex that lacks ATP and all enzymes for ATP regeneration. The inventors first optimized the system to detect the production of a green fluorescent protein (EGFP) or a luciferase (NanoLuc) from a DNA template, starting with ATP concentrations as low as 80 $\mu$M and ADP/ATP ratios as high as 4:1. The inventors then run the $AAA_P$ cycle for 5 h with electricity and transferred the reaction mixture in a 2:5 ratio into the PUREfrex system to produce either EGFP (Fig. 25) or NanoLuc Luciferase (Fig. 26). These experiments demonstrated the successful powering of *in vitro* protein production from electrically-generated ATP serving as an mRNA monomer, as well as an energy source. Together, these experiments showed that one of the core processes of biological systems can in principle directly powered by electricity.

Thus, in a preferred embodiment, the method further comprises the steps:

iv) obtaining the AAA cycle reaction mixture comprising adenosine triphosphate;
v") using the AAA cycle reaction mixture of step iv) for cell-free *in vitro* production of proteins.

Thus, in a preferred embodiment, the method further comprises the steps:
iv) use of the solution of step iii) comprising adenosine triphosphate for cell-free in *vitro* production of proteins.

Thus, in a preferred embodiment, the method further comprises the steps:

iv) obtaining the AAA cycle reaction mixture comprising adenosine triphosphate;
v") using the AAA cycle reaction mixture of step iv) for cell-free *in vitro* production of proteins,

wherein step v") further comprises providing a protein synthesis system comprising transcription factors, translational factors, aminoacyl-tRNA synthetases, ribosome, amino acids, CTP, GTP, TTP, a DNA template, and wherein said protein synthesis system does not comprises ATP and ATP regenerating enzymes.

Therefore, a preferred embodiment of the invention is directed to a method for producing adenosine triphosphate (ATP) through an acid/aldehyde-ATP (AAA) cycle in an electrochemical cell comprising the steps:

i) providing an AAA cycle reaction mixture in the electrochemical cell, the AAA cycle comprising:

a) a carboxylic acid or a salt thereof;
b) a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2) a CoA-acetylating aldehyde dehydrogenase for catalyzing the conversion of the aldehyde into the corresponding CoA ester;
(b3) a phosphate acyltransferase for catalyzing the conversion of the CoA ester to the corresponding acyl phosphate;
(b4) a carboxylic acid kinase for catalyzing the conversion of the acyl phosphate to the corresponding carboxylic acid;
(b5) optionally a lactate dehydrogenase;

or a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2-3) a phosphate reductase for catalyzing the conversion of the aldehyde to the corresponding acyl phosphate;
(b4) a carboxylic acid kinase for catalyzing the conversion of the acyl phosphate to the corresponding carboxylic acid;
(b5) optionally a lactate dehydrogenase;

c) adenosine diphosphate and Pi;
d) coenzyme A and optionally pyruvate;
e) $NAD^+$ or $NADP^+$,
f) a low-potential electron carrier;

ii) applying electricity to the electrochemical cell for conversion of the electron carrier into its reduced form, and
iii) producing adenosine triphosphate from the AAA cycle and electricity,
iv) obtaining the AAA cycle reaction mixture comprising adenosine triphosphate;
v") using the AAA cycle reaction mixture of step iv) for cell-free *in-vitro* production of proteins, preferably providing a protein synthesis system comprising transcription factors, translational factors, aminoacyl-tRNA synthetases, ribosome, amino acids, CTP, GTP, TTP, a DNA template, and wherein said protein synthesis system does not comprises ATP and ATP regenerating enzymes.

In some embodiments, the carboxylic acid is preferably selected from the group comprising short chain fatty acids, medium chain fatty acids, glyceric acid, glyceric acid derivatives, benzoic acid, benzoic acid derivatives, aromatic acids. In preferred embodiments, step ii) comprises applying electricity or a current to the electrochemical cell at potential between -0.57 to -0.53 mV *versus* SHE (Standard hydrogen Electrode) preferably for 4 - 5 h. In preferred embodiments, the ferredoxin-dependent aldehyde oxidoreductase is a tungsten-containing aldehyde oxidoreductase from the mesophilic betaproteobacterium *Aromatoleum aromaticum* EbN1 $AOR_{Aa}$. In preferred embodiments, the CoA-acetylating aldehyde dehydrogenase is a NADPH-dependent CoA-acetylating aldehyde dehydrogenase, and $NADP^+$ is provided in step e). In preferred embodiment, the CoA-acetylating aldehyde dehydrogenase is PduP-NP (SEQ ID NO: 9), and/or the phosphate acyltransferase is phosphate acetyltransferase Pta from *E. coli*; and the carboxylic acid kinase is proprionate kinase TdcD from *E. coli.* In preferred embodiments, the carboxylic acid is acetic acid or propionic acid or acetate or propionate, more preferably propionic acid or propionate. In preferred embodiments, the low-potential electron carrier is selected from a viologen derivative, hexamethyl viologen, tetramethyl viologen, Ti(III) citrate, and Eu (II)-EGTA. In alternative embodiments, the electrochemical cell comprises a cathodic compartment, an anodic compartment, and a proton-conducting membrane, wherein the proton-conducting membrane physically separates the cathodic and anodic compartments.

A still more preferred embodiment of the invention is directed to a method for producing adenosine triphosphate (ATP) through an acid/aldehyde-ATP (AAA) cycle in an electrochemical cell comprising the steps:

i) providing an $AAA_P$ cycle reaction mixture in the electrochemical cell, the $AAA_P$ cycle comprising:

a) propionic acid or propionate;
b) a set of enzymes comprising:

(b1) a ferredoxin-dependent aldehyde oxidoreductase for catalyzing the conversion of propionic acid to propionaldehyde, preferably $AOR_{Aa}$;
(b2) a NADPH-dependent CoA-acetylating aldehyde dehydrogenase for catalyzing the conversion of proprionaldehyde into propionyl-CoA, preferably PduP-NP (SEQ ID NO: 9);
(b3) a phosphate acyltransferase for catalyzing the conversion of propionyl-CoA to propionylphosphate, preferably Pta from *E.coli;*
(b4) a propionate kinase for catalyzing the conversion of propionylphosphate to propionic acid, preferably TdcD from *E. coli;* and
(b5) optionally a lactate dehydrogenase;

c) adenosine diphosphate and Pi;
d) coenzyme A and optionally pyruvate;
e) $NAD^+$ or $NADP^+$,
f) a low-potential electron carrier;

ii) applying electricity to the electrochemical cell for conversion of the electron carrier into its reduced form, and
iii) producing adenosine triphosphate from the AAA cycle and electricity,
iv) obtaining the AAA cycle reaction mixture comprising adenosine triphosphate;
v") using the AAA cycle reaction mixture of step iv) for cell-free *in-vitro* production of proteins, preferably providing a protein synthesis system comprising transcription factors, translational factors, aminoacyl-tRNA synthetases, ribosome, amino acids, CTP, GTP, TTP, a DNA template, and wherein said protein synthesis system does not comprises ATP and ATP regenerating enzymes.

In preferred embodiments, step ii) comprises applying electricity or a current to the electrochemical cell at potential between -0.57 to -0.53 mV *versus* SHE (Standard hydrogen Electrode) preferably for 4 - 5 h. In preferred embodiments, the low-potential electron carrier is selected from a viologen derivative, hexamethyl viologen, tetramethyl viologen, Ti(III) citrate, and Eu (II)-EGTA. In alternative embodiments, the electrochemical cell comprises a cathodic compartment, an anodic compartment, and a proton-conducting membrane, wherein the proton-conducting membrane physically separates the cathodic and anodic compartments.

**Description of the Figures**

Figure 1 shows ATP synthesis through oxidative phosphorylation, electrons from NADH and $FADH_2$ pass to $O_2$ through a series of redox centers in the electron transport chain (ETC), going from a higher energy level to a lower energy level, creating a proton gradient across the membrane. The electrochemical energy stored in this gradient then drives the synthesis of ATP as protons flow back through ATP synthase. In the scheme, a P/O ratio of 2.5 for NAD(P)H and 1.5 for $FADH_2$ is assumed.

Figure 2 shows ATP synthesis through the AAA cycle according to the present invention. Enzymatic conversion of an aldehyde to a carboxylic acid can either produce reduced ferredoxin, or NAD(P)H and ATP. Coupling the ferredoxin- and ATP/NAD(P)H-dependent branches allows to draft a cycle, in which a carboxylic acid is first reduced to an aldehyde using reduced ferredoxin as reducing equivalent, and then recycled back while generating NAD(P)H and ATP. The net reaction is converting an electron carrier of higher energy, reduced ferredoxin, to one of lower energy reducing power, NAD(P)H, while using the energy difference to produce ATP.

Figure 3 shows a technical implementation of the AAA cycle according to the present invention. A propionate-based AAA cycle converts electricity into ATP, which can power various *in vitro* systems as described herein. Suc, succinate; Fum, fumarate; $Fd^{2-}_{red}$, reduced ferredoxin; $Fd_{ox}$, oxidized ferredoxin; Med: low potential electron carrier.

Figure 4 shows four AAA cycle schemes. A AAA cycle designed based on FOR; B AAA cycle designed based on GAPOR; C, D AAA cycle designed based on bona fide AOR. The net reaction is $NAD(P)^+ + ADP + Pi + Fd^{2-}_{red} \rightarrow NAD(P)H + ATP + Fd_{ox}$. GAP, glyceraldehyde 3-phosphate; 1,3-BPG, 1,3-bisphosphoglycerate; 3-PG, 3-phosphoglycerate; FOR, formaldehyde ferredoxin oxidoreductase; Fpr, formylphosphate reductase; Ack, acetate kinase; GAPOR, glyceraldehyde-3-phosphate ferredoxin oxidoreductase; GapA, glyceraldehyde-3-phosphate dehydrogenase; Pgk, phosphoglycerate kinase; AOR, aldehyde ferredoxin oxidoreductase; Ald, acetaldehyde dehydrogenase (acetylating); Pta, phosphate acetyltransferase; PduP, propionaldehyde dehydrogenase (acetylating); TdcD, propionate kinase.

Figure 5 shows the verification of propionate reduction reaction of $AOR_{Aa}$ with LC-MS. Scheme of proprionate reduction reaction of $AOR_{Aa}$ is shown on the left. HMV is reduced by Ti(III)-citrate to serve as electron donor. Phenylhydrazine is added to drain away acetaldehyde. LC-MS analysis of the AOR propionate reduction reaction mixture with two negative controls is shown on the right. Reaction mixture in absence of $AOR_{Aa}$ or propionate is used as the negative control.

Figure 6 shows the verification of acetate reduction reaction of $AOR_{Aa}$ with LC-MS. Scheme of acetate reduction reaction of $AOR_{Aa}$ is shown on the left. HMV is reduced by Ti(III)-citrate to serve as electron donor. Phenylhydrazine is added to drain away acetaldehyde. LC-MS analysis of the AOR acetate reduction reaction mixture with two negative controls is shown on the right. The AOR acetate reduction reaction mixture (50 $\mu$l) contained 100 mM phosphate buffer pH 6, 5 mM HMV, 0.9 mM Ti(III)-citrate, 60 mM acetate, 10 mM phenylhydrazine, and 11 $\mu$g $AOR_{Aa}$. Negative controls were done in absence of $AOR_{Aa}$ or propionate. All reaction mixtures were quenched with 3% formic acid after reacting for 2 h at 30 °C. HMV, hexamethyl viologen; PH, phenylhydrazine; Ace-PH, acetaldehyde phenylhydrazone.

Figure 7 shows the specific activities of $AOR_{Aa}$ on propionate and acetate. Kinetic assay schemes are shown on the left. The data represent mean $\pm$ SD (standard deviation) determined from n = 3 independent measurements.

Figure 8 shows the Michaelis-Menten kinetics of PduP-NP on propionaldehyde and acetaldehyde. Kinetic assay schemes are shown on the left. The data represent mean ± SD (standard deviation) determined from n = 3 independent measurements.

Figure 9 shows that coupling AOR with PduP-NP enabled the conversion of propionate to propionyl-CoA. A Scheme of converting propionate to propionyl-CoA with AOR and PduP-NP. HMV is reduced by Ti(III)-citrate to serve as electron donor for AOR. B LC-MS analysis of AOR PduP-NP coupled reaction mixtures (with/without Ldh for NADPH recycling) and the negative control (without propionate). The reaction mixture (100 μl) contained 100 mM phosphate buffer pH 6, 5 mM HMV, 0.35 mM Ti(III)-citrate, 5 mM $MgCl_2$, 1 mM $NADP^+$, 1 mM ADP, 0.5 mM CoA, 0.5 mM pyruvate, 60 mM propionate, 0 μg or 3.3 μg Ldh, 18.6 μg $AOR_{Aa}$, and 17.9 μg PduP-NP. The negative control contained everything except propionate. 30 μl of mixtures were withdrawn and quenched with 3% formic acid after reacting at 30 °C for 0 min, 30 min, and 60 min.

Figure 10 shows the specific activities of Pta, TdcD, and AckA. Kinetic assay schemes are shown above. The data represent mean ± SD (standard deviation) determined from n = 3 independent measurements.

Figure 11 shows the ATP production of the PduP-NP, Pta, and TdcD cascade under the working conditions of $AOR_{Aa}$. Pyruvate and Ldh were added for NADPH recycling. As shown, the cascade produced ATP from propionaldehyde under the working conditions of $AOR_{Aa}$ (i.e., pH=6 with 60 mM propionate present), and recycling NADPH increased the yield.

Figure 12 shows myokinase contamination caused background ATP production. A Scheme of the $AAA_P$ cycle powered by Ti(III)-citrate reduced HMV. B Dynamics of ATP and AMP of the $AAA_P$ cycle over 40 min. As shown, the negative control in absence of propionate also produced ATP. Moreover, the AMP concentrations increased with time, indicating myokinase activities in the reaction mixtures. After adding the myokinase inhibitor, AP5A, the background ATP formation was suppressed (orange bars). The reaction mixture (100 μl) contained 100 mM phosphate buffer pH 6, 5 mM $MgCl_2$, 5 mM HMV, 0.4 mM Ti(III)-citrate, 1 mM $NADP^+$, 1 mM ADP, 0.25 mM CoA, 0.5 mM pyruvate, 60 mM propionate, 17.4 μg $AOR_{Aa}$, 3.6 μg PduP-NP, 1.4 μg Pta, 0.8 μg TdcD, and 1.7 μg Ldh. 0.2 mM AP5A was added to inhibit myokinase. ATP and AMP concentrations were quantified with LC-MS. AP5A, P1,P5-di(adenosine-5')pentaphosphate.

Figure 13 shows ATP production of the $AAA_P$ cycle powered by Ti(III) reduced HMV. Shown are the dynamics of ATP of the $AAA_P$ cycle over 80 min.

Figure 14 shows the characterization of hexamethyl viologen (HMV) by cyclic voltammetry with a glassy carbon electrode (0.07 $cm^2$ area) at various scan rates. The measurement was done in a two-compartment electrochemical cell with a Nafion membrane. The resulting data include the anodic peak potential ($E_{p,a}$), cathodic peak potential ($E_{p,c}$), peak-to-peak potential separation ($\Delta E_p$), redox midpoint potential (E'°), anodic peak current ($i_{p,a}$), and cathodic peak current ($i_{p,c}$) at different scan rates.

Figure 15 shows the negative effect of the single compartment electrochemical cell set-up on the AOR reaction. A Scheme of powering the propionate reduction reaction of AOR with electricity in a single compartment electrochemical cell. B LC-MS analysis of propionaldehyde phenylhydrazone (Pro-PH) production with AOR powered by electricity. After applying -0.745 V for 1 h, only a small amount of Pro-PH was produced (blue line). While, after turning off the electricity, a much larger amount of Pro-PH was produced with the electricity reduced HMV accumulated in the system (red line). This indicates the AOR reaction was inhibited with electricity in this system. At time 0 min (T0), the reaction mixture (300 μl) contained 100 mM phosphate buffer pH 6, 5 mM HMV, 10 mM phenylhydrazine, 60 mM propionate, and 55.8 μg $AOR_{Aa}$.

Figure 16 shows the ATP production of the $AAA_P$ cycle powered by electricity reduced HMV. A Scheme of the $AAA_P$ cycle powered by electricity reduced HMV. B Dynamics of ATP and AMP of the $AAA_P$ cycle over 45 min. AMP concentrations remained relatively constant, indicating the ATP production was not from myokinase activities. 5 mM HMV in 100 mM phosphate buffer pH 6 was first reduced by electricity to produce 1 mM reduced HMV, then other components were added. The reaction mixture (50 μl) contained 100 mM phosphate buffer pH 6, 0.85 mM reduced HMV, 3.4 mM HMV (oxidized form), 5 mM $MgCl_2$, 1 mM $NADP^+$, 1 mM ADP, 0.25 mM CoA, 0.5 mM pyruvate, 60 mM propionate, 0.2 mM AP5A, 13.3 μg $AOR_{Aa}$, 2.1 μg PduP-NP, 0.7 μg Pta, 0.4 μg TdcD, and 0.8 μg Ldh. ATP and AMP concentrations were

quantified with LC-MS. The data represent mean ± SD (standard deviation) determined from n = 3 independent experiments.

Figure 17 shows a scheme of the $AAA_P$ cycle producing ATP from electricity. In this cycle, AOR first converts propionate to propionaldehyde using HMV reduced by electricity. Propionaldehyde is further converted into propionate through three enzymatic steps, generating NADPH and ATP. Pyruvate and Ldh recycle NADPH to push the equilibrium towards the ATP-producing direction.

Figure 18 shows the electricity-dependent ATP production of the $AAA_P$ cycle. A Scheme of the $AAA_P$ cycle powered by electricity. B ATP production of the $AAA_P$ cycle powered with electricity using an on-off mode. The reaction mixture was placed in a two-compartment electrochemical cell, applying - 0.745 V with stirring in the center of the cell. The reaction mixture (600 μl) contained 100 mM phosphate buffer pH 6, 5 mM HMV, 5 mM $MgCl_2$, 1 mM $NADP^+$, 1 mM ADP, 0.25 mM CoA, 0.5 mM pyruvate, 60 mM propionate, 0.2 mM AP5A, 160 μg $AOR_{Aa}$, 23.9 μg PduP-NP, 8.4 μg Pta, 5.1 μg TdcD, and 10 μg Ldh. ATP concentrations were quantified with luciferin-luciferase assay.

Figure 19 shows excess reduced HMV decreased the ATP yield. A Chronoamperometry (CA) of the $AAA_P$ cycle under low or high current for 3h. B Electrosynthesis of ATP via the $AAA_P$ cycle under low or high current for 3h. Under low current condition, -0.745 V was applied with stirring in the center of the cell. Under high current condition, -0.8 V was applied with stirring directly under the working electrode. The reaction mixture quickly turned dark purple under the high current condition, indicating excess reduced HMV was accumulated. This decreased the ATP yield, probably due to the reduction of $NADP^+$ by reduced HMV at high concentrations. The reaction mixture (800 μl) contained 100 mM phosphate buffer pH 6, 5 mM HMV, 5 mM $MgCl_2$, 1 mM $NADP^+$, 1 mM ADP, 0.25 mM CoA, 0.5 mM pyruvate, 60 mM propionate, 0.2 mM AP5A, 200 μg $AOR_{Aa}$, 31.1 μg PduP-NP, 11.2 μg Pta, 6.8 μg TdcD, and 13.3 μg Ldh. ATP concentrations were quantified with luciferin-luciferase assay.

Figure 20 shows Faradaic efficiencies of reducing HMV at various potential. HMV (5 mM) was continuously reduced for 5-10 min at three different potentials (-0.57/-0.55/-0.53 V vs SHE) with stirring (at 1000 rpm) directly under the electrode. All measurements done with a glassy carbon electrode (0.07 $cm^2$ area) in a two-compartment electrochemical cell with 100 mM phosphate buffer pH 6. The data represent mean ± SD (standard deviation) determined from n = 3 independent measurements.

Figure 21 shows ATP production of the $AAA_P$ cycle over time, driven by electricity. The starting composition of the full cycle includes five enzymes (AOR, PduP-NP, Pta, TdcD, and Ldh), HMV, cofactors (CoA, $NADP^+$, ADP), propionate, pyruvate, and AP5A. Omitting AOR or applied electricity from the system did not result in ATP production and served as negative controls. ATP concentrations were quantified by the luciferin-luciferase assay. The same samples were also quantified with LC-MS.

Figure 22 shows the ATP production of the $AAA_P$ cycle over time, driven by electricity. The starting composition of the whole cycle includes five enzymes (AOR, PduP-NP, Pta, TdcD, and Ldh), HMV, cofactors (CoA, $NADP^+$, ADP), propionate, pyruvate, and AP5A. Whole cycle without applying electricity, and whole cycle except AOR with applying electricity were used as negative controls. Shown are ATP and AMP concentrations quantified with LC-MS. The AMP concentrations remained relatively constant, indicating no myokinase activities in reaction mixtures. The data represent mean ± SD (standard deviation) obtained in triplicate experiments (n = 3) except the no AOR control (n=1).

Figure 23 shows the coupling of electricity-produced ATP with G6P production from glucose. G6P was continuously produced from glucose and electricity when glucose and HK were coupled with the $AAA_P$ cycle. Omitting AOR or electricity from the system abolished G6P production.

Figure 24 shows the coupling of electricity-produced ATP with in vitro transcription (IVT). A Scheme of the used IVT system. T7 RNA polymerase transcription of the 3WJdB gene under consumption of NTPs. The 3WJdB aptamer binds the fluorogenic dye DFHBI-1T and thereby stabilizes its fluorescent confirmation in which DFHBI-1T emits light in the green spectra upon excitation at 472 nm. B The $AAA_P$ cycle produced ATP which was incorporate into RNA, while a negative control of the $AAA_P$ cycle without AOR failed to do so. The fluorescence output of the $AAA_P$ cycle corresponds to about 20 μM ATP. Kinetics are recorded at 37°C. Data shown are for n = 3 biological replicates ± SD (standard deviation) with raw fluorescence values standardized to micromolar equivalent fluorescein (MEF).

Figure 25 shows coupling of electricity-produced ATP with *in vitro* transcription (TX) and translation (TL). Shown is the fluorescence signal resulting from TX-TL of EGFP in a custom-prepared PURE system lacking ATP and energy-regenerating enzymes. The green curve is obtained by supplementing the PURE system with the full $AAA_P$ cycle, after applying electricity for 5 hours as sole source of ATP for performing TX-TL. In the negative control (grey curve) the PURE system was fed with an $AAA_P$ cycle setup lacking the AOR enzyme, after applying electricity for 5 hours. The data represent mean ± SD (standard deviation) obtained in triplicate experiments (n = 3) except for the w/o AOR controls in b and c (n=1).

Figure 26 shows coupling of electricity-produced ATP with *in vitro* transcription (TX) and translation (TL). Bar graphs show the luminescence signal obtained by performing TX-TL of NanoLuc luciferase in a custom-prepared PURE system (lacking ATP and energy regenerating enzymes) supplemented with the $AAA_P$ cycle, after applying electricity for 5 hours, as unique source of ATP. The experimental results (green bar) are compared with negative controls (dark gray bar) where the PURE system was fed with an $AAA_P$ cycle missing the AOR enzyme with applying electricity for 5 hours. The light grey bar represents the background signal of the luciferase assay reagent mixture. The data represent mean ± SD (standard deviation) obtained in triplicate experiments (n = 3). Nluc, NanoLuc luciferase.

Figure 27 shows $^1$H-NMR (400 MHz, $D_2O$) spectrum of chemically synthesized hexamethyl viologen chloride.

Figure 28 shows $^{13}$C-NMR (101 MHz, $D_2O$) spectrum of chemically synthesized hexamethyl viologen chloride.

Figure 29 shows chronoamperomtery (CA) of producing ATP from electricity through the $AAA_P$ cycle. The potential applied were adjusted according to the color of the reaction mixture to keep the reduced HMV (purple color) concentration low. The potentials were: -0.570 V vs. SHE for Stage 1;-0.560 V vs. SHE for Stage 2; -0.550 V vs. SHE for Stage 3; -0.540 V vs. SHE for Stage 4; -0.535 V vs. SHE for Stage 5; -0.530 V vs. SHE for Stage 6.

Figure 30 shows chronoamperomtery (CA) of producing G6P from electricity by coupling the AAAP cycle with hexokinase. The potential applied were adjusted according to the color of the reaction mixture to keep the reduced HMV (purple color) concentration low. The potentials were: -0.570 V vs. SHE for Stage 1; -0.565 V vs. SHE for Stage 2; -0.555 V vs. SHE for Stage 3; -0.545 V vs. SHE for Stage 4; -0.540 V vs. SHE for Stage 5;-0.530 V vs. SHE for Stage 6..

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

Further modifications and alternative embodiments of various aspects of the invention will be apparent to those skilled in the art in view of this description. Accordingly, this description is to be construed as illustrative only and is for the purpose of teaching those skilled in the art the general manner of carrying out the invention. It is to be understood that the forms of the invention shown and described herein are to be taken as examples of embodiments. Elements and materials may be substituted for those illustrated and described herein, parts and processes may be reversed, and certain features of the invention may be utilized independently, all as would be apparent to one skilled in the art after having the benefit of this description of the invention. Changes may be made in the elements described herein without departing from the spirit and scope of the invention as described in the following claims.

## EXAMPLES

### Materials

Chemicals used were purchased from Sigma-Aldrich and Carl Roth unless otherwise specified. Coenzyme A was obtained from Roche Diagnostics. Calbiochem® extra pure ADP (ATP impurity ≤ 0.2%) was purchased from Merck Millipore. Oligonucleotides were obtained from Eurofins Genomics Germany GmbH or Sigma-Aldrich. Synthesized genes (pTE28a-PduP-NP having SEQ ID NO 10, and gBlock encoding EGFP having SEQ ID NO 14) were obtained from Twist

Bioscience. Q5 Hot Start High-Fidelity DNA polymerase was purchased from New England Biolabs. Phusion High-Fidelity DNA polymerase was purchased from Thermo Fisher Scientific. Other materials for cloning and protein expression were obtained from Thermo Fisher Scientific, New England Biolabs, and Macherey-Nagel. Materials and equipment for protein purification were obtained from GE Healthcare, Bio-Rad, Macherey-Nagel, and Merck Millipore. ATP Bioluminescence Assay Kit CLS II was supplied from Roche. Custom PUREfrex™ Solution II Δ(MK, CK, NDK, PPiase) (enzymes), and PUREfrex™ Solution III (ribosomes) were supplied by Cosmo Bio USA, Inc. Nano-Glo® luciferase assay substrate, and Nano-Glo® luciferase assay buffer were supplied by Promega. tRNAs were purchased from Roche. L-lactate dehydrogenase (Ldh) (rabbit muscle), hexokinase (HK) (*Saccharomyces cerevisiae*), and glucose-6-phosphate dehydrogenase (GePDH) (*Leuconostoc mesenteroides*) were purchased from Sigma-Aldrich.

**Table 1: Nucleic acid sequences used in the experiments**

| **Primers** | **Description** | **Sequence** |
|---|---|---|
| SS322 PduP_NP f | PduP_NP amplification | SEQ ID NO 1 |
| SS323 PduP_NP r | PduP_NP amplification | SEQ ID NO 2 |
| SS324 JZ73 f | PduP_NP cloning | SEQ ID NO 3 |
| SS325 JZ73 r | PduP_NP cloning | SEQ ID NO 4 |
| oSB0021 | 3WJdB | SEQ ID NO 5 |
| oSB0022 | 3WJdB | SEQ ID NO 6 |
| EGFP-for | EGFP amplification | SEQ ID NO 7 |
| EGFP-rev | EGFP amplification | SEQ ID NO 8 |
| Gene construct | | |
| PduP-NP | Nucleic acid encoding PduP-NP from *R. palustris* BisB18 | SEQ ID NO 9 |
| | | |
| **Plasmids** | | |
| pAOR_1 | pBBR1 ori, PtetP::Strep-aorA-aorB-aorC-aorD-aorEAa, AmpR | Winiarska, A et al., 2022 |
| JZ73 | pET16b, Strep-PduPRp, AmpR | Zarzycki, J., et al., 2017 |
| pTE28a-PduP-NP | pET28a, His-PduPRp-NP, KanR | This study, SEQ ID NO 10 |
| pTE3259 | pET16b, Strep-PduPRp-NP, AmpR | This study, SEQ ID NO 11 |
| JW2294 | pCA24N, Pta, CamR, | ASKA collection, Kitagawa, M., et al., 2005 |
| JW2293 | pCA24N, AckA, CamR, | ASKA collection, Kitagawa, M., et al., 2005 |
| JW5806 | pCA24N, TdcD, CamR, | ASKA collection, Kitagawa, M., et al., 2005 |
| p3WJdB | ColE1 ori, PT7-tetO::3WJdB, AmpR | Gift from L. Brenker, SEQ ID NO 12 |
| pME-Nanoluc | ColE1 ori, PT7::Nanoluc, AmpR | Gift from R. Inckemann, SEQ ID NO 13 |
| gBlock encoding eGFP | | SEQ ID NO 14 |
| Kitagawa, M., et al. (2005). Complete set of ORF clones of Escherichia coli ASKA library (A complete set of E. coli K-12 ORF archive): unique resources for biological research. DNA Res. 12, 291-299.<br>Winiarska, A., et al. (2022). Tungsten Enzyme Using Hydrogen as an Electron Donor to Reduce Carboxylic Acids and NAD+. ACS Catal. 12, 8707-8717.<br>Zarzycki, J., et al. (2017). In Vitro Characterization and Concerted Function of Three Core Enzymes of a Glycyl Radical Enzyme - Associated Bacterial Microcompartment. Sci. Rep. 7, 1-12.<br>Legend: Aa, *Aromatoleum aromaticum;* Rp, *Rhodopseudomonas palustris* BisB18. | | |

## Example 1

### Synthesis of propionyl-CoA

Propionyl-CoA was synthesized from propionic anhydride and purified using a HPLC (1260 Infinity, Agilent Technologies GmbH) with a Gemini® 10μm NX-C18 110 Å Column (Phenomenex, Aschaffenburg, Germany) as described previously[38]. The concentration was quantified by determining the absorption at 260 nm ($\varepsilon$ = 16.4 $mM^{-1}$ $cm^{-1}$).

### Synthesis of Ti(III)-citrate

Ti(III)-citrate was synthesized by addition of 1.9 M Na citrate solution to a 12% solution of Ti(III)-chloride (CAS 7705-07-9, Riedel-deHaën, Seelze, Germany) in a molar ratio of 2:1. pH was adjusted by the successive addition of small amounts of NaHCOs until no further development of $CO_2$ could be observed.

### Synthesis of hexamethylviologen chloride (4,4'-Bipyridinium, 1,1',2,2',6,6'-hexamethyl-, chloride)

Tetramethyl bipyridine (0.8 g, 3.8 mmol) was slowly heated in dimethyl sulfate (6 mL) to 110 °C. After 1 hour, the cooled solution was poured into $Et_2O$ (20 mL) to precipitate the viologen product as a $MeSO_4^-$ salt which was filtered and washed with $Et_2O$. The $MeSO_4^-$ counterion was exchanged in two steps into $Cl^-$. First, the $MeSO_4^-$ counterion was exchanged to $PF_6^-$ by adding portion-wise $NH_4PF_6$ (6.2 g, 38 mmol) to a solution of the $MeSO_4^-$ viologen in water (10 mL). The reaction mixture was stirred at room temperature overnight. The $PF_6^-$ viologen product was filtered, washed with cold water and dried under a high vacuum. Finally, the counterion was exchanged into $Cl^-$ by adding tetrabutylammonium chloride (10.6 g, 38 mmol) to a solution of the $PF_6^-$ viologen in ACN (50 mL). The mixture was stirred overnight at room temperature. The product was filtered, washed with cold ACN and dried under a high vacuum. The viologen chloride was obtained with 41% yield (0.49 g, 1.6 mmol). $^1H$ NMR (400 MHz, $D_2O$) $\delta$/ppm: 8.18 (s, 4H, aromatic), 4.18 (s, 6H, N-$CH_3$), 2.93 (s, 12H, C-$CH_3$) (Fig. 27). $^{13}C$ NMR (101 MHz, $D_2O$) $\delta$/ppm: 157.32, 148.75, 125.45, 40.28, 21.50 (Fig. 28). The redox potential of the viologen derivative was measured using a three-electrode set up (glassy carbon working electrode, platinum counter electrode and Ag/AgCl 3.5 M reference electrode) in 100 mM phosphate buffer pH 6. A redox potential of -610 mV vs SHE was found for the single electron reduction step of the viologen dication into the radical cation state (Fig. 14). The reaction scheme for this synthesis is:

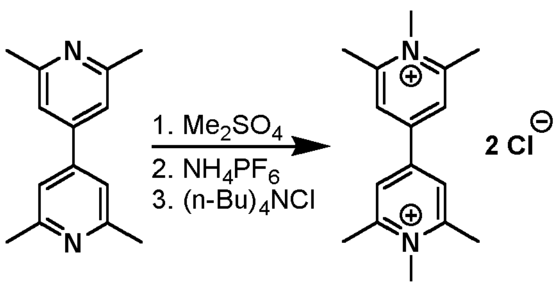


### Plasmid construction

pTE3259 (SEQ ID NO 11) was constructed using the Gibson isothermal DNA assembly method while DNA fragments were amplified by Q5 Hot Start High-Fidelity DNA polymerase. pJZ73 was used as the plasmid backbone. The insert was amplified from pTE28a-PduP-NP, which was synthesized by Twist Bioscience. Primers and plasmids used are listed in Table 1. All plasmids were kept in E. *coli* strain DH5α or Top10 for propagation and storage. For general molecular biology purposes, *E. coli* strains were grown in lysogeny broth (LB) with appropriate antibiotics at 37 °C. Antibiotics were used at the following concentrations: ampicillin (Amp), 100 μg/ml; kanamycin (Kan), 50 μg/ml; and chloramphenicol (Cam), 34 μg/ml.

### Recombinant expression and purification of AOR

Recombinant expression of AOR used *Aromatoleum evansii* KB740 as host containing the plasmid (pAOR_1) with the whole aor operon aorABCDE as described in Winiarska et al. 2022. The minimal medium used for cell cultivation contained 30 mM potassium phosphate buffer pH 7.8, 10 mM $NH_4Cl$, 0.8 mM $MgSO_4$, 0.15 mM $CaCl_2$, 1% trace element solution (14 mM Na-EDTA, 7.5 mM $FeSO_4$, 0.5 mM $MnCl_2$, 0.1 mM $CoCl_2$, 0.17 mM $CuCl_2$, 0.1 mM $NiCl_2$, 0.15 mM $Na_2MoO_4$, 0.5 mM $H_3BO_3$, 0.5 mM $ZnSO_4$, pH 6.5), 0.5% vitamin solution (100 mg/L cyanocobalamine, 300 mg/L

pyridoxamine dihydrochloride, 20 mg/L thiamine hydrochloride, 100 mg/L Ca-D-pantothenate, 80 mg/L 4-aminobenzoic acid, 20 mg/L D-biotin, 200 mg/L niacin) and 0.1% Se/W solution (10 mM NaOH, 23 μM $Na_2SeO_3$, 24 μM $Na_2WO_4$). Cells were either grown aerobically in Erlenmeyer flasks (2 L culture stirred in 5 L flasks) with 5 mM Na succinate as carbon source or under anaerobic conditions in a 30 L fermenter with 4 mM Na benzoate and 10 mM $KNO_3$. Cultivation, induction, cell harvest and cell lysis followed the procedure described in Winiarska et al. 2022. Cell lysis and all subsequent purification steps were carried out under anoxic conditions under protective gas atmosphere (97% $N_2$, 3% $H_2$). AOR was purified from the cell free extract via Streptactin affinity chromatography (IBA Lifesciences, Göttingen, Germany) using a FPLC system. After loading the extract onto the column, the enzyme was rinsed with stock buffer (100 mM Tris/HCl pH 8.0, 150 mM KCl, 10% (w/v) glycerol) and eluted with stock buffer additionally containing 2 mM desthiobiotin. Further purification via size exclusion chromatography was carried out in SEC buffer (50 mM TRIS/HCl pH 8.0, 100 mM KCl, 10% (w/v) glycerol) and used a HiLoad Superdex 200 prep grade column (GE Healthcare Life Sciences).

**Recombinant expression and purification of PduP-NP**

For heterologous overexpression of PduP-NP, plasmid pTE3259 was transformed into chemically competent *E. coli* BL21 cells. The cells were grown on LB agar plates containing 100 μg $ml^{-1}$ ampicillin at 37 °C overnight. Next day, the colonies obtained from the overnight culture were inoculated in 0.5 L terrific broth (TB) medium and grown at 37 °C to an OD600 of 0.4 to 1.0, induced with 0.25 mM isopropyl-β-D-thiogalactoside (IPTG). Then, cells were grown overnight at 25 °C. Cells were harvested at 6000 × g for 12 min at 4 °C and cell pellets were stored at -20 °C until purification. For purification, cell pellets were resuspended in two-fold volume of buffer Strep (50 mM HEPES, 150 mM NaCl, pH 7.8, 5 mM $MgCl_2$, 10 μg $ml^{-1}$ DNase I), lysed by ultrasonification and centrifuged at 50,000 × g and 4 °C for 40 min. The supernatant was filtered through a 0.45 μm filter, loaded onto a 1 ml StrepTrap™ HP column (GE Healthcare). After sufficient washing (until UV baseline is nearly at 0), the protein was eluted with buffer Strep containing 2.5 mM desthiobiotin. Further purification via size exclusion chromatography was carried out in SEC buffer (50 mM HEPES, 200 mM NaCl, 10% glycerol) and used a Superdex 200 increase 10/300GL column (GE Healthcare Life Sciences). Purified proteins were concentrated with Amicon centrifugal filters (Merck Millipore) and stored in 50% glycerol at -20 °C. Protein concentrations were determined by the Bradford protein assay.

**Recombinant expression and purification of Pta, AckA, and TdcD**

For overexpression of Pta (UniProt: P0A9M8), AckA (acetate kinase A and propionate kinase 2, UniProt: P0A6A3), and TdcD (UniProt: P11868) from *E. coli,* the corresponding plasmids from the ASKA collection were transformed into chemically competent E. *coli* BL21 cells. The cells were grown on LB agar plates containing 34 μg $ml^{-1}$ chloramphenicol at 37 °C overnight. Next day, the colonies obtained from the overnight culture were inoculated in 0.5 L terrific broth (TB) medium and grown at 37 °C to an OD600 of 0.4 to 1.0, induced with 0.25 mM IPTG. Then, cells were grown overnight at 25 °C. Cells were harvested at 6000 × g for 12 min at 4 °C and cell pellets were stored at -20 °C until purification. For purification, cell pellets were resuspended in two-fold volume of lysis buffer (50 mM sodium phosphate buffer PH=7.7, 300 mM NaCl, 5 mM $MgCl_2$, 10 μg $ml^{-1}$ DNase I), lysed by ultrasonification and centrifuged at 50,000 × g and 4 °C for 40 min. The lysate was filtered through a 0.45 μm filter, mixed with 2 ml Protino Ni-NTA agarose beads (Macherey-Nagel), and incubated on ice for 30 min. Then the agarose beads were transferred into a Protino gravity column (Machery-Nagel), and unbound protein was washed out with 15 ml wash buffer (50 mM sodium phosphate buffer PH=7.7, 300 mM NaCl, 50 mM Imidazole). The protein was eluted with 2.7 ml elution buffer (50 mM sodium phosphate buffer PH=7.7, 300 mM NaCl, 500 mM Imidazole). Further purification via size exclusion chromatography was carried out in SEC buffer (50 mM HEPES, 200 mM NaCl, 10% glycerol) and used a Superdex 200 increase 10/300GL column (GE Healthcare Life Sciences). Purified proteins were concentrated with Amicon centrifugal filters (Merck Millipore) and stored in 50% glycerol at -20 °C. Protein concentrations were determined by the Bradford protein assay.

**Example 2**

**Enzyme assays**

Assays of AOR were carried out at 30 °C in a glovebox (Vinyl Glove Box, COY lab Products) with protective gas atmosphere (97% $N_2$, 3% $H_2$). The absorbance was measured on a Cary 4000 UV-Vis spectrophotometer (Agilent) with a fiber optic probe (10 mm path length). Assays of PduP-NP, Pta, AckA, and TdcD were conducted at 30 °C under aerobic conditions. The absorbance at a given wavelength was measured on a Cary 60 UV-Vis spectrophotometer (Agilent) in quartz cuvettes with a path length of 10 mm.

**Activity assay of AOR**

The carboxylic acid reductase activity of AOR was measured by monitoring the decrease of absorbance at 595 nm corresponding to the consumption of reduced hexamethyl viologen (HMV) ($\varepsilon_{595\ nm}$ = 10.1 $mM^{-1}\ cm^{-1}$). The reaction mixture contained 100 mM phosphate buffer pH 6, 5 mM HMV, 0.1 mM Ti(III)-citrate, 10 mM phenylhydrazine, 60 mM acetate/propionate, and 84.9 μg/ml AOR. Ti(III)-citrate was added into phosphate buffer containing HMV first to produce reduced HMV ($HMV^{+\bullet}$). AOR and phenylhydrazine were added later when the absorbance at 595 nm stayed constant. Acetate/propionate was added last to start the reaction. One unit is defined as 2 μmol $HMV^{+\bullet}$ oxidized per min.

**Activity assay of PduP-NP**

The aldehyde dehydrogenase activity of PduP-NP was assayed by monitoring the increase of absorbance at 340 nm corresponding to the production of NADPH ($\varepsilon_{340\ nm}$ = 6.22 $mM^{-1}\ cm^{-1}$). The reaction mixture (200 μL) contained 100 mM phosphate buffer pH 6, 0.5 mM CoA, 1 mM $NADP^+$, 0.1 - 6 mM acetaldehyde or 0.1 - 3 mM propionaldehyde, and 1.8 - 16.5 μg PduP-NP. One unit is defined as 1 μmol NADPH produced per min.

**Activity assay of Pta**

The activity of Pta was measured in a coupled assay with TdcD, hexokinase (HK), and glucose-6-phosphate dehydrogenase (G6PDH). Pta converts propionyl-CoA to propionylphosphate, providing the substrate for TdcD. HK converts glucose to G6P using ATP formed from the TdcD reaction, and G6PDH subsequently oxidizes G6P using $NADP^+$ as electron acceptor. The production of NADPH was followed spectrophotometrically at 340 nm. The reaction mixture (200 μL) contained 100 mM phosphate buffer pH 6, 5 mM $MgCl_2$, 1 mM ADP, 0.5 mM $NADP^+$, 2 mM glucose, 4.3 μg HK, 3.6 μg G6PDH, 2 μg TdcD, and 0.16 μg Pta. One unit is defined as 1 μmol NADPH produced per min.

**Activity assay of AckA or TdcD**

The activity of AckA or TdcD was measured in a coupled assay with Pta, HK, and G6PDH. Pta converts propionyl-CoA to propionylphosphate, providing the substrate for AckA or TdcD. HK converts glucose to G6P using ATP formed from the AckA/TdcD reaction, and G6PDH subsequently oxidizes G6P using $NADP^+$ as electron acceptor. The production of NADPH was followed spectrophotometrically at 340 nm. The reaction mixture (200 μL) contained 100 mM phosphate buffer pH 6, 5 mM $MgCl_2$, 1 mM ADP, 0.5 mM $NADP^+$, 2 mM glucose, 4.3 μg HK, 3.6 μg G6PDH, 15.8 μg Pta, and 0.115 μg AckA or 0.02 μg TdcD. One unit is defined as 1 μmol NADPH produced per min.

**Assay of the PduP-NP, Pta, and TdcD cascade**

The PduP-NP, Pta, and TdcD cascade was assayed aerobically under the working conditions of $AOR_{Aa}$. The assay was performed in a 50 μL reaction mixture containing 100 mM phosphate buffer pH 6, 5 mM $MgCl_2$, 0.2 mM propionaldehyde, 1 mM $NADP^+$, 1 mM ADP, 0.2 mM or 0.5 mM CoA, 0.5 mM pyruvate, 60 mM propionate, 0.2 mM P1,P5-di(adenosine-5')pentaphosphate (AP5A), 1.8 μg PduP-NP, 0.7 μg Pta, 0.4 μg TdcD, and 0.9 μg Ldh. The reaction was started with the addition of propionaldehyde. A reaction mixture without adding propionaldehyde was used as the negative control. A reaction mixture containing everything (with 0.5 mM CoA) except Ldh was setup to check the effect of NADPH recycling. Samples (10 μL) were withdrawn, mixed with 40 μL AXP internal standard mixture (2 μM AMP-$^{15}N_5$, 2 μM ADP-$^{15}N_5$, and 2 μM ATP-$^{13}C_{10}$ in $H_2O$), quenched with 3% formic acid, and analyzed for AXPs with LC-MS/MS using the method described below.

**Assay of the $AAA_P$ cycle powered with Ti(III) reduced HMV**

The assay was performed anaerobically in a 50 μL reaction mixture containing 100 mM phosphate buffer pH 6, 5 mM $MgCl_2$, 5 mM hexamethyl viologen (HMV), 0.6 mM Ti(III)-citrate, 1 mM $NADP^+$, 1 mM ADP, 0.25 mM CoA, 0.5 mM pyruvate, 60 mM propionate, 0.2 mM AP5A, 8.7 μg $AOR_{Aa}$, 2.1 μg PduP-NP, 0.7 μg Pta, 0.4 μg TdcD, and 0.8 μg Ldh. Ti(III)-citrate was first added into phosphate buffer containing HMV to produce reduced HMV. Subsequently, other components were added to the reduced HMV containing buffer. The reaction was started with the addition of propionate. A reaction mixture without adding propionate was used as the negative control. Samples (10 μL) were withdrawn, mixed with 40 μL AXP internal standard mixture (2 μM AMP-$^{15}N_5$, 2 μM ADP-$^{15}N_5$, and 2 μM ATP-$^{13}C_{10}$ in $H_2O$), quenched with 3% formic acid, and analyzed for AXPs with LC-MS/MS using the method described below.

## Example 3

### Setup of electrochemical experiments

All electrochemical experiments were performed using an EmStatblue (Palmsense) potentiostat. A three-electrode set-up was employed using a glassy carbon electrode as the working electrode, a platinum as the counter electrode, and an Ag/AgCl (3.5 M KCl) (eday, ETO72) as the reference electrode. All potentials were reported versus the standard hydrogen electrode (SHE) using E (SHE) = E (Ag/AgCl 3.5 M) + 205 mV. The glassy carbon electrodes with a diameter of 3 mm were polished using alumina powder following the standard protocols. The electrochemical experiments were run under oxygen-free conditions inside a glovebox (Vinyl Glove Box, COY Lab Products). Electrochemical experiments were carried out in a single compartment or in a two-compartment set up separated by a Nafion membrane (Nafion N117, Ion Power).

### Assay of the $AAA_P$ cycle powered with electricity reduced HMV

The assay was performed anaerobically in a 50 μL reaction mixture containing 100 mM phosphate buffer pH 6, 0.85 mM reduced HMV, 3.4 mM HMV (oxidized form), 5 mM $MgCl_2$, 1 mM $NADP^+$, 1 mM ADP, 0.25 mM CoA, 0.5 mM pyruvate, 60 mM propionate, 0.2 mM AP5A, 13.3 μg $AOR_{Aa}$, 2.1 μg PduP-NP, 0.7 μg Pta, 0.4 μg TdcD, and 0.8 μg Ldh. To produce electricity reduced HMV, 5 mM HMV in 100 mM phosphate buffer pH 6 was continuously reduced for 1 h at -0.745 V vs SHE with stirring (at 500 rpm) in the center of the electrochemical cell. A glassy carbon electrode (0.07 $cm^2$ area) and a two-compartment electrochemical cell were used in this process. After reduction, the concentration of reduced HMV was quantified spectrophotometrically at 595 nm. Other components were subsequently added to the electricity reduced HMV solution. The reaction was started with the addition of propionate. A reaction mixture without adding propionate was used as the negative control. Samples (10 μL) were withdrawn, mixed with 40 μL AXP internal standard mixture (2 μM AMP-$^{15}N_5$, 2 μM ADP-$^{15}N_5$, and 2 μM ATP-$^{13}C_{10}$ in $H_2O$), quenched with 3% formic acid, and analyzed for AXPs with LC-MS/MS using the method described below.

### Assay of electricity-powered $AAA_P$ cycle

The assay was performed anaerobically in a 800 μL reaction mixture containing 100 mM phosphate buffer pH 6, 5 mM HMV, 5 mM $MgCl_2$, 1 mM $NADP^+$, 1 mM ADP, 0.25 mM CoA, 0.5 mM pyruvate, 60 mM propionate, 0.2 mM AP5A, 177.8 μg $AOR_{Aa}$, 27.6 μg PduP-NP, 10.0 μg Pta, 6.0 μg TdcD, and 11.8 μg Ldh. Except for $AOR_{Aa}$, other enzymes were prepared aerobically and contained oxygen. To guarantee an anaerobic environment before adding $AOR_{Aa}$, a small amount of reduced HMV was first produced to scavenge oxygen. To do so, the HMV containing phosphate buffer was reduced for 330 s at -0.56 V vs SHE with stirring (at 500 rpm) directly under the working electrode. Subsequently, other components were added to a final 900 μL volume. Then 100 μL mixture was taken out to react without electricity as the negative control. The rest 800 μL reaction mixture was placed in a two-compartment electrochemical cell with a glassy carbon electrode (0.07 $cm^2$ area), and reduced for 5 h at potentials between -0.57 to -0.53 V vs SHE. The potential was adjusted according to the color of the reaction mixture to keep the reduced HMV (purple color) concentration low (see Fig. 29 for details). A reaction mixture containing everything except $AOR_{Aa}$ was applied electricity with the same potentials for 5 h as another negative control. Samples (17 μL) were withdrawn at different time points. 10 μL sample was mixed with 40 μL AXP internal standard mixture (2 μM AMP-$^{15}N_5$, 2 μM ADP-$^{15}N_5$, and 2 μM ATP-$^{13}C_{10}$ in $H_2O$), quenched with 3% formic acid, and analyzed for AXPs with LC-MS/MS using the method described below. 2 μL sample was mixed with 198 μL water, and analyzed for ATP with the luciferin-luciferase assay using the ATP Bioluminescence Assay Kit CLS II and following the manufacturer's protocol.

The Faradic efficiency of ATP production, $FE_{ATP}$, was calculated based on the following equation:

$$FE_{ATP} = \frac{(C_{ATP60m} \times V_{60m} + (C_{ATP20m} + C_{ATP40m}) \times V_s) \times 2 \times F}{\int i\,(A)\,dt}$$

Here $C_{ATP20m}$, $C_{ATP40m}$, $C_{ATP60m}$ (mol $L^{-1}$) are the ATP concentration (quantified with the luciferin-luciferase assay) in the reaction mixture subtract the ATP concentration in the without electricity control at time 20 min, 40 min, and 60 min, respectively. $V_{60m}$ (L) is the volume of the reaction mixture at time 60 min, $V_S$ is the sample volume (17 μL), F (C $mol^{-1}$) is the Faraday constant, and ∫ i (A) dt is the amount of electrons passed through the working electrode in the first hour.

## Example 4

### Assay of coupling the $AAA_P$ cycle with hexokinase

The assay was performed anaerobically in a 800 μL reaction mixture containing 100 mM phosphate buffer pH 6, 5 mM HMV, 5 mM $MgCl_2$, 1 mM $NADP^+$, 1 mM ADP, 0.25 mM CoA, 0.5 mM pyruvate, 60 mM propionate, 2 mM glucose, 0.2 mM AP5A, 177.8 μg $AOR_{Aa}$, 27.6 μg PduP-NP, 10.0 μg Pta, 6.0 μg TdcD, 15.2 HK, and 11.8 μg Ldh. To guarantee an anaerobic environment before adding $AOR_{Aa}$, a small amount of reduced HMV was first produced to scavenge oxygen by reducing the HMV containing phosphate buffer for 330 s at -0.56 V vs SHE. Subsequently, other components were added to a final 900 μL volume. Then 100 μL mixture was taken out to react without electricity as the negative control. The rest 800 μL reaction mixture was placed in a two-compartment electrochemical cell with a glassy carbon electrode (0.07 $cm^2$ area), and reduced for 4 h at potentials between -0.57 to -0.53 V vs SHE, with stirring (at 500 rpm) directly under the working electrode. The potential was adjusted according to the color of the reaction mixture to keep the reduced HMV (purple color) concentration low (see Fig. 30 for details). A reaction mixture in absence of $AOR_{Aa}$ was applied electricity with the same potentials for 4 h as another negative control. Samples (10 μL) were withdrawn and mixed with 40 μL $H_2O$, quenched with 3% formic acid, and analyzed for glucose-6-phosphate (G6P) with LC-MS/MS using the method described below.

The Faradic efficiency of G6P production, $FE_{G6P}$, was calculated based on the following equation:

$$FE_{G6P} = \frac{(C_{G6P60m} \times V_{60m} + (C_{G6P20m} + C_{G6P40m}) \times V_s) \times 2 \times F}{\int i\,(A)\,dt}$$

Here $C_{G6P20m}$, $C_{G6P40m}$, $C_{G6P60m}$ (mol $L^{-1}$) are the G6P concentration in the reaction mixture subtract the G6P concentration in the without electricity control at time 20 min, 40 min, and 60 min, respectively. $V_{60m}$ (L) is the volume of the reaction mixture at time 60 min, $V_S$ is the sample volume (10 μL), F (C $mol^{-1}$) is the Faraday constant, and ∫ i (A) dt is the amount of electrons passed through the working electrode in the first hour.

### Characterization of the Faradic efficiency of HMV reduction

The HMV reduction was performed in a two-compartment electrochemical cell with a glassy carbon electrode (0.07 $cm^2$ area). HMV (5 mM) in 100 mM phosphate buffer pH 6 was continuously reduced for 5-10 min at three different potentials (-0.57/-0.55/- 0.53 V vs SHE) with stirring (at 500 rpm) directly under the electrode. The reduced HMV concentrations ($C_{HMV^{+\cdot}}$) after the reduction were quantified by determining the absorption at 595 nm. The Faradic efficiency of HMV reduction, $FE_{HMV}$, is calculated based on the following equation:

$$FE_{HMV} = \frac{C_{HMV^{+\cdot}}\,(mol\ L^{-1}) \times V\,(L) \times F\,(C\ mol^{-1})}{\int i\,(A)\,dt}$$

Here V (L) is the volume of HMV solution, F (C $mol^{-1}$) is the Faraday constant, and ∫ i (A) dt is the amount of electrons passed through the working electrode.

## Example 5

### *In vitro* transcription (TX) assay

Reactions were set up in a 20 μL scale by mixing the following components to the listed final concentrations: TX buffer (40 mM Tris-HCl pH 8.0, 8 mM $MgCl_2$, 10 mM DTT, 20 mM NaCl, and 2 mM spermidine), 0.2 mM DFHBI-1T (Sigma-Aldrich), 2.85 mM each of nucleoside triphosphates CTP, GTP and UTP (80 mM stocks buffered in 200 mM tris base, Thermo Fisher), 0.3 U thermostable inorganic pyrophosphatase (New England Biolabs), 0.2 mM AP5A (Sigma-Aldrich), 100 U T7 RNA polymerase (New England Biolabs), 10 nM linear template DNA, and 2 μL sample. Samples are the reaction mixture of the $AAA_P$ cycle after applying electricity for 5 h, and the reaction mixture of the $AAA_P$ cycle without $AOR_{Aa}$ after applying electricity for 5 h (the negative control). The DNA template of a fluorogenic dye-binding RNA aptamer named 3WJdB was amplified by polymerase chain reaction from plasmid p3WJdB (SEQ ID NO 12) using the primer set oSB0021/oSB0022. Reactions were characterized on a plate reader (Infinite M Plex, Tecan, Bottom read) in 2 min intervals with excitation and emission wavelengths of 472 and 507 nm, respectively, for 1 h at 37 °C. Therefore, 18 μL of a reaction were loaded onto a 384-well, black, optically clear, flat-bottomed plate (Greiner Bio-One). Arbitrary fluorescence units were standardized to a National Institute of Standards and Technology (NIST) traceable standard (Invitrogen).

Therefore, a 1:2 serial dilution with MilliQ $dH_2O$ was prepared in the range of 0-2 μM fluorescein and measured together with samples, n = 3 technical replicates. Linear regression was performed between the measured arbitrary units and the concentration of the NIST standard to identify a conversion factor from arbitrary units to micromolar equivalent fluorescein (MEF).

## Example 6

### *In vitro* transcription-translation (TX-TL) assay

### Energy solution preparation

The following solutions were prepared. SolutionA(-Salts-tRNAs-AAs-ATP) (2 ml): Creatine phosphate (147.06 mM), Folinic acid (0.15 mM), Spermidine (14.71 mM), DTT (7.4 mM), GTP (14.71 mM), CTP (7.4 mM), UTP (7.4 mM), and HEPES (pH 7.6, 367.65 mM). Salts solution (2 ml): Magnesium acetate (184.38 mM), and Potassium glutamate (1.563 M). tRNAs solution (200 μL): tRNAs (560 $A_{260}$ $ml^{-1}$). tRNAs were quantified by using UV absorption $A_{260}$ in Thermo Scientific NanoDrop 2000 spectrophotometer. AAs solution (4 ml): 20 proteinogenic AAs (3 mM). The four solutions were combined in a 50 μL reaction, by mixing 6.8/3.2/5/5 v/v/v/v solutionA(-Salts-tRNAs-AAs-ATP):salts solution:tRNAs solution:AAs solution, in order to get the desired concentrations, adapted from Ueda and coworkers[40]: Creatine phosphate (20 mM), Folinic acid (0.02 mM), Spermidine (2 mM), DTT (1 mM), GTP (2 mM), CTP (1 mM), UTP (1 mM), HEPES (pH 7.6, 50 mM), Magnesium acetate (11.8 mM), Potassium glutamate (100 mM), tRNAs (56 $A_{260}$ $ml^{-1}$), and AAs (0.3 mM).

### Coupling the $AAA_P$ cycle with TXTL for EGFP production

6.8 μL of solutionA(-Salts-tRNAs-Aas-ATP), 3.2 μL of salts solution, 5 μL of tRNAs solution, 5 μL of AAs solution, 2.5 μL custom PUREfrex™ Solution II Δ(MK, CK, NDK, PPiase) (enzymes), 2.5 μL PUREfrex™ Solution III (ribosomes), 1 μL RNAse inhibitor, and 150 ng of EGFP DNA template, 1.7 μL HEPES (pH 8.2, 1.5 M), and 20 μL of sample solution were mixed on ice. Nuclease-free water was added to bring the reaction volume to 50 μL. EGFP linear DNA template was amplified from gBlock encoding EGFP (SEQ ID NO 14) by Phusion High-Fidelity DNA polymerase. Sample solutions are the reaction mixture of the $AAA_P$ cycle after applying electricity for 5 h, and the reaction mixture of the $AAA_P$ cycle without $AOR_{Aa}$ after applying electricity for 5 h (the negative control).

The reactions were gently mixed, transferred into a 384-well black transparent bottom plate, sealed to avoid evaporation, spun down at 2000 rcf, 4 °C in Thermo Fisher Scientific Heraeus Multifuge X1R centrifuge, and incubated at 37 °C for 6 h into Tecan Infinite M Plex plate reader. The plate reader parameters were the following: mode = fluorescence bottom reading, interval time = 2 min, $\lambda_{exc}$ = 488 nm, $\lambda_{em}$ = 515 nm, gain = 100, number of flashes = 25, integration time = 20 μs, shaking orbital duration = 20 s, and shaking orbital amplitude = 5.5 mm.

### Coupling the $AAA_P$ cycle with TXTL for Nanoluc Luciferase production

3.4 μL of solutionA(-Salts-tRNAs-Aas-ATP), 1.6 μL of salts solution, 2.5 μL of tRNAs solution, 2.5 μL of AAs solution, 1.25 μL custom PUREfrex™ Solution II Δ(MK, CK, NDK, PPiase) (enzymes), 1.25 μL PUREfrex™ Solution III (ribosomes), 0.5 μL RNAse inhibitor, and 75 ng of pME-Nanoluc (SEQ ID NO 13), 0.85 μL HEPES (pH 8.2, 1.5 M), and 10 μL of sample solution were mixed on ice. Nuclease-free water was added to bring the reaction volume to 25 μL. Sample solutions are the reaction mixture of the $AAA_P$ cycle after applying electricity for 5 h, and the reaction mixture of the $AAA_P$ cycle without $AOR_{Aa}$ after applying electricity for 5 h (the negative control). The reactions were gently mixed, and incubated at 37 °C, for 6 h into the Thermo Fisher Scientific ProFlex™ PCR System. 200 μL luciferase assay reagent was prepared by mixing 1/50 v/v luciferase assay substrate: luciferase assay buffer. The reactions were diluted 1/1 v/v reaction: luciferase assay reagent, and transferred into a 384-well white transparent bottom plate. The plate reader parameters were the following: mode = luminescence, attenuation = none, integration time = 1000 ms.

## Example 7

### High resolution LC-MS analysis of phenylhydrozones

Acetaldehyde phenylhydrozone and propionaldehyde phenylhydrozone were analyzed using HRES LC-MS. The chromatographic separation was performed on an Agilent Infinity II 1290 HPLC system using a Kinetex EVO C18 column (10 × 2.1 mm, 3 μm particle size, 100 Å pore size, Phenomenex) connected to a guard column of similar specificity (20 × 2.1 mm, 3 μm particle size, Phenomoenex) at a constant flow rate of 0.2 ml $min^{-1}$ with mobile phase comprised of 0.1% formic acid in water (A) and 0.1% formic acid in methanol (Honeywell) (B). The column oven was set to 40 °C and the

autosampler was maintained at 4 °C. The injection volume was 5 μL. The mobile phase profile consisted of the following steps and linear gradients: 0 to 2 min constant at 5% B; 2 to 11 min from 5 to 95% B; 11 to 12 min constant at 95% B; 12 to 13 min from 95 to 5% B; 13 to 14 min constant at 5% B. An Agilent 6550 ion funnel Q-TOF mass spectrometer was used in positive mode with an dual jet stream electrospray ionization source and the following conditions: ESI spray voltage 3500 V, nozzle voltage 500 V, sheath gas 400 °C at 12 L $min^{-1}$, nebulizer pressure 20 psig, and drying gas 225 °C at 13 L $min^{-1}$. The TOF was calibrated using an ESI-L Low Concentration Tuning Mix (Agilent) before measurement (residuals less than 2 ppm for five reference ions) and was recalibrated during a run using 922 *m/z* as reference mass. MS data were acquired with a scan range of 100-1200 *m/z*. LC-MS data were analyzed using MassHunter Qualitative Analysis software (Agilent).

### High resolution LC-MS analysis of propionyl-CoA

Propionyl-CoA was analyzed using HRES LC-MS. The chromatographic separation was performed on an Agilent Infinity II 1290 HPLC system using a Kinetex EVO C18 column (150 × 2.1 mm, 3 μm particle size, 100 Å pore size, Phenomenex) connected to a guard column of similar specificity (20 × 2.1 mm, 3 μm particle size, Phenomoenex) at a constant flow rate of 0.25 ml $min^{-1}$ with mobile phase comprised of 50 mM ammonium acetate in water at pH 8.1 (A) and 100% methanol (Honeywell) (B). The column oven was set to 25 °C and the autosampler was maintained at 4 °C. The injection volume was 5 μL. The mobile phase profile consisted of the following steps and linear gradients: 0 to 1 min constant at 2.5% B; 1 to 6 min from 2.5 to 95% B; 6 to 8 min constant at 95% B; 8 to 8.1 min from 95 to 2.5% B; 8.1 to 10 min constant at 2.5% B. An Agilent 6550 ion funnel Q-TOF mass spectrometer was used in positive mode with an dual jet stream electrospray ionization source and the following conditions: ESI spray voltage 3500 V, nozzle voltage 500 V, sheath gas 400 °C at 12 L $min^{-1}$, nebulizer pressure 20 psig and drying gas 225 °C at 13 L $min^{-1}$. The TOF was calibrated using an ESI-L Low Concentration Tuning Mix (Agilent) before measurement (residuals less than 2 ppm for five reference ions) and was recalibrated during a run using 922 *m/z* as reference mass. MS data were acquired with a scan range of 100-1200 *m/z*. LC-MS data were analyzed using MassHunter Qualitative Analysis software (Agilent).

### LC-MS/MS analysis of AMP, ADP, and ATP

AMP, ADP and ATP were analyzed using LC-MS/MS with AMP-$^{15}N_5$, ADP-$^{15}N_5$, and ATP-$^{13}C_{10}$ as their corresponding internal standards. The chromatographic separation was performed on an Agilent Infinity II 1290 HPLC system using a SeQuant ZIC-pHILIC column (150 × 2.1 mm, 5 μm particle size, peek coated, Merck) connected to a guard column of similar specificity (20 × 2.1 mm, 5 μm particl size, Phenomoenex) at a constant flow rate of 0.1 ml $min^{-1}$ with mobile phase comprised of 10 mM ammonium acetate in water, pH 9, supplemented with medronic acid to a final concentration of 5 μM (A) and 10 mM ammonium acetate in 90:10 acetonitrile to water, pH 9, supplemented with medronic acid to a final concentration of 5 μM (B). The column oven was set to 25 °C and the autosampler was maintained at 4 °C. The injection volume was 2 μL. The mobile phase profile consisted of the following steps and linear gradients: 0 to 1 min constant at 75% B; 1 to 10 min from 75 to 40% B; 10 to 12 min constant at 40% B; 12 to 12.5 min from 40 to 75% B; 12.5 to 20 min constant at 75% B. An Agilent 6495 ion funnel mass spectrometer was used in negative mode with an electrospray ionization source and the following conditions: ESI spray voltage 3000 V, nozzle voltage 1000 V, sheath gas 400 °C at 11 L $min^{-1}$, nebulizer pressure 20 psig, and drying gas 100 °C at 11 L $min^{-1}$. Cell accelerator voltage, dwell time, and fragmentor voltage were set to 5 V, 70 ms, and 380 V respectively. Mass transitions and optimized collision energy used were as follows: ATP (505.9 m/z → 407.9 m/z, 21 V, 505.9 m/z --+ 158.8 m/z, 28 V); ATP-$^{13}C_{10}$ (516 m/z --+ 417.9 m/z, 21 V, 516 m/z --+ 158.8 m/z, 28 V); ADP (425.9 m/z --+ 327.8 m/z, 17 V, 425.9 m/z --+ 133.9 m/z, 22 V); ADP-$^{15}N_5$ (431 m/z → 332.9 m/z, 17 V, 431 m/z --+ 139 m/z, 22 V); AMP (346 m/z --+ 96.9 m/z, 24 V, 346 m/z --+ 78.9 m/z, 30 V); and AMP-$^{15}N_5$ AMP (351 m/z --+ 96.9 m/z, 24 V, 351 m/z --+ 78.9 m/z, 30 V). LC-MS data were analyzed and quantified using MassHunter Qualitative Navigator and QQQ Quantitative Analysis software (Agilent). Absolute concentrations were calculated based on an external calibration curve prepared in sample matrix.

### LC-MS/MS analysis of G6P

Glucose-6-phosphate (G6P) was analyzed using LC-MS/MS. The chromatographic separation was performed on an Agilent Infinity II 1290 HPLC system using a SeQuant ZIC-pHILIC column (150 × 2.1 mm, 5 μm particle size, peek coated, Merck) connected to a guard column of similar specificity (20 × 2.1 mm, 5 μm particle size, Phenomoenex) at a constant flow rate of 0.1 ml $min^{-1}$ with mobile phase comprised of 10 mM ammonium acetate in water, pH 9, supplemented with medronic acid to a final concentration of 5 μM (A) and 10 mM ammonium acetate in 90:10 acetonitrile to water, pH 9, supplemented with medronic acid to a final concentration of 5 μM (B). The column oven was set to 25 °C and the autosampler was maintained at 4 °C. The injection volume was 2 μL. The mobile phase profile consisted of the following steps and linear gradients: 0 to 1 min constant at 75% B; 1 to 10 min from 75 to 40% B; 10 to 12 min constant at 40% B; 12 to 12.5 min from 40 to 75% B; 12.5 to 20 min constant at 75% B. An Agilent 6495 ion funnel mass spectrometer was used in

negative mode with an electrospray ionization source and the following conditions: ESI spray voltage 3000 V, nozzle voltage 1000 V, sheath gas 400 °C at 11 L $min^{-1}$, nebulizer pressure 20 psig, and drying gas 100 °C at 11 L $min^{-1}$. Cell accelerator voltage, dwell time, and fragmentor voltage were set to 4 V, 300 ms, and 380 V respectively. Mass transitions and optimized collision energy used were as follows: G6P (259 m/z → 96.9 m/z, 20 V, 259 m/z --+ 78.9 m/z, 20 V). LC-MS data were analyzed and quantified using MassHunter Qualitative Navigator and QQQ Quantitative Analysis software (Agilent). Absolute concentrations were calculated based on an external calibration curve prepared in sample matrix.

**Example 8:**

Characterization of hexamethyl viologen (HMV)

Hexamethyl viologen (HMV) was characterized by cyclic with a glassy carbon electrode (0.07 $cm^2$ area) at various scan rates. The measurement was done in a two-compartment electrochemical cell with a Nafion membrane. The tested solution contained 5 mM HMV in 100 mM phosphate buffer pH 6. The resulting data, which includes the anodic peak potential ($E_{p,a}$), cathodic peak potential ($E_{p,c}$), peak-to-peak potential separation ($\Delta E_p$), redox midpoint potential (E'°), anodic peak current ($i_{p,a}$), and cathodic peak current ($i_{p,c}$) at different scan rates, are listed in the Table 2 below.

**Table 2**

| Scan rate mV/s | $E_{p.a.}$ mV | $E_{p.c.}$ mV | $\Delta E_p$ mV | E'° mV | $i_{p,a}$ mA/$cm^2$ | $i_{p,c}$ mA/$cm^2$ |
|---|---|---|---|---|---|---|
| 2 | -584 | -637 | 53 | -611 | 5.73 | -7.04 |
| 5 | -584 | -635 | 52 | -610 | 9.62 | -11.56 |
| 10 | -582 | -637 | 54 | -609 | 13.81 | -16.58 |
| 25 | -580 | -635 | 56 | -608 | 20.94 | -26.74 |
| 50 | -580 | -639 | 60 | -610 | 31.18 | -38.30 |
| 100 | -580 | -641 | 61 | -611 | 42.53 | -53.78 |

**Claims**

**1.** A method for producing adenosine triphosphate (ATP) through an acid/aldehyde-ATP (AAA) cycle in an electrochemical cell comprising the steps:

i) providing an AAA cycle reaction mixture in the electrochemical cell, the AAA cycle comprising:

a) a carboxylic acid or a salt thereof,
b) a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2) a CoA-acetylating aldehyde dehydrogenase for catalyzing the conversion of the aldehyde into the corresponding CoA ester;
(b3) a phosphate acyltransferase for catalyzing the conversion of the CoA ester to the corresponding acyl phosphate;
(b4) a carboxylic acid kinase for catalyzing the conversion of the acyl phosphate to the corresponding carboxylic acid;

or a set of enzymes comprising:

(b1) a ferredoxin-dependent aldehyde oxidoreductase or (b1') a mutated aldehyde dehydrogenase for catalyzing the conversion of the carboxylic acid to the corresponding aldehyde;
(b2-3) a phosphate reductase for catalyzing the conversion of the aldehyde to the corresponding acyl phosphate;
(b4) a carboxylic acid kinase for catalyzing the conversion of the acyl phosphate to the corresponding carboxylic acid;

c) adenosine diphosphate and Pi;
d) coenzyme A;
e) $NAD^+$ or $NADP^+$,
f) a low-potential electron carrier;

ii) applying electricity to the electrochemical cell for conversion of the electron carrier into its reduced form, and
iii) producing adenosine triphosphate from the AAA cycle and electricity.

2. The method according to claim 1, wherein the carboxylic acid is selected from the group comprising short chain fatty acids, medium chain fatty acids, glyceric acid, glyceric acid derivatives, benzoic acid, benzoic acid derivatives, aromatic acids.

3. The method according to claim 1 or 2, wherein the set of enzymes further comprises (b5) a lactate dehydrogenase for catalyzing the conversion of NADPH to $NADP^+$, and wherein the reaction mixture further comprises pyruvate.

4. The method according to any one of the claims 1 - 3, wherein the ferredoxin-dependent aldehyde oxidoreductase is a tungsten-containing aldehyde oxidoreductase from *Aromatoleum aromaticum* EbN1 $AOR_{Aa}$, and /or wherein the CoA-acetylating aldehyde dehydrogenase is a NADPH-dependent CoA-acetylating aldehyde dehydrogenase, and wherein $NADP^+$ is provided in step e).

5. The method according to any one of the claims 1 - 4, wherein the carboxylic acid is propionic acid or propionate.

6. The method according to claim 5, wherein the CoA-acetylating aldehyde dehydrogenase is PduP-NP (SEQ ID NO: 9), and/or the phosphate acyltransferase is phosphate acetyltransferase Pta from *E. coli*; and the carboxylic acid kinase is propionate kinase TdcD from *E. coli.*

7. The method according to any one of the claims 1 - 6, wherein the low-potential electron carrier is selected from a viologen derivative, hexamethyl viologen, tetramethyl viologen, Ti(III) citrate, and Eu (II)-EGTA.

8. The method according to any one of the claims 1 - 7, wherein the electrochemical cell comprises a cathodic compartment, an anodic compartment, and a proton-conducting membrane, wherein the proton-conducting membrane physically separates the cathodic and anodic compartments.

9. The method according to any one of the claims 1 - 8, wherein the method comprises the steps:

i) providing an $AAA_P$ cycle reaction mixture in the electrochemical cell, the $AAA_P$ cycle comprising:

a) propionic acid or propionate;
b) a set of enzymes comprising:

(b1) a ferredoxin-dependent aldehyde oxidoreductase for catalyzing the conversion of propionic acid to propionaldehyde, preferably $AOR_{Aa}$;
(b2) a NADPH-dependent CoA-acetylating aldehyde dehydrogenase for catalyzing the conversion of proprionaldehyde into propionyl-CoA, preferably PduP-NP (SEQ ID NO: 9);
(b3) a phosphate acyltransferase for catalyzing the conversion of propionyl-CoA to propionylphosphate, preferably Pta from *E.coli;*
(b4) a propionate kinase for catalyzing the conversion of propionylphosphate to propionic acid, preferably TdcD from *E. coli*; and
(b5) optionally a lactate dehydrogenase;

c) adenosine diphosphate and Pi;
d) coenzyme A and optionally pyruvate;
e) $NADP^+$,
f) a low-potential electron carrier selected from hexamethyl viologen, tetramethyl viologen, Ti(III) citrate, and Eu (II)-EGTA;

ii) applying electricity to the electrochemical cell for conversion of the electron carrier into its reduced form, and
iii) producing adenosine triphosphate from the AAA cycle and electricity.

**10.** The method according to any one of the claims 1 - 9, wherein the AAA cycle further comprises glucose and (b6) hexokinase, and step iii) further comprises producing glucose-6-phosphate.

**11.** The method according to any one of the claims 1 - 9, wherein the method further comprises the steps:

iv) obtaining the AAA cycle reaction mixture comprising adenosine triphosphate;
v') using the AAA cycle reaction mixture of step iv) for cell-free *in-vitro* production of nucleic acids.

**12.** The method of claim 11, wherein step v') further comprises providing a transcription system comprising CTP, GTP, UTP, a thermostable inorganic pyrophosphatase, an adenylate kinase inhibitor, a T7 RNA polymerase, a DNA template.

**13.** The method according to any one of the claims 1 - 9, wherein the method further comprises the steps:

iv) obtaining the AAA cycle reaction mixture comprising adenosine triphosphate;
v") using the AAA cycle reaction mixture of step iv) for cell-free *in vitro* production of proteins.

**14.** The method of claim 13, wherein step v") further comprises providing a protein synthesis system comprising transcription factors, translational factors, aminoacyl-tRNA synthetases, ribosome, amino acids, CTP, GTP, TTP, a DNA template, and wherein said protein synthesis system does not comprises ATP and ATP regenerating enzymes.

**15.** The method according to anyone of the claims 1 - 14, wherein step ii) comprises applying electricity to the electrochemical cell at potential between -0.57 to -0.53 mV *versus* SHE (Standard hydrogen Electrode) preferably for 4 - 5 h.

Figure 1

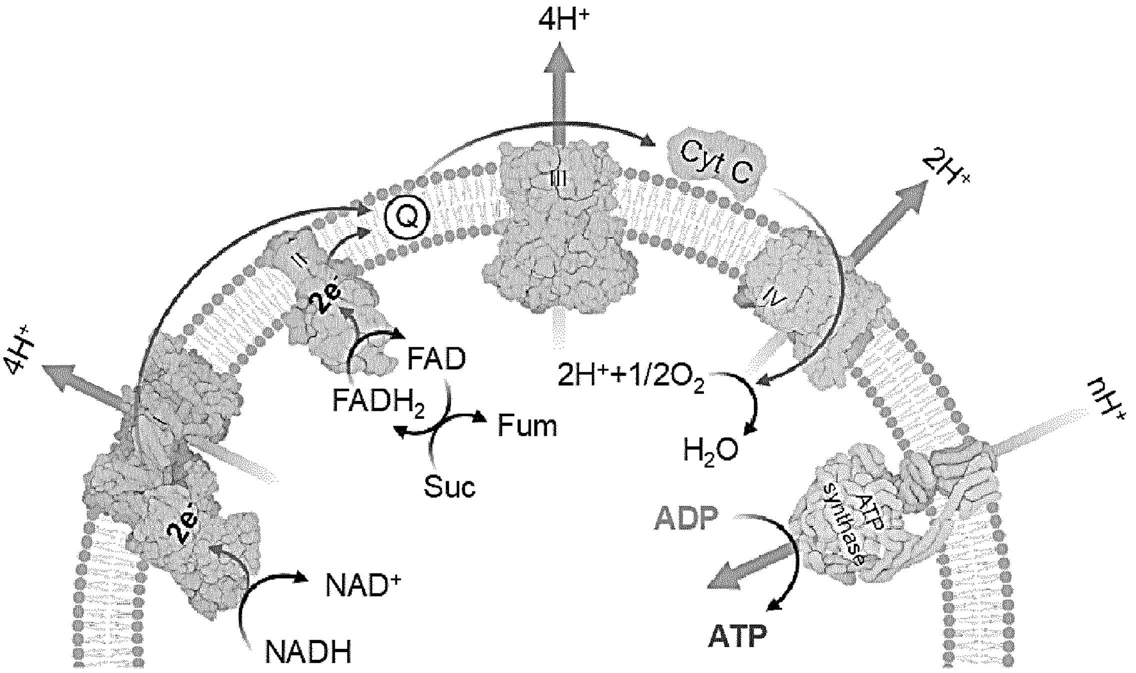


$$NAD(P)H + 2.5\ ADP + 2.5\ Pi + 0.5\ O_2 \rightarrow NAD(P)^+ + 2.5\ ATP + H_2O$$

$$FADH_2 + 1.5\ ADP + 1.5\ Pi + 0.5\ O_2 \rightarrow FAD + 1.5\ ATP + H_2O$$

**Figure 2**

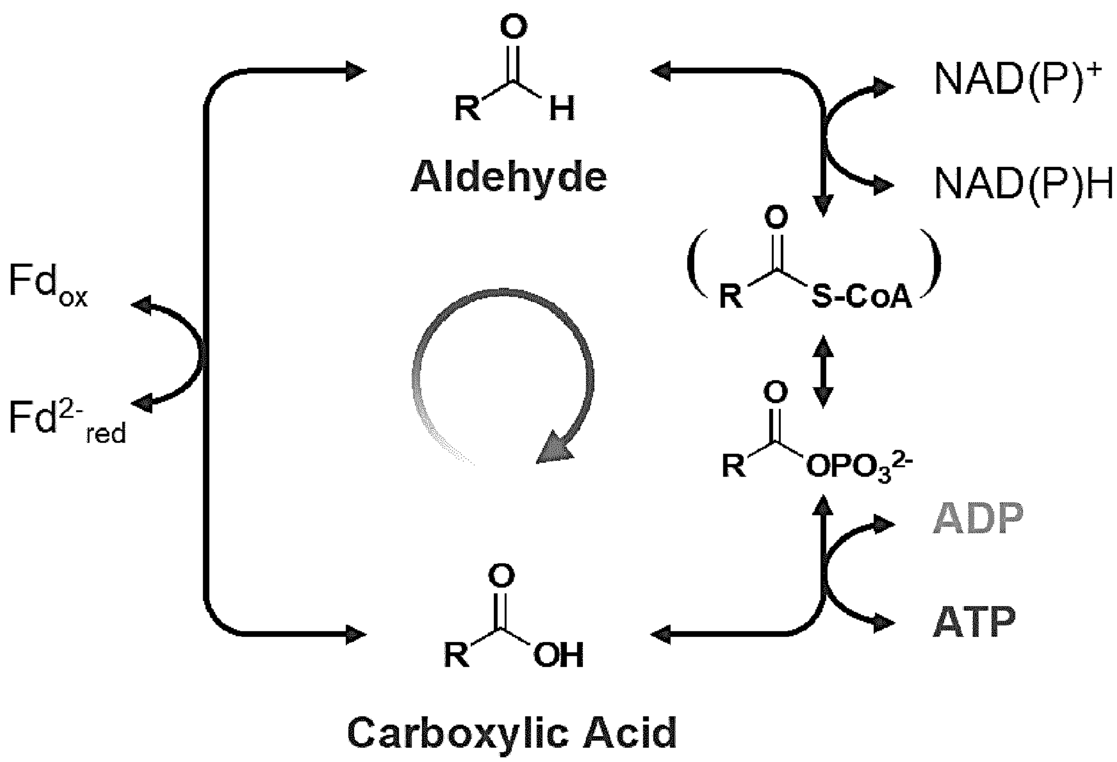


Aldehyde

Carboxylic Acid

$$Fd^{2-}_{red} + NAD(P)^{+} + ADP + Pi \rightarrow Fd_{ox} + NAD(P)H + ATP$$

$$(\Delta G'^{0} = 8.9\ kJ/mol)$$

# Figure 3

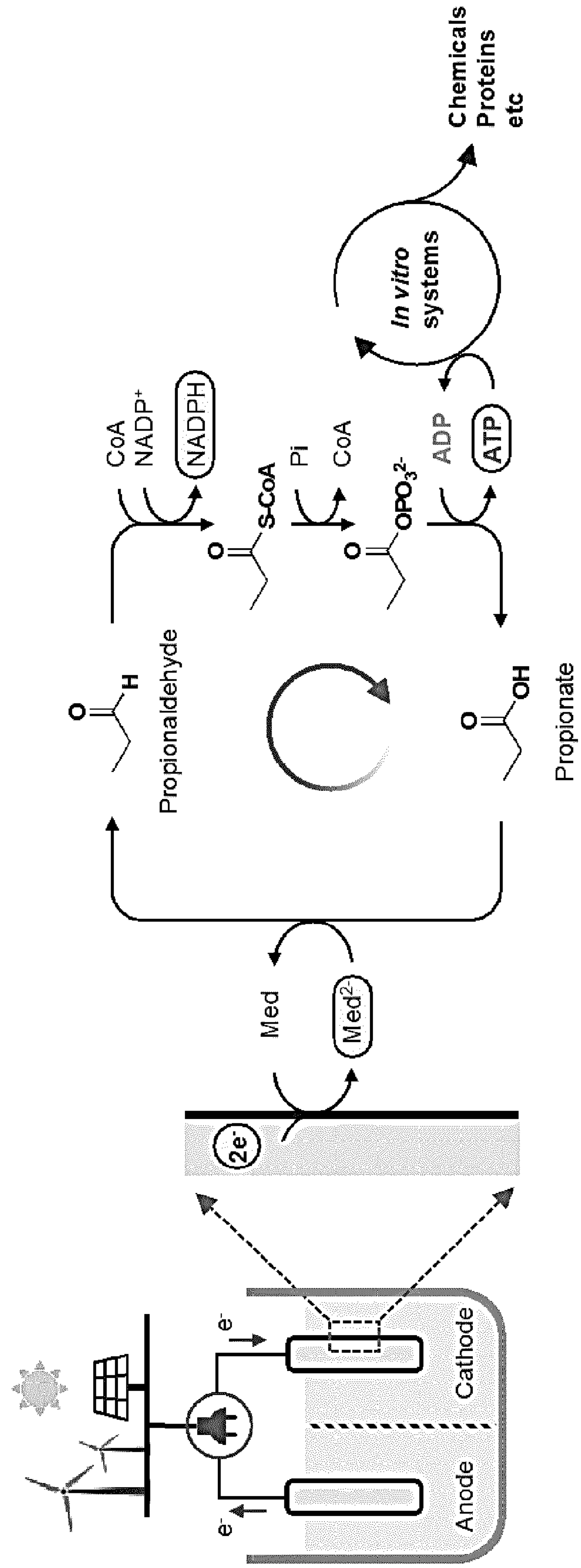
Chemicals
Proteins
etc
In vitro systems
CoA
NADP+
NADPH
Pi
CoA
ADP
ATP
Propionaldehyde
Propionate
Med
Med2-
2e-
e-
e-
Cathode
Anode

# Figure 4

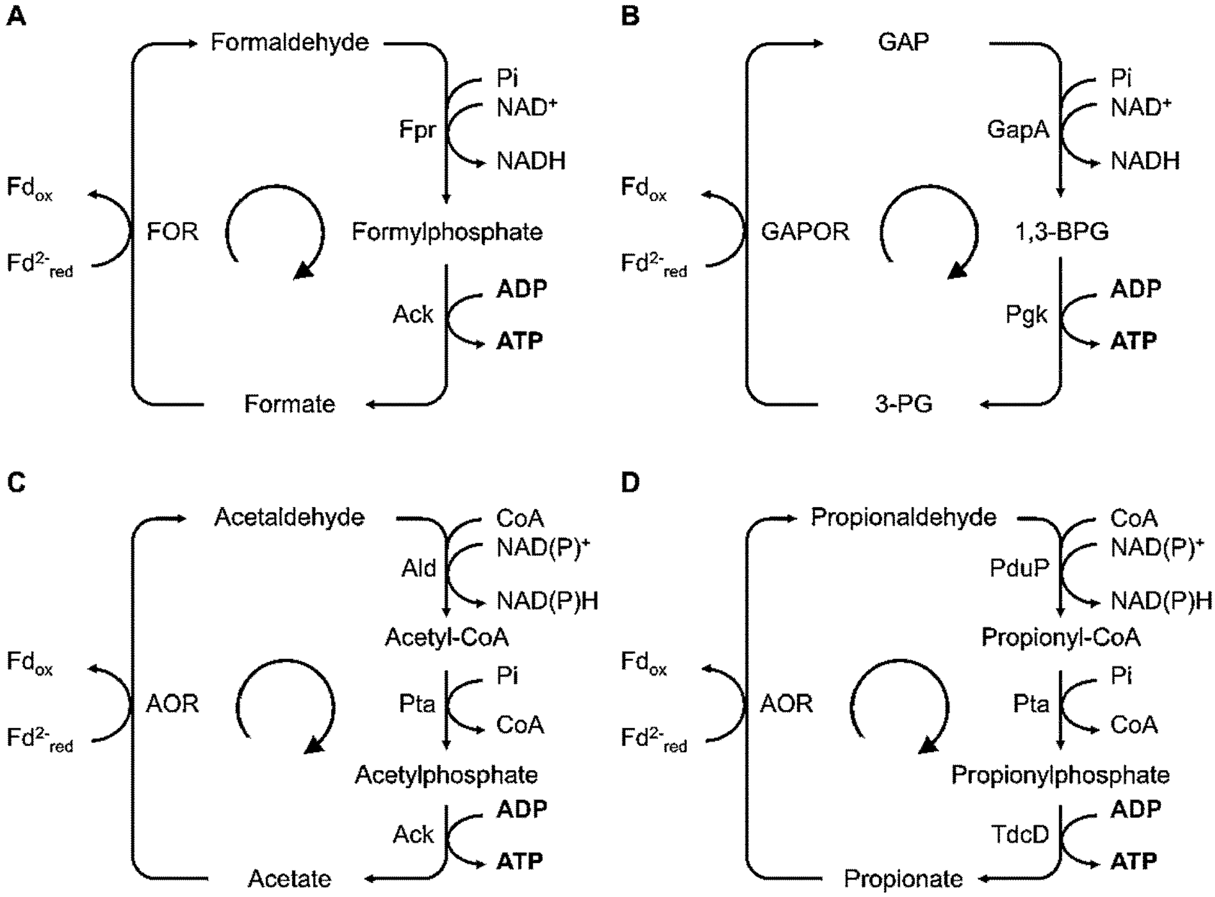
A
Formaldehyde
Pi
NAD+
Fpr
NADH
Fdox
FOR
Formylphosphate
Fd2-red
ADP
Ack
ATP
Formate
B
GAP
Pi
NAD+
GapA
NADH
Fdox
GAPOR
1,3-BPG
Fd2-red
ADP
Pgk
ATP
3-PG
C
Acetaldehyde
CoA
NAD(P)+
Ald
NAD(P)H
Acetyl-CoA
Fdox
Pi
AOR
Pta
Fd2-red
CoA
Acetylphosphate
ADP
Ack
ATP
Acetate
D
Propionaldehyde
CoA
NAD(P)+
PduP
NAD(P)H
Propionyl-CoA
Fdox
Pi
AOR
Pta
Fd2-red
CoA
Propionylphosphate
ADP
TdcD
ATP
Propionate

Figure 5

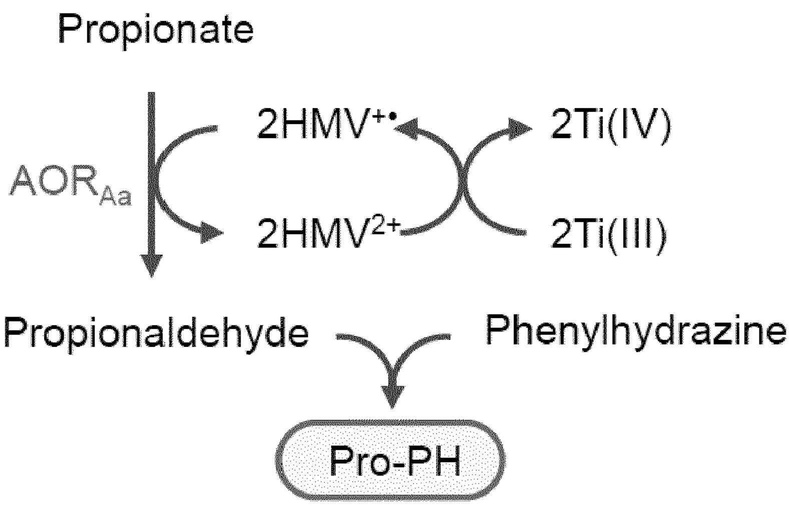
Propionate
$AOR_{Aa}$
$2HMV^{+\bullet}$
$2HMV^{2+}$
2Ti(IV)
2Ti(III)
Propionaldehyde
Phenylhydrazine
Pro-PH

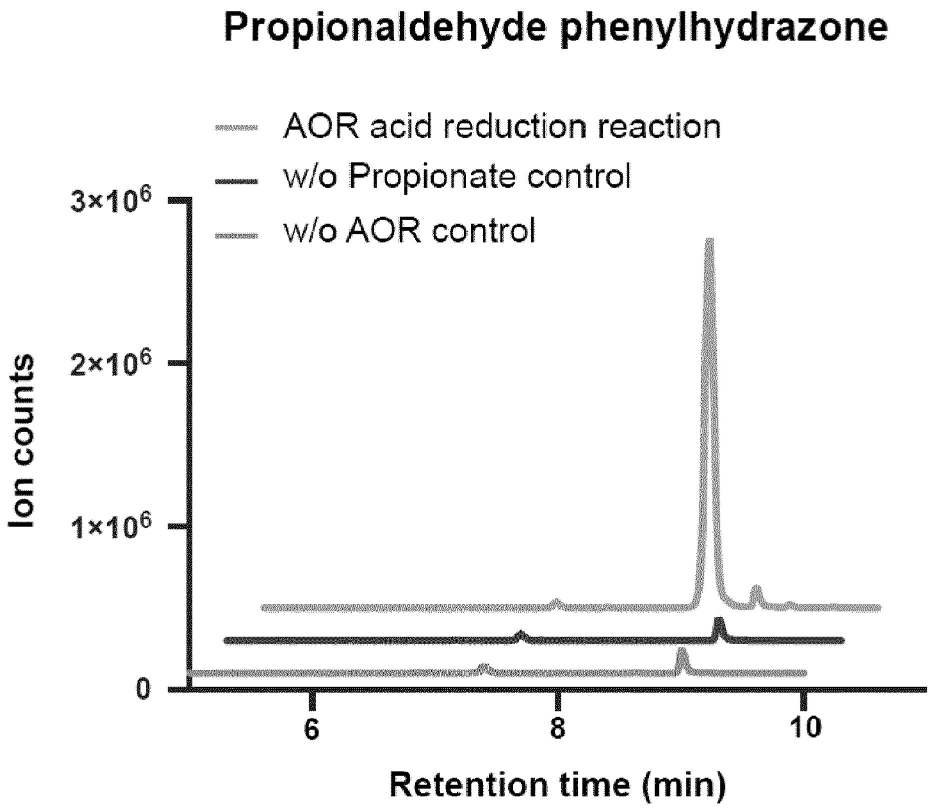
Propionaldehyde phenylhydrazone
AOR acid reduction reaction
w/o Propionate control
w/o AOR control
$3\times10^6$
$2\times10^6$
$1\times10^6$
0
Ion counts
6
8
10
Retention time (min)

Figure 6

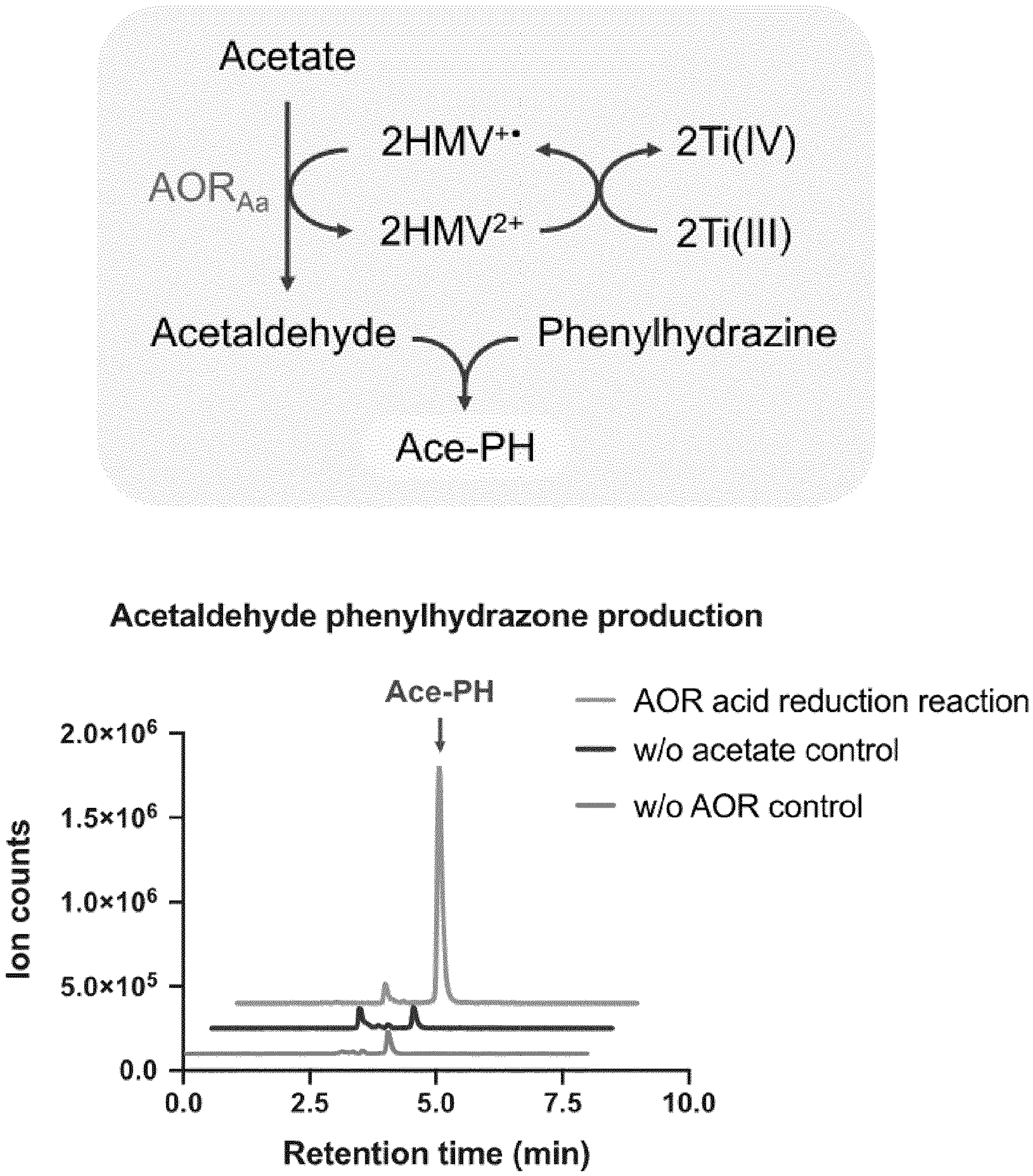
Acetate
AOR$_{Aa}$
2HMV$^{+\bullet}$
2HMV$^{2+}$
2Ti(IV)
2Ti(III)
Acetaldehyde
Phenylhydrazine
Ace-PH
Acetaldehyde phenylhydrazone production
Ace-PH
AOR acid reduction reaction
w/o acetate control
w/o AOR control
Ion counts
2.0×10$^6$
1.5×10$^6$
1.0×10$^6$
5.0×10$^5$
0.0
0.0
2.5
5.0
7.5
10.0
Retention time (min)

## Figure 7

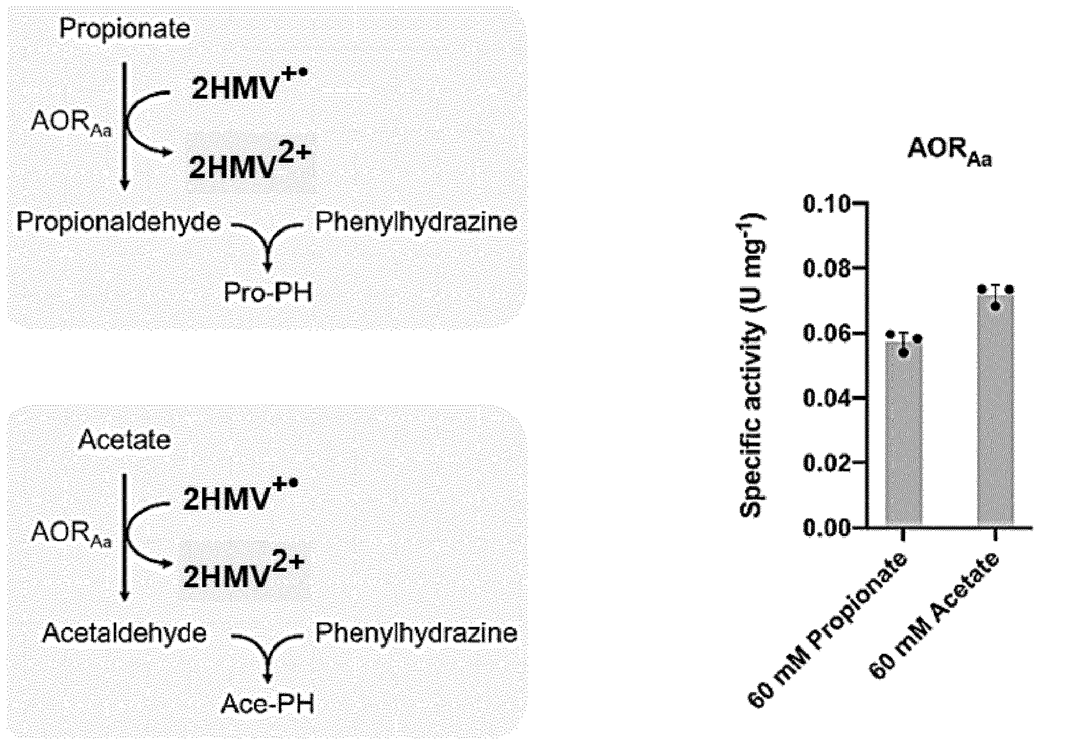


## Figure 8

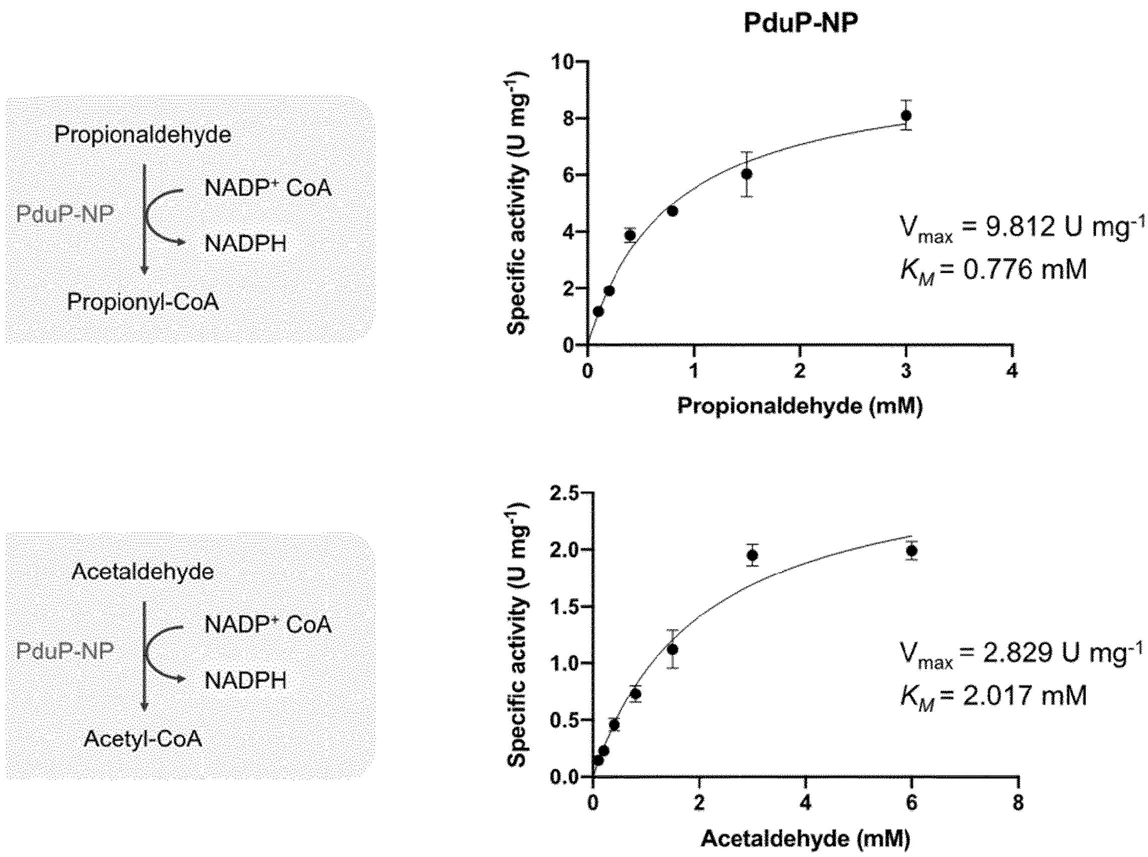

Figure 9

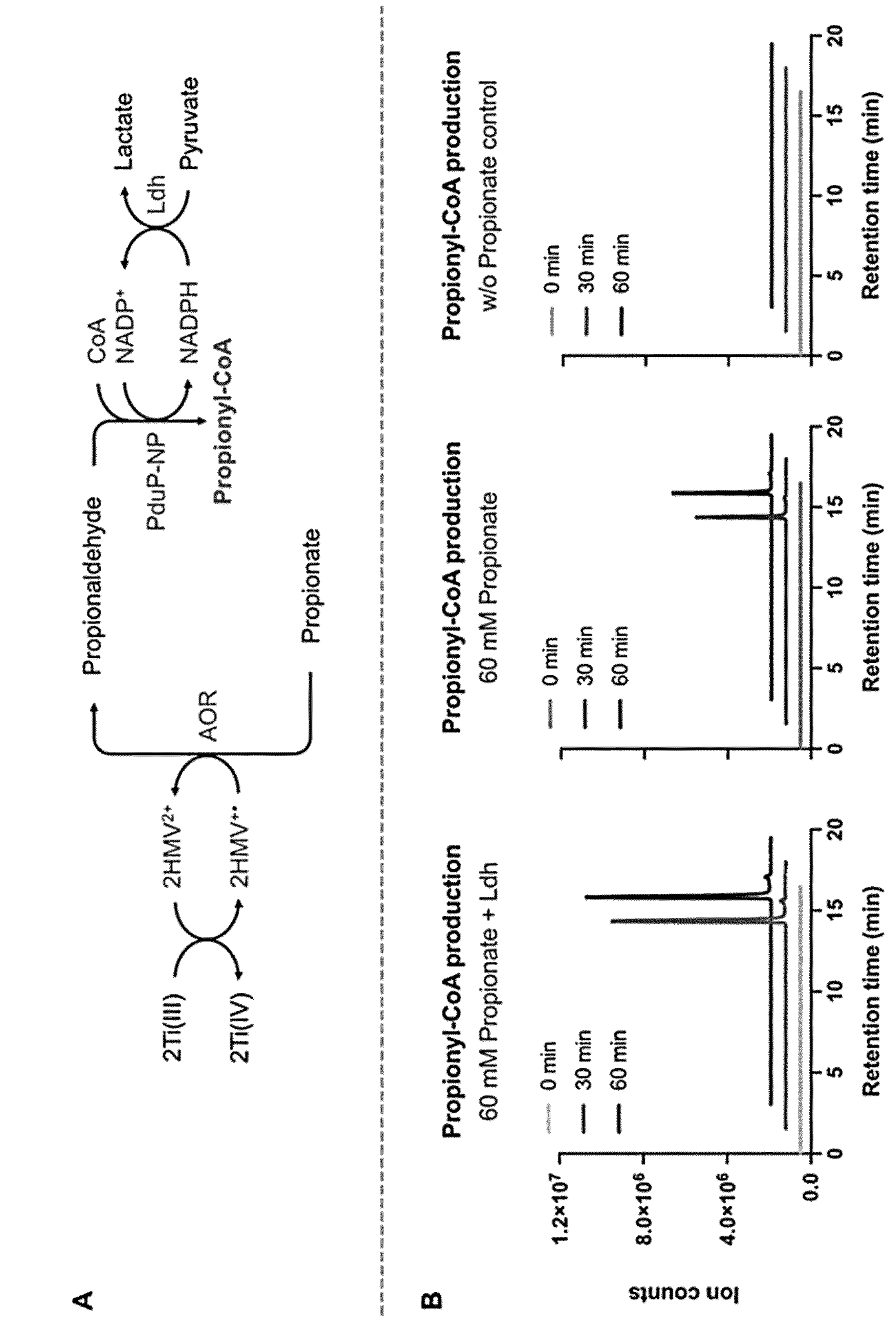
A
2Ti(III)
2Ti(IV)
2HMV2+
2HMV+•
AOR
Propionaldehyde
Propionate
PduP-NP
CoA
NADP+
NADPH
Propionyl-CoA
Ldh
Lactate
Pyruvate
B
Ion counts
1.2×10^7
8.0×10^6
4.0×10^6
0.0
Propionyl-CoA production
60 mM Propionate + Ldh
Propionyl-CoA production
60 mM Propionate
Propionyl-CoA production
w/o Propionate control
0 min
30 min
60 min
Retention time (min)
0
5
10
15
20

## Figure 10

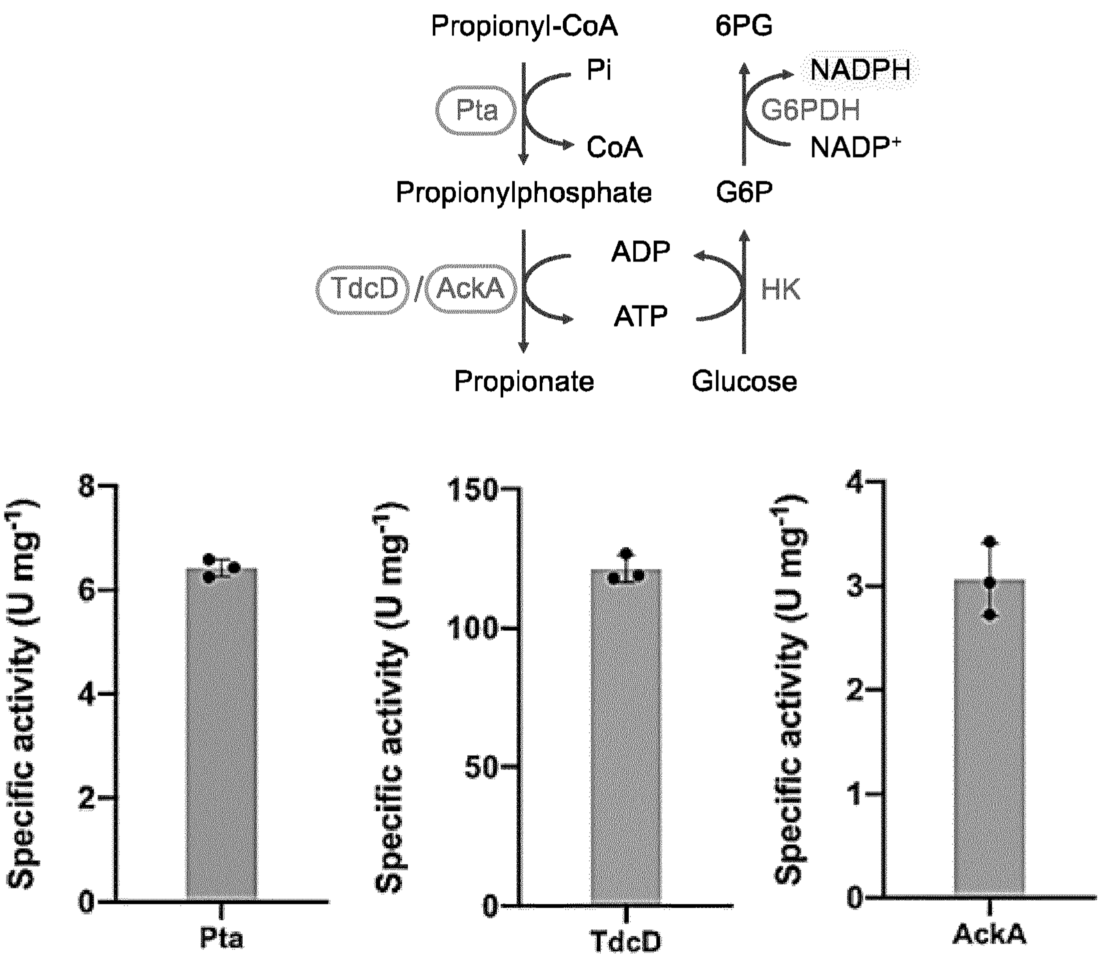
Propionyl-CoA
Pi
Pta
CoA
Propionylphosphate
TdcD
AckA
ADP
ATP
Propionate
6PG
NADPH
G6PDH
NADP+
G6P
HK
Glucose
Specific activity (U mg-1)
Pta
TdcD
AckA

Figure 11

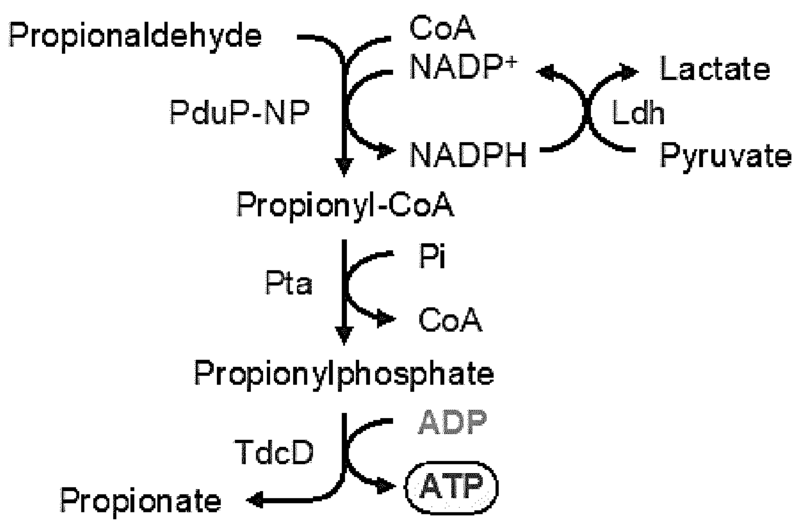
Propionaldehyde
CoA
NADP+
Lactate
PduP-NP
Ldh
NADPH
Pyruvate
Propionyl-CoA
Pta
Pi
CoA
Propionylphosphate
TdcD
ADP
ATP
Propionate

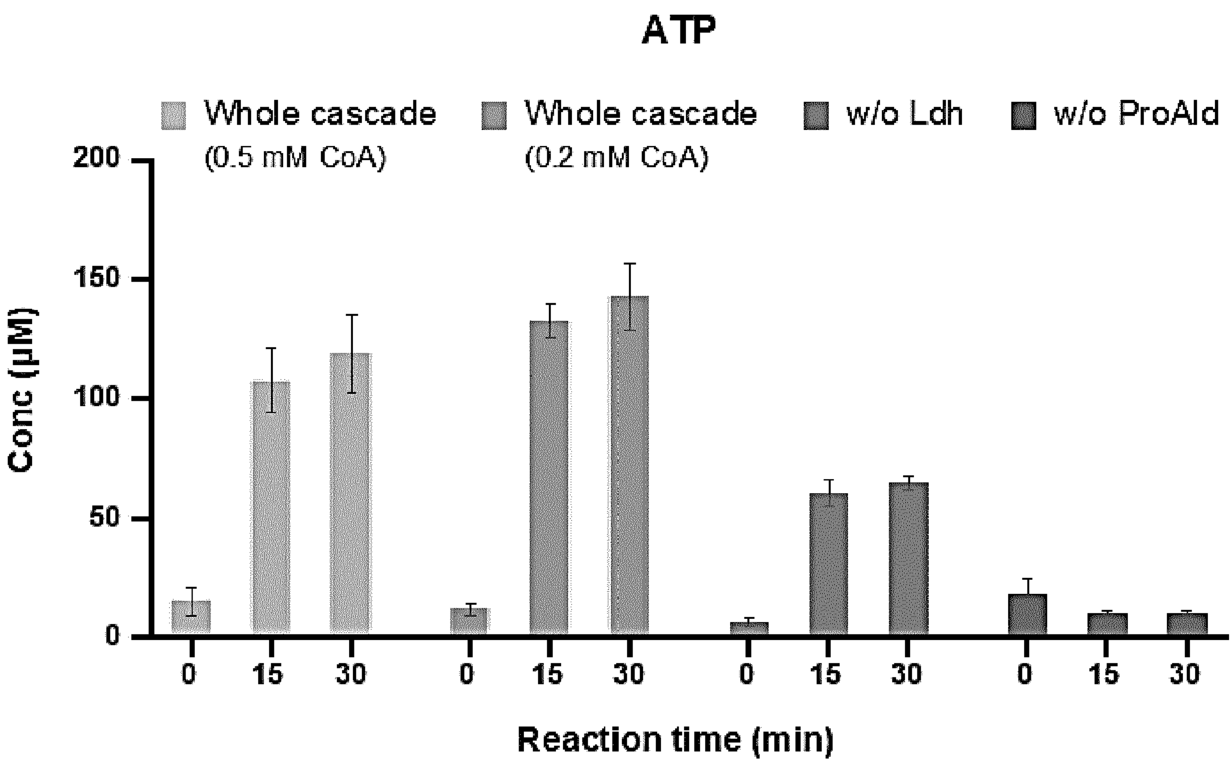
ATP
Whole cascade (0.5 mM CoA)
Whole cascade (0.2 mM CoA)
w/o Ldh
w/o ProAld
Conc (μM)
200
150
100
50
0
0 15 30 0 15 30 0 15 30 0 15 30
Reaction time (min)

Figure 12

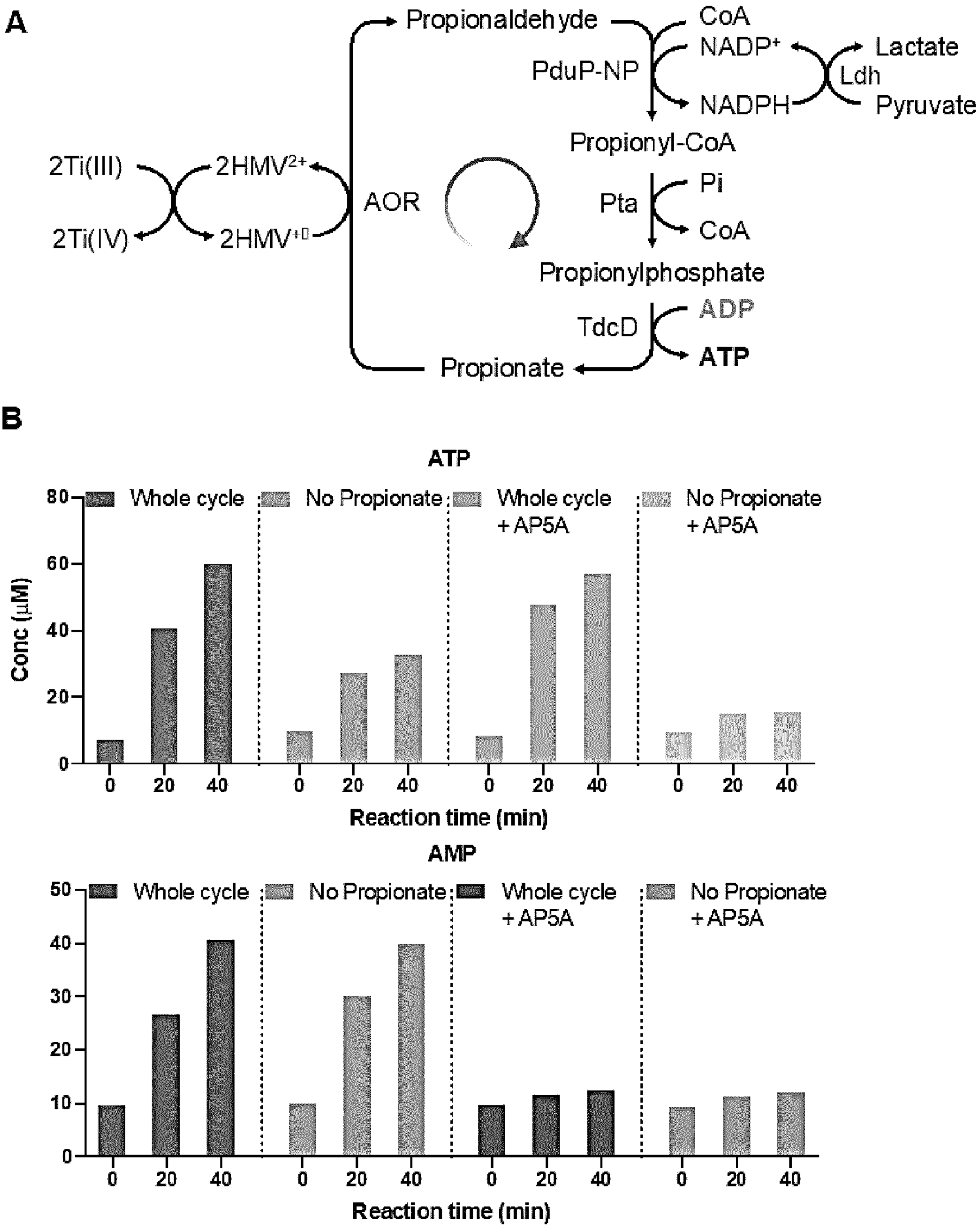
A
Propionaldehyde
CoA
NADP+
Lactate
PduP-NP
Ldh
NADPH
Pyruvate
Propionyl-CoA
2Ti(III)
2HMV2+
Pi
AOR
Pta
2Ti(IV)
2HMV+
CoA
Propionylphosphate
ADP
TdcD
ATP
Propionate
B
ATP
Whole cycle
No Propionate
Whole cycle + AP5A
No Propionate + AP5A
Conc (μM)
Reaction time (min)
AMP
Whole cycle
No Propionate
Whole cycle + AP5A
No Propionate + AP5A
Reaction time (min)

Figure 13

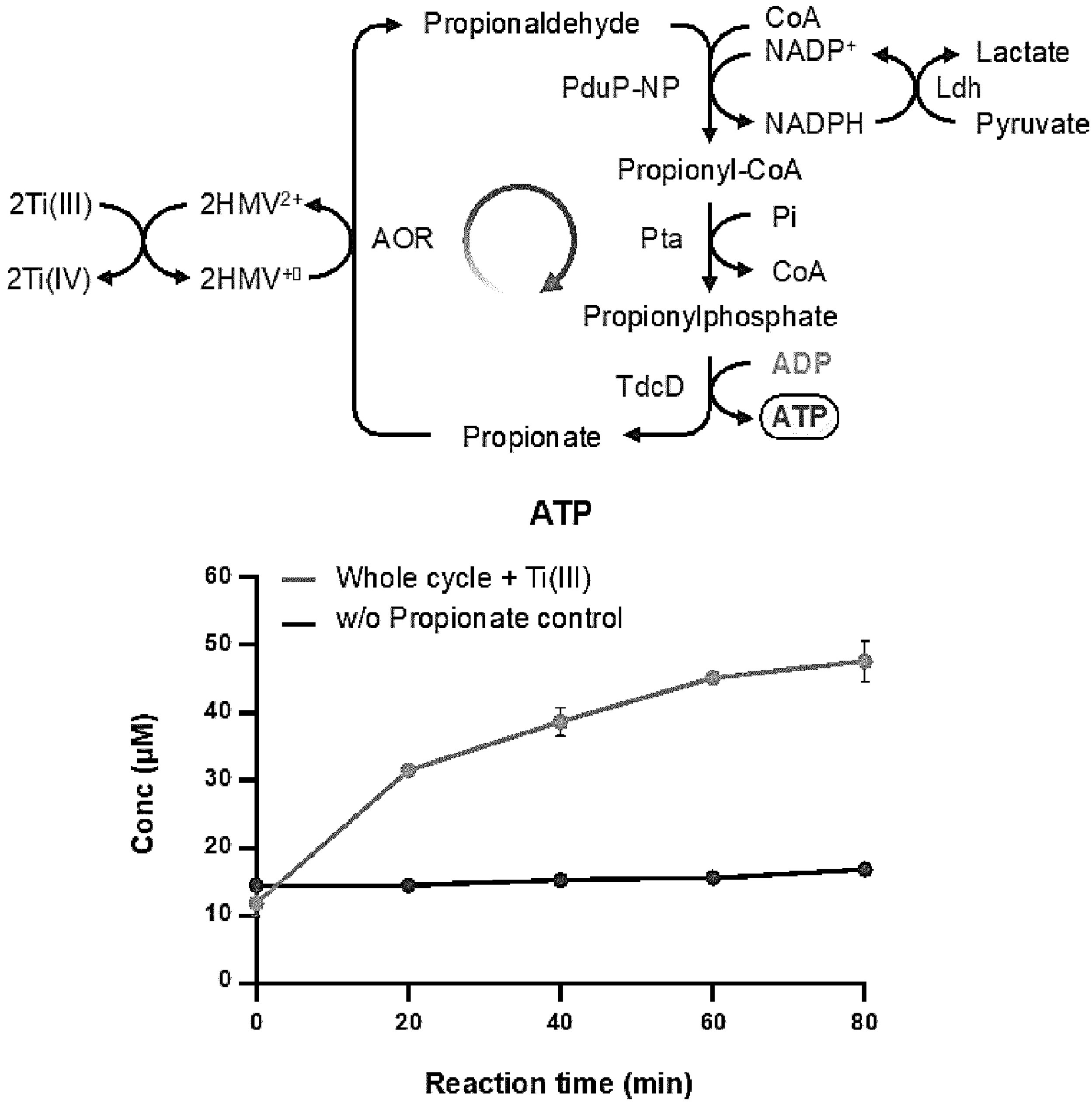
Propionaldehyde
CoA
NADP+
Lactate
PduP-NP
Ldh
NADPH
Pyruvate
Propionyl-CoA
2Ti(III)
2HMV2+
Pi
AOR
Pta
2Ti(IV)
2HMV+
CoA
Propionylphosphate
ADP
TdcD
ATP
Propionate
ATP
Whole cycle + Ti(III)
w/o Propionate control
Conc (μM)
0
10
20
30
40
50
60
0
20
40
60
80
Reaction time (min)

Figure 14

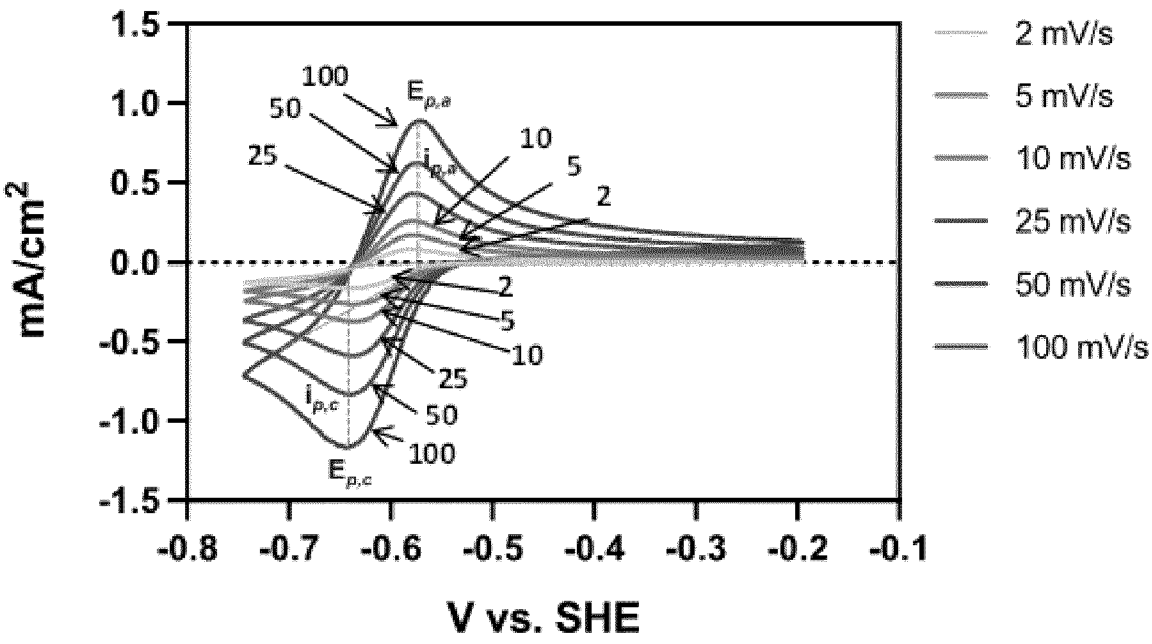
1.5
1.0
0.5
0.0
-0.5
-1.0
-1.5
mA/cm²
-0.8
-0.7
-0.6
-0.5
-0.4
-0.3
-0.2
-0.1
V vs. SHE
100
50
25
10
5
2
$E_{p,a}$
$i_{p,a}$
$i_{p,c}$
$E_{p,c}$
2 mV/s
5 mV/s
10 mV/s
25 mV/s
50 mV/s
100 mV/s

Figure 15

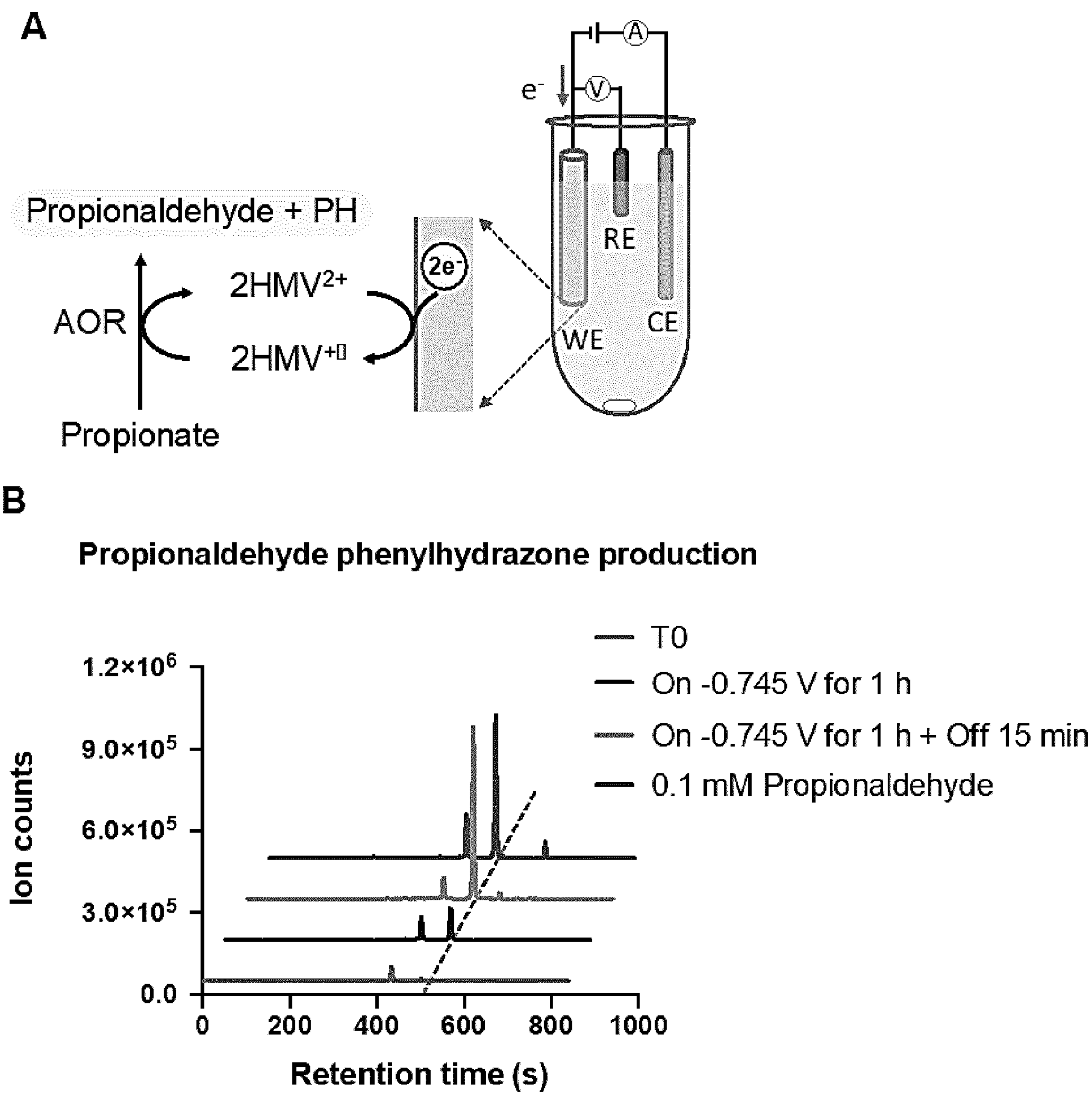
A
Propionaldehyde + PH
AOR
2HMV2+
2HMV+
Propionate
2e-
e-
A
V
RE
WE
CE
B
Propionaldehyde phenylhydrazone production
T0
On -0.745 V for 1 h
On -0.745 V for 1 h + Off 15 min
0.1 mM Propionaldehyde
Ion counts
1.2×10^6
9.0×10^5
6.0×10^5
3.0×10^5
0.0
0
200
400
600
800
1000
Retention time (s)

## Figure 16

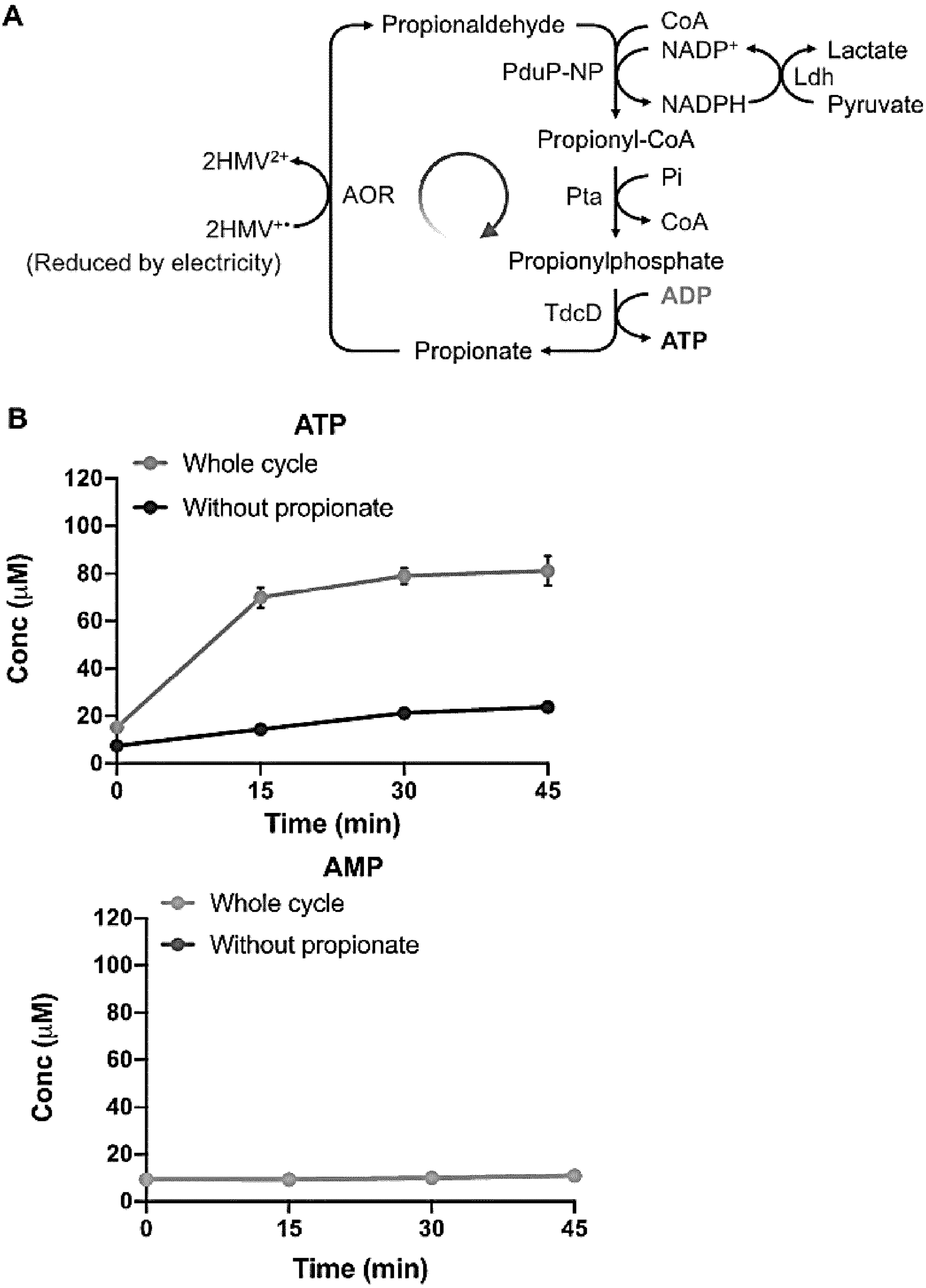
A
Propionaldehyde
CoA
NADP+
Lactate
PduP-NP
Ldh
NADPH
Pyruvate
Propionyl-CoA
2HMV2+
AOR
Pi
Pta
CoA
2HMV+•
(Reduced by electricity)
Propionylphosphate
ADP
TdcD
ATP
Propionate
B
ATP
Whole cycle
Without propionate
Conc (μM)
Time (min)
AMP
Whole cycle
Without propionate
Conc (μM)
Time (min)

## Figure 17

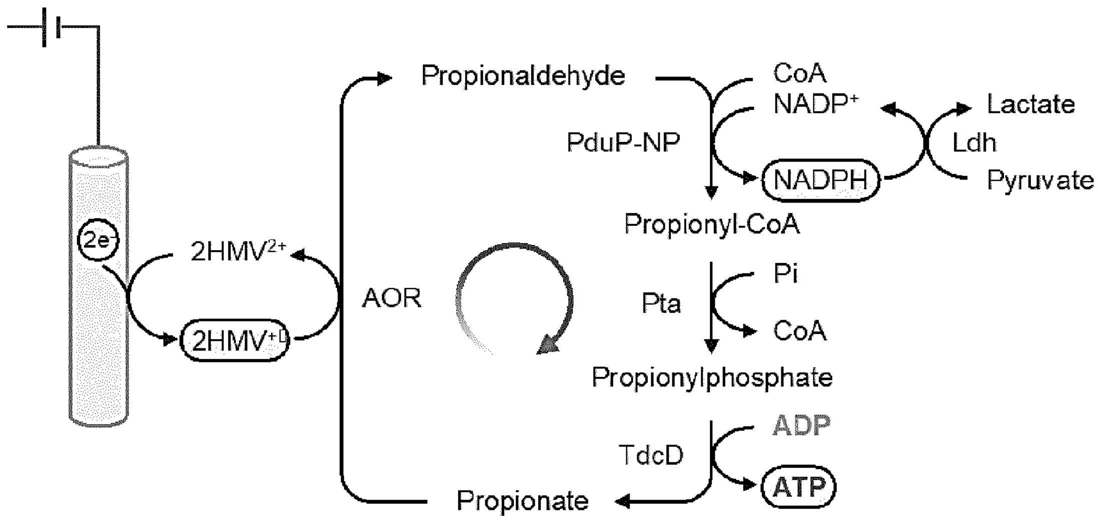


## Figure 18

A

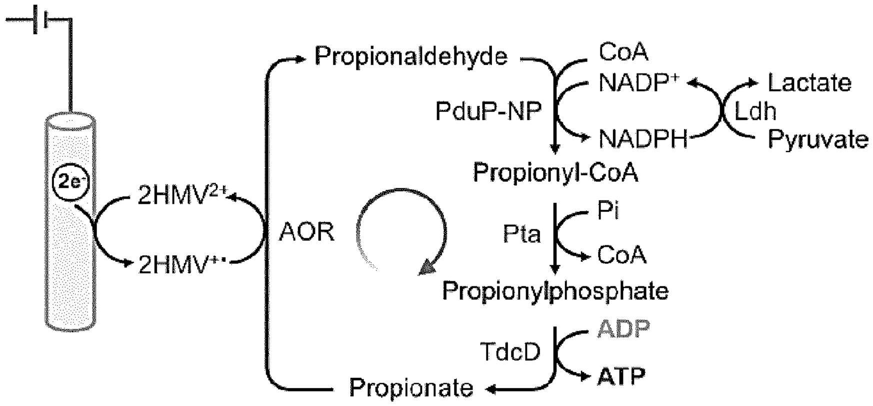


B

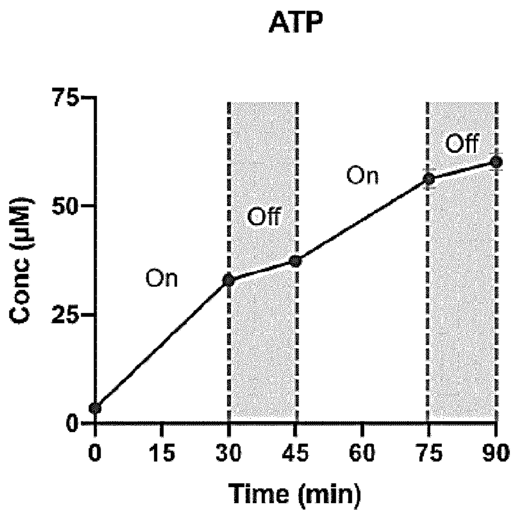

## Figure 19

A

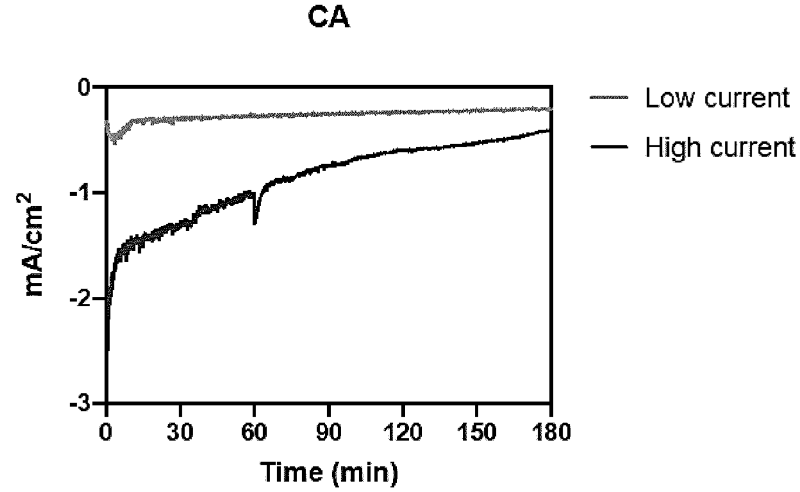


B

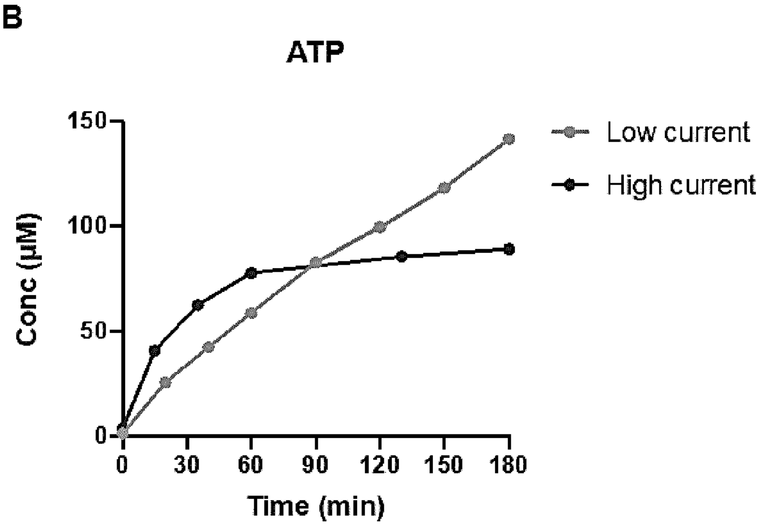


## Figure 20

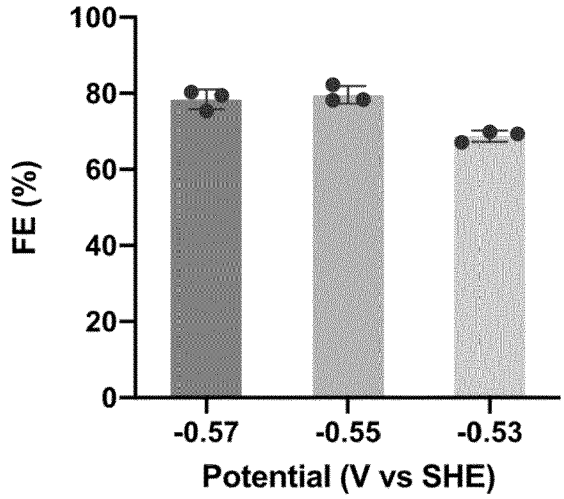

## Figure 21

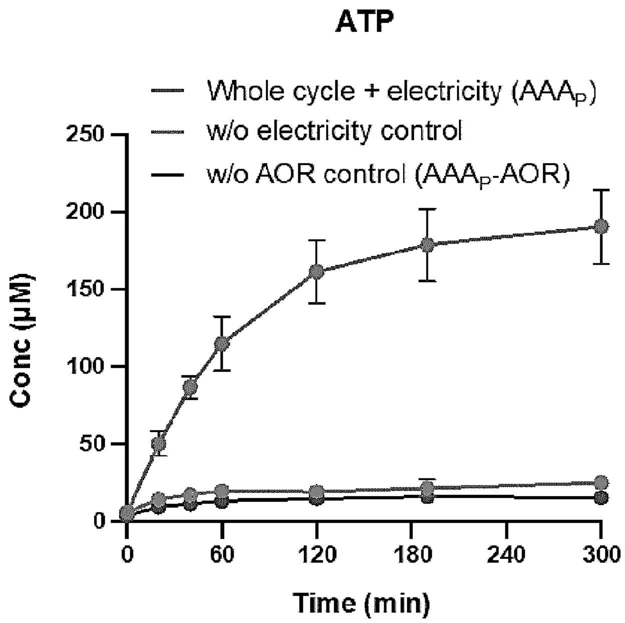


## Figure 22

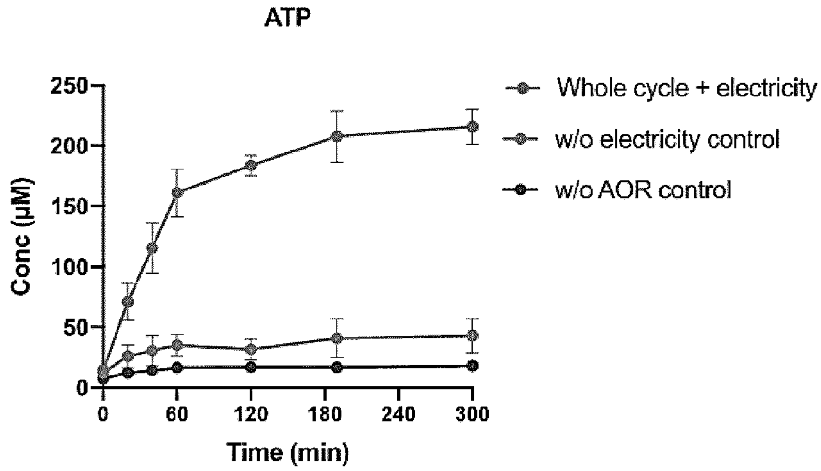


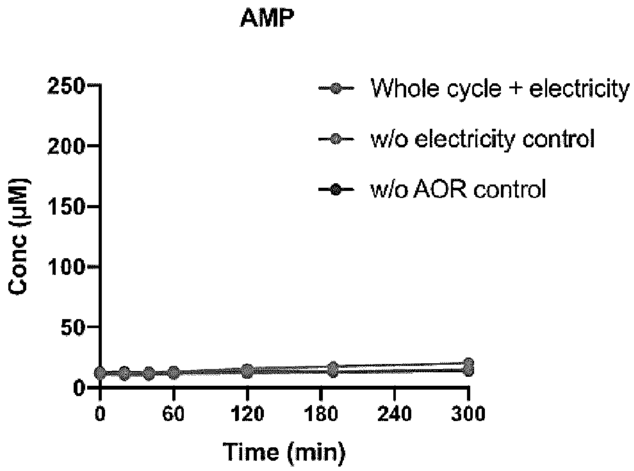

Figure 23

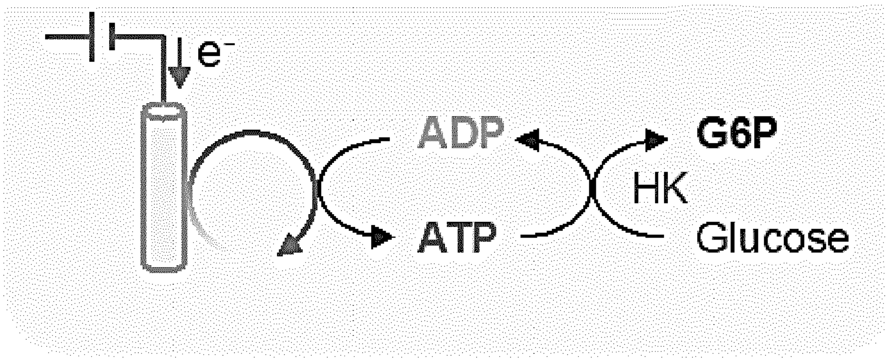
e⁻
ADP
G6P
HK
ATP
Glucose

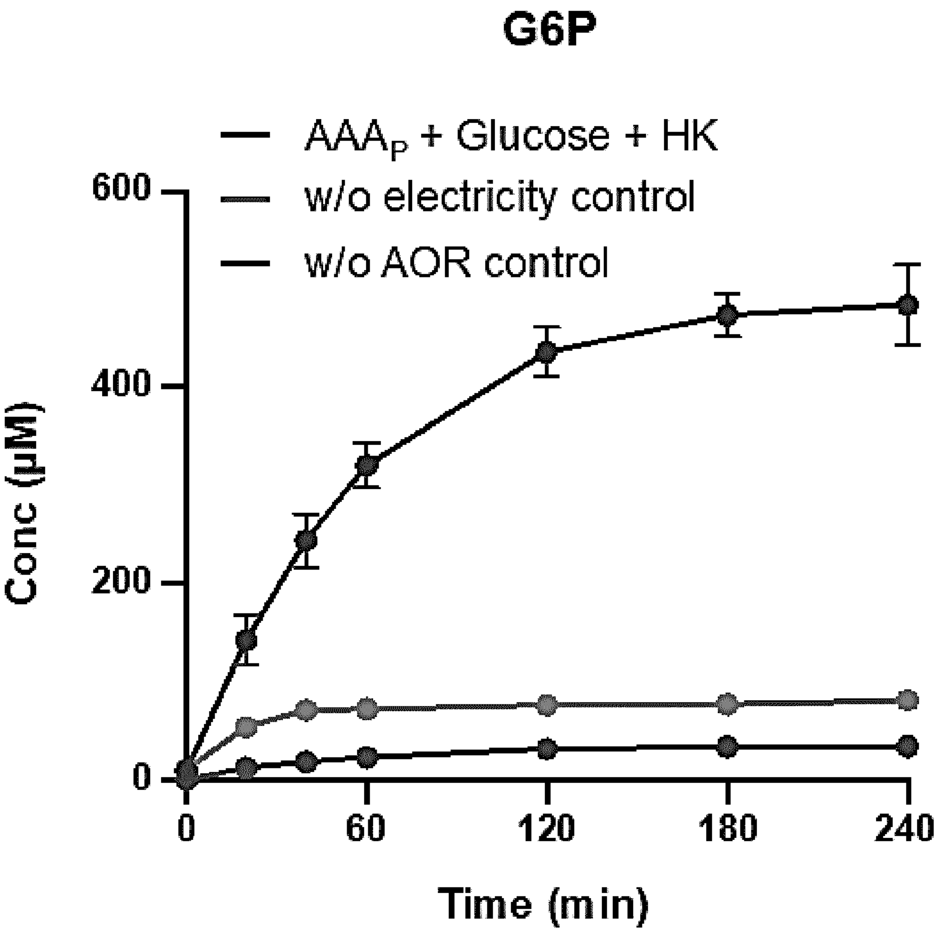
G6P
AAA$_P$ + Glucose + HK
w/o electricity control
w/o AOR control
600
400
200
0
Conc (μM)
0
60
120
180
240
Time (min)

Figure 24

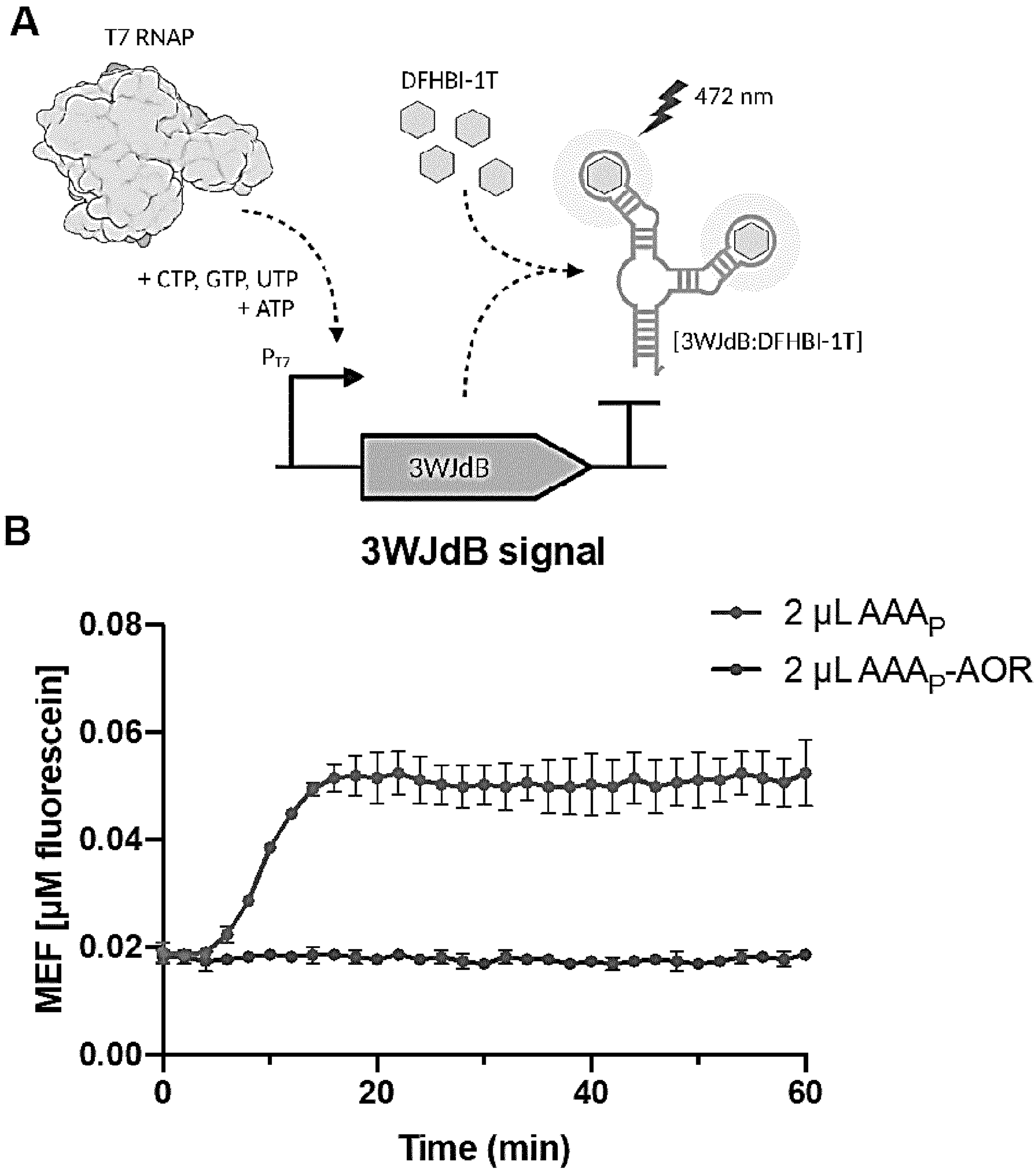
A
T7 RNAP
DFHBI-1T
472 nm
+ CTP, GTP, UTP
+ ATP
[3WJdB:DFHBI-1T]
$P_{T7}$
3WJdB
B
3WJdB signal
2 µL $AAA_P$
2 µL $AAA_P$-AOR
MEF [µM fluorescein]
0.08
0.06
0.04
0.02
0.00
0
20
40
60
Time (min)

Figure 25

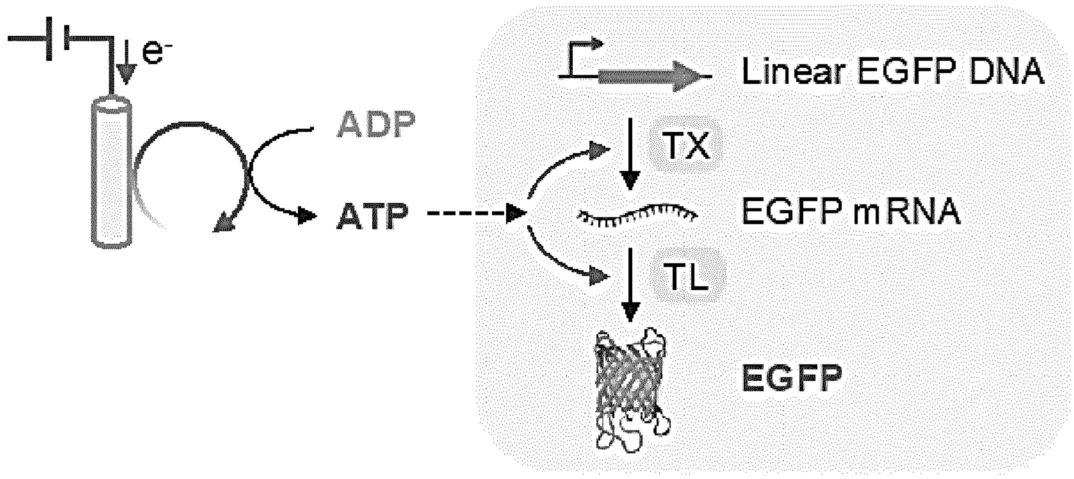
e-
ADP
ATP
Linear EGFP DNA
TX
EGFP mRNA
TL
EGFP

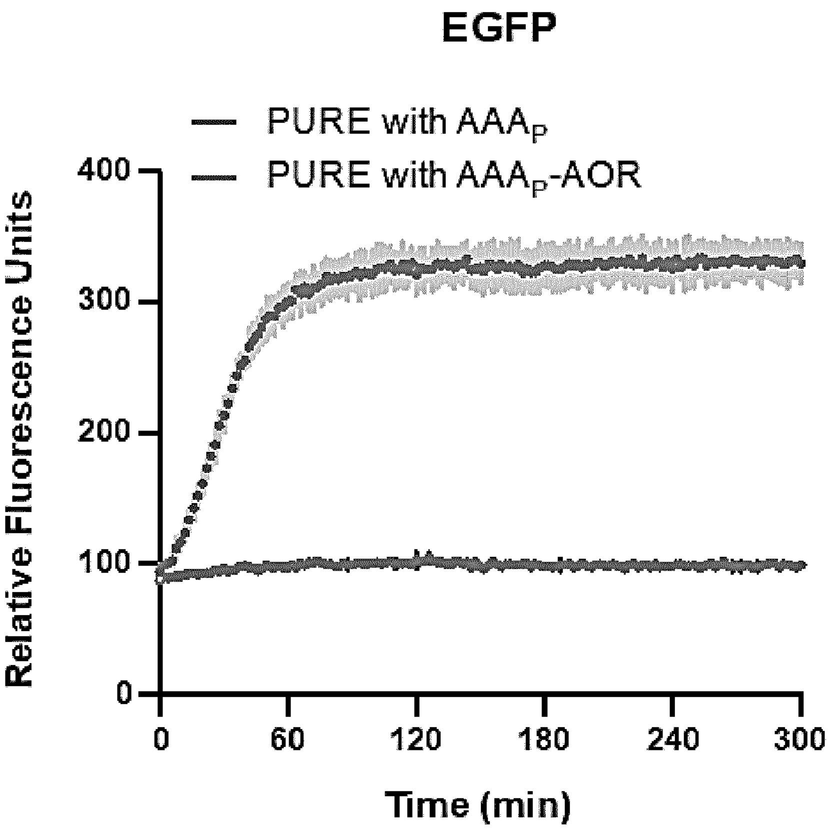
EGFP
PURE with AAA$_P$
PURE with AAA$_P$-AOR
Relative Fluorescence Units
400
300
200
100
0
0
60
120
180
240
300
Time (min)

Figure 26

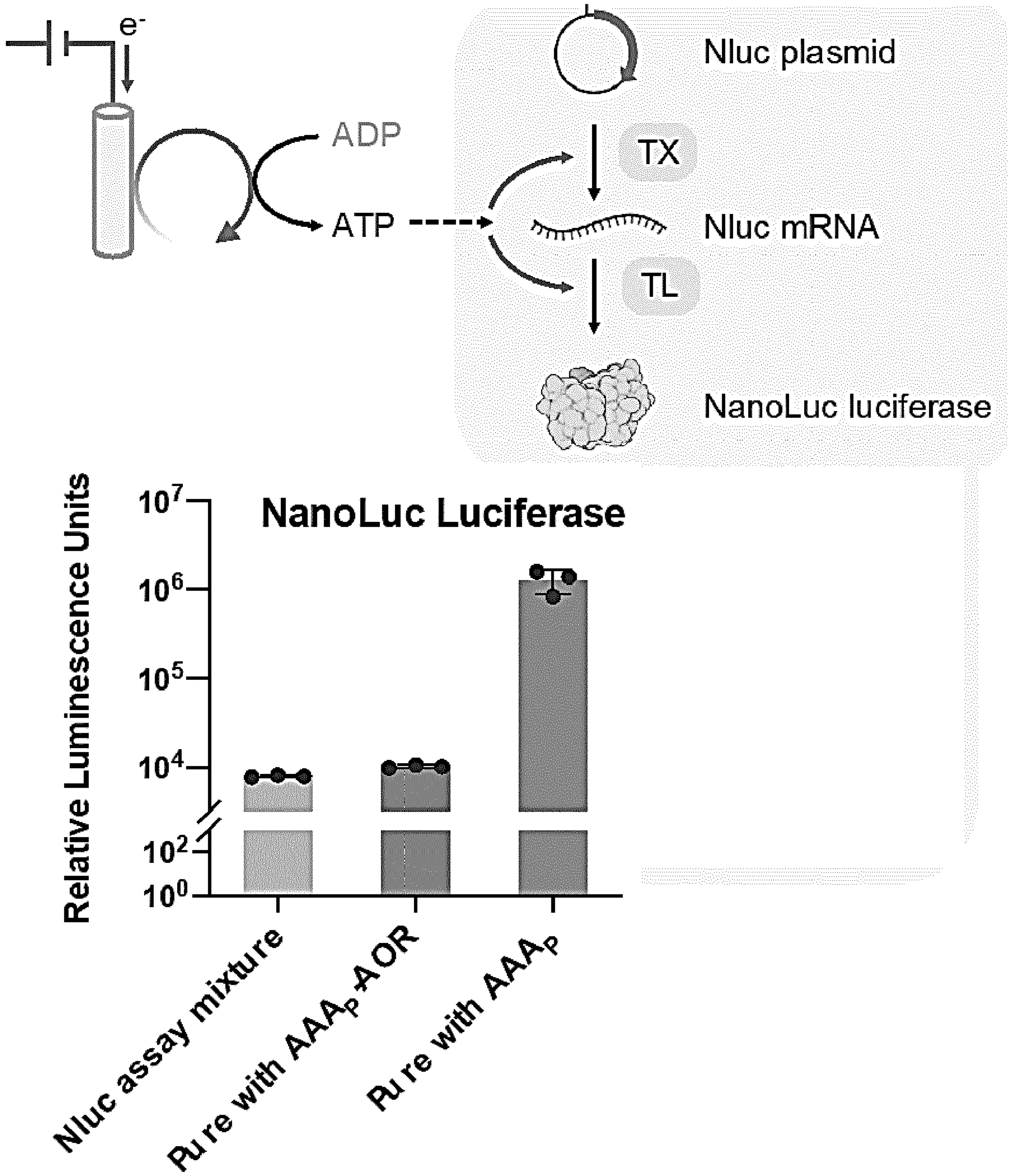
e-
ADP
ATP
Nluc plasmid
TX
Nluc mRNA
TL
NanoLuc luciferase
NanoLuc Luciferase
Relative Luminescence Units
$10^7$
$10^6$
$10^5$
$10^4$
$10^2$
$10^0$
Nluc assay mixture
Pu re with AAA$_P$-AOR
Pu re with AAA$_P$

**Figure 27**

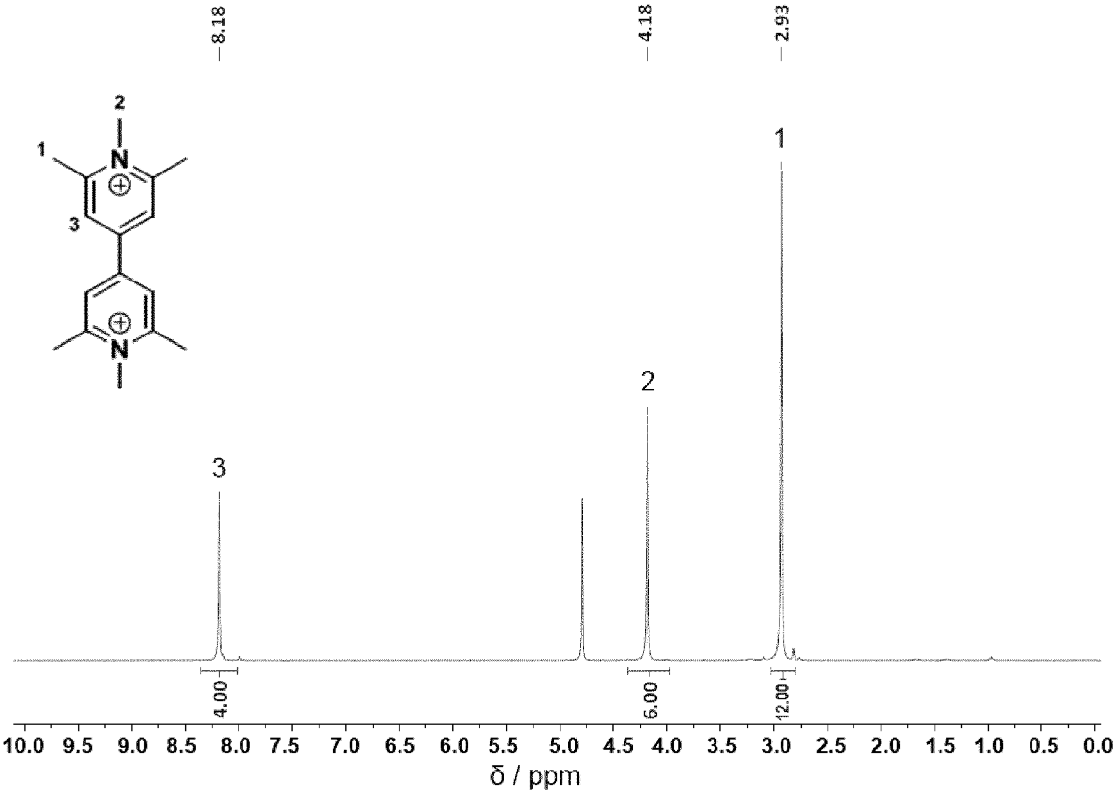


**Figure 28**

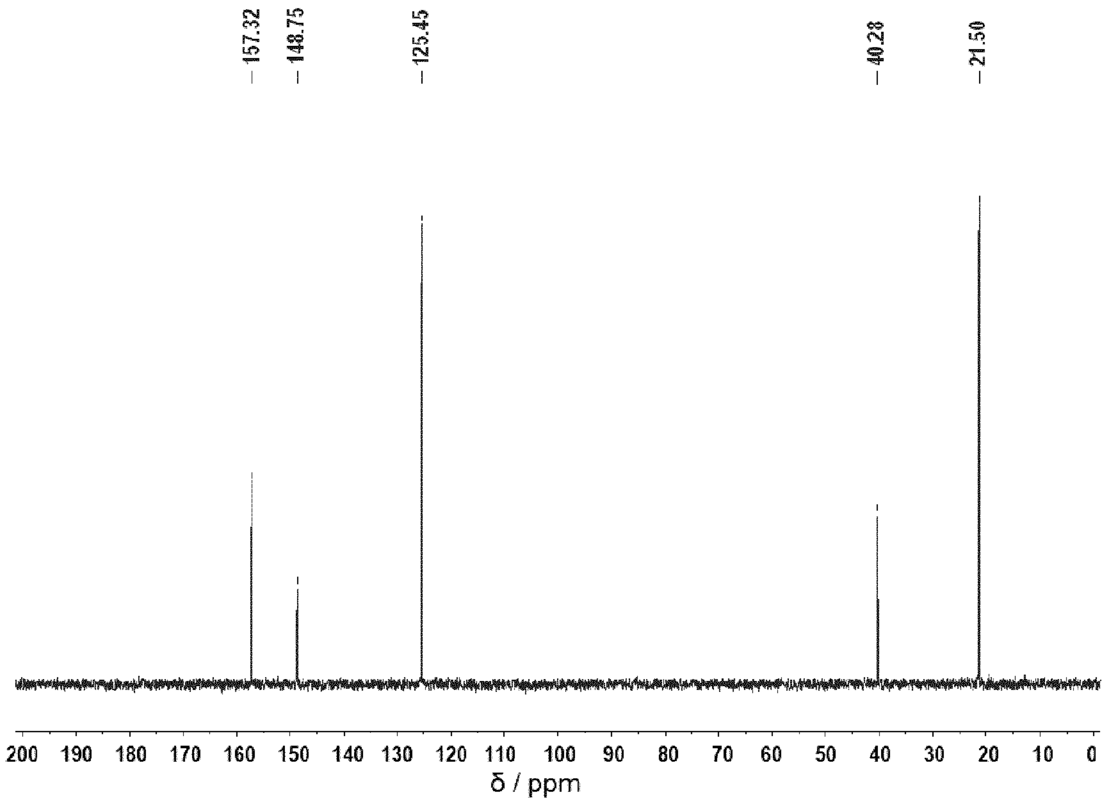

Figure 29

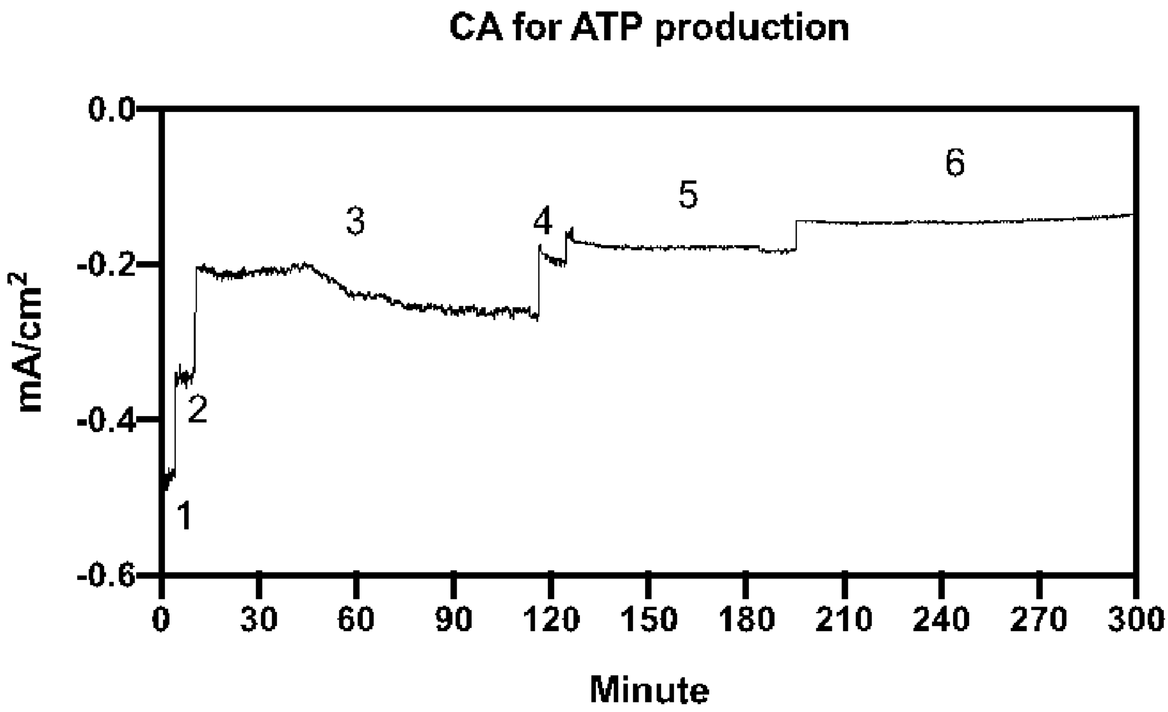


Figure 30

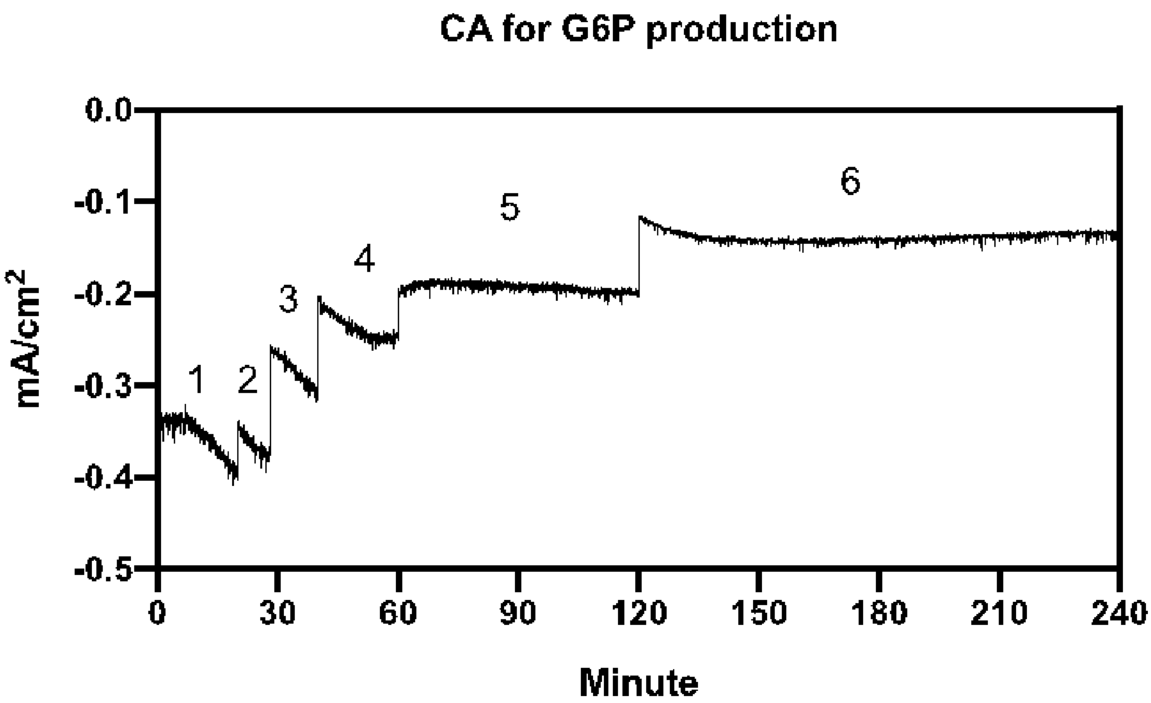

Europäisches Patentamt
European Patent Office
Office européen des brevets

# EUROPEAN SEARCH REPORT

Application Number
EP 23 18 1910

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2022/249085 A1 (SINOPOLI PAOLO [IT]; PUGLIESE ROBERTO [IT]) 1 December 2022 (2022-12-01) * page 1, paragraph [0003] * * page 3, paragraph [0012] - page 4, paragraph ]0015] * * page 5, paragraph [0019]-[0021]; figures 4-6 * * example 7 * * claims 1-40 * ----- | 1-15 | INV. C12P19/32 C12N15/52 C25B3/09 |
| A | WO 2009/052278 A1 (CORNELL RES FOUNDATION INC [US]; TRAVIS ALEXANDER [US]) 23 April 2009 (2009-04-23) * page 1, lines 5-13 * * page 4, line 21 - page 16, line 14; figures 1,2 * * claims 1-11 * ----- | 1-15 | |
| A | ÓSCAR GUTIÉRREZ-SANZ ET AL: "H2-Fueled ATP Synthesis on an Electrode: Mimicking Cellular Respiration", ANGEWANDTE CHEMIE INTERNATIONAL EDITION, VERLAG CHEMIE, HOBOKEN, USA, vol. 55, no. 21, 15 March 2016 (2016-03-15), pages 6216-6220, XP072072823, ISSN: 1433-7851, DOI: 10.1002/ANIE.201600752 * abstract * * page 6216, right-hand column, last paragraph - page 6218, right-hand column, last paragraph * ----- -/-- | 1-15 | TECHNICAL FIELDS SEARCHED (IPC) C12P C12N C25B |

The present search report has been drawn up for all claims

1

| Place of search | Date of completion of the search | Examiner |
|---|---|---|
| Berlin | 6 December 2023 | Mateo Rosell, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 23 18 1910

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2020/167999 A1 (BIOCHEMINSIGHTS INC [US]) 20 August 2020 (2020-08-20)<br>* page 1, paragraph [0002] *<br>* page 2, paragraph [0008] - page 3, paragraph [0014] *<br>* page 16, paragraph [0074] - page 17, paragraph [0076]; figures 3,4 *<br>* examples 1-4 *<br>* claims 1-15 *<br>----- | 1-15 | |
| A | WO 2016/137976 A1 (BIOCHEMINSIGHTS INC [US]) 1 September 2016 (2016-09-01)<br>* page 1, paragraph [0002]; figure 1 *<br>* claims 1-21 *<br>----- | 1-15 | |
| A | WO 2010/074760 A1 (GREENLIGHT BIOSCIENCES [US]; ZARUR ANDREY J [US]; SWARTZ JAMES [US]) 1 July 2010 (2010-07-01)<br>* page 2, line 21 - page 4, line 24; figures 1-3; example 1 *<br>----- | 1-15 | TECHNICAL FIELDS SEARCHED (IPC) |
| A | WINIARSKA AGNIESZKA ET AL: "Tungsten Enzyme Using Hydrogen as an Electron Donor to Reduce Carboxylic Acids and NAD +",<br>ACS CATALYSIS,<br>vol. 12, no. 14, 6 July 2022 (2022-07-06), pages 8707-8717, XP093109026,<br>US<br>ISSN: 2155-5435, DOI: 10.1021/acscatal.2c02147<br>Retrieved from the Internet:<br>URL:https://pubs.acs.org/doi/pdf/10.1021/acscatal.2c02147><br>* abstract *<br>* page 8708, right-hand column, paragraph 2 - page 8714, left-hand column, paragraph 1; figures 1,2; tables 1,2 *<br>-----<br>-/-- | 1-15 | |

The present search report has been drawn up for all claims

1

| Place of search | Date of completion of the search | Examiner |
|---|---|---|
| Berlin | 6 December 2023 | Mateo Rosell, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........
& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 23 18 1910

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| T | LUO SHANSHAN ET AL: "ATP production from electricity with a new-to-nature electrobiological module", JOULE, vol. 7, no. 8, 1 August 2023 (2023-08-01), pages 1745-1758, XP093108589, ISSN: 2542-4351, DOI: 10.1016/j.joule.2023.07.012 * the whole document * ----- | | |
| | | | TECHNICAL FIELDS SEARCHED (IPC) |

The present search report has been drawn up for all claims

1

| Place of search | Date of completion of the search | Examiner |
|---|---|---|
| Berlin | 6 December 2023 | Mateo Rosell, A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 3 of 3

## ANNEX TO THE EUROPEAN SEARCH REPORT ON EUROPEAN PATENT APPLICATION NO.

EP 23 18 1910

This annex lists the patent family members relating to the patent documents cited in the above-mentioned European search report. The members are as contained in the European Patent Office EDP file on
The European Patent Office is in no way liable for these particulars which are merely given for the purpose of information.

06-12-2023

| Patent document cited in search report | Publication date | Patent family member(s) | Publication date |
|---|---|---|---|
| WO 2022249085 A1 | 01-12-2022 | NONE | |
| WO 2009052278 A1 | 23-04-2009 | CN 101889094 A | 17-11-2010 |
| | | EP 2205755 A1 | 14-07-2010 |
| | | US 2011059373 A1 | 10-03-2011 |
| | | US 2020131548 A1 | 30-04-2020 |
| | | WO 2009052278 A1 | 23-04-2009 |
| WO 2020167999 A1 | 20-08-2020 | BR 112021015954 A2 | 25-01-2022 |
| | | CA 3129926 A1 | 20-08-2020 |
| | | CN 114729336 A | 08-07-2022 |
| | | EP 3924471 A1 | 22-12-2021 |
| | | US 2022145282 A1 | 12-05-2022 |
| | | WO 2020167999 A1 | 20-08-2020 |
| WO 2016137976 A1 | 01-09-2016 | BR 112017018025 A2 | 10-04-2018 |
| | | CA 3015513 A1 | 01-09-2016 |
| | | CN 107849584 A | 27-03-2018 |
| | | EP 3262179 A1 | 03-01-2018 |
| | | US 2018037914 A1 | 08-02-2018 |
| | | US 2020325498 A1 | 15-10-2020 |
| | | WO 2016137976 A1 | 01-09-2016 |
| WO 2010074760 A1 | 01-07-2010 | CA 2749877 A1 | 01-07-2010 |
| | | CN 102333878 A | 25-01-2012 |
| | | CN 105132387 A | 09-12-2015 |
| | | EP 2379728 A1 | 26-10-2011 |
| | | IL 213691 A | 29-10-2015 |
| | | JP 5905724 B2 | 20-04-2016 |
| | | JP 2012513213 A | 14-06-2012 |
| | | KR 20110134380 A | 14-12-2011 |
| | | US 2011008867 A1 | 13-01-2011 |
| | | WO 2010074760 A1 | 01-07-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **KITAGAWA, M. et al.** Complete set of ORF clones of Escherichia coli ASKA library (A complete set of E. coli K-12 ORF archive): unique resources for biological research. *DNA Res.*, 2005, vol. 12, 291-299
- **WINIARSKA, A. et al.** Tungsten Enzyme Using Hydrogen as an Electron Donor to Reduce Carboxylic Acids and NAD+. *ACS Catal.*, 2022, vol. 12, 8707-8717
- **ZARZYCKI, J. et al.** In Vitro Characterization and Concerted Function of Three Core Enzymes of a Glycyl Radical Enzyme - Associated Bacterial Microcompartment. *Sci. Rep.*, 2017, vol. 7, 1-12